# EUROPEAN PATENT SPECIFICATION

(11) **EP 1 759 210 B1**
(45) Date of publication and mention of the grant of the patent: **31.12.2008**
(21) Application number: 05742946.6
(22) Date of filing: 06.05.2005
(51) Int. Cl.: G01N 33/543, G01N 33/552, C12Q 1/68, C07F 7/18, C08L 83/04, C08G 18/00

(54) **METHOD FOR PREPARING SUBSTRATES HAVING IMMOBILIZED MOLECULES AND SUBSTRATES**
VERFAHREN ZUR HERSTELLUNG VON SUBSTRATEN MIT IMMOBILISIERTEN MOLEKÜLEN UND SUBSTRATEN
PROCEDE POUR LA PREPARATION DE SUBSTRATS COMPORTANT DES MOLECULES IMMOBILISEES ET SUBSTRATS

(30) Priority: 06.05.2004 US 568879 P; 06.05.2004 US 568767 P
(43) Date of publication of application: 07.03.2007
(73) Proprietor: Nanosphere, Inc., Northbrook, Illinois 60062 (US)
(72) Inventor: GARIMELLA, Viswanadham, Vernon Hills, IL 60061 (US); ZHUKOVYTSKYY, Vadym, Des Plaines, IL 60016 (US)
(74) Representative: Grund, Martin
(86) International application number: PCT/US2005/016134
(87) International publication number: WO 2005/108625

(56) References cited:
- WO-A-01/46213
- WO-A-01/51689
- WO-A-03/006676
- US-A- 5 399 501
- US-A- 5 532 170
- US-A1- 2002 155 442
- US-A1- 2003 166 261
- US-A1- 2004 096 856
- US-B1- 6 248 127
- HUCKEL M ET AL: "Porous zirconia: a new support material for enzyme immobilization." JOURNAL OF BIOCHEMICAL AND BIOPHYSICAL METHODS. 5 FEB 1996, vol. 31, no. 3-4, 5 February 1996 (1996-02-05), pages 165-179, XP002361448 ISSN: 0165-022X
- ANSPACH F B ET AL: "High-performance liquid affinity chromatography with phenylboronic acid, benzamidine, tri-L-alanine, and concanavalin A immobilized on 3-isothiocyanatopropyltriethoxysilane-acti vated nonporous monodisperse silicas." ANALYTICAL BIOCHEMISTRY. 15 MAY 1989, vol. 179, no. 1, 15 May 1989 (1989-05-15), pages 171-181, XP008057923 ISSN: 0003-2697
- WIESER R J ET AL: "Plasma membrane glycoproteins covalently bound to silica beads as a model for molecular studies of cell-cell interactions in culture." JOURNAL OF BIOCHEMICAL AND BIOPHYSICAL METHODS. OCT 1987, vol. 15, no. 1, October 1987 (1987-10), pages 13-21, XP002361450 ISSN: 0165-022X
- KUMAR A ET AL: "Silanized nucleic acids: a general platform for DNA immobilization." NUCLEIC ACIDS RESEARCH. 15 JUL 2000, vol. 28, no. 14, 15 July 2000 (2000-07-15), page E71, XP002361451 ISSN: 1362-4962 cited in the application
- LINDROOS K ET AL: "Minisequencing on oligonucleotide microarrays: comparison of immobilisation chemistries." NUCLEIC ACIDS RESEARCH. 1 JUL 2001, vol. 29, no. 13, 1 July 2001 (2001-07-01), pages E69-E69, XP002361533 ISSN: 1362-4962 cited in the application

## Description

### BACKGROUND OF THE INVENTION

Surface modification plays an important role in micro-array biomolecule detection technology for controlling backgrounds and spot morphology. Several modifications were developed using different type of commercially available silanes such as silyl amines, aldehydes, thiols etc. for immobilization of biomolecules such as oligonucleotides. After coating the surface with reactive silanes, the next challenge is immobilization of required biomolecules on the modified surface. The surface loadings always vary with different silanes and even same silane may not give reproducible results. Reproducibility of optimum surface loading has always been a great challenge in this field since surface loading dictates the performance of the assay. Even with simple linear molecules for immobilization, the optimum loading on the surface is difficult to achieve. Attaching DNA to a modified glass surface is a central step for many applications in DNA diagnostics industry including gene expression analysis. In general, DNA can be attached to a glass surface either through non-covalent, ionic interactions, or through multi-step processes or simple coupling reactions. Several methods have been reported in the literature using glass surface modified with different types of silylating agents. See, for instance, Nucleic Acids research, vol 22, 5456-5465 (1994); Nucleic Acids research, vol 24, 3040-3047 (1996); Nucleic Acids research, vol 24, 3031-3039 (1996); Nucleic Acids research, vol 27, 1970-1977 (1999); Angew Chem. Int. Ed, 38, No.9, 1297 (1999); Analytical biochemistry 280, 143-150 (2000).All these reported methods involve silylating step which uses expensive reagents and analytical tools. Also, these methods are also multi-step processes that are labor intensive and expensive. See, for instance, Nucleic Acids research, vol 29, 955-959 (2001); Nucleic Acids research, vol 29, No.13 e69 (2001).Earlier reported methods have involved a laborious synthesis and time consuming procedure. See, for instance, Nucleic Acids research, vol. 28, No.13 E71 (2000); Huber et al. WO 01/46214, published June 28, 2001; Huber et al. WO 01/46213, published June 28, 2001; and Huber et al. WO 01/46464, published June 28, 2001.

Indeed, many of the current immobilization methods suffer from one or more of a number of disadvantages. Some of these are, complex and expensive reaction schemes with low oligonucleotide loading yields, reactive unstable intermediates prone to side reactions and unfavorable hybridization kinetics of the immobilized oligonucleotide. The efficient immobilization of oligonucleotides or other molecules on glass surface in arrays requires a) simple reliable reactions giving reproducible loading for different batches, b) stable reaction intermediates, c) arrays with high loading and fast hybridization rates, d) high temperature stability, e) low cost, f) specific attachment at either the 5'- or 3'-end or at an internal nucleotide and g) low background noise.

One important development in DNA detection methods involves the use of gold nanoparticle probes modified with oligonucleotides to indicate the presence of a particular DNA. For instance, one such method is described in application number PCT/US00/17507. Typically, oligonucleotides are attached to a nanoparticle that have sequences complementary to the nucleic acid to be detected. The nanoparticle conjugate formed by hybridization to the nucleic acid results in a detectable change, thereby indicating the presence of the targeted nucleic acid. Many methods of detecting nucleic acids utilize an array substrate, such as described in U.S. published application number 2004/0072231. By employing a substrate, the detectable change can be amplified using silver staining techniques and the sensitivity of the assay is greatly increased.

In cases involving nanoparticle-labeled probes, particularly gold nanoparticle probes, for detection of target analytes on capture substrates, the detection of extremely low amounts of target analytes in a sample may be complicated by a relative high background signal due to non-specific binding of the nanoparticle-based detection probes onto substrate surfaces. Similarly, in cases involving relatively low concentrations of target analyte, it would be desirable to confirm that the absence of nanoparticle-labeled detection probes immobilized on the surface of substrates is either due to the absence of the target analyte in a sample or due to defective substrate surface preparation. Accordingly, a substrate and method of preparation which eliminates or substantially reduces the level of background noise in nanoparticle-based detection systems would be highly desirable. In addition, a method for direct immobilization of nanoparticles on a substrate surface would be useful in several detection methods, including those described above, such as a positive control to detect hybridization efficiency (and therefore quality) of different batches of modified substrates and for detecting targets using surface plasmon resonance (SPR) angle shift techniques with different sized DNA modified nanoparticle probes.

The present invention represents a significant step in the direction of meeting or approaching several of these objectives.

### SUMMARY OF THE INVENTION

The present invention relates to methods, substrates, and kits as defined in the appended claims. Methods for the attachment of molecules such as oligonucleotides or proteins onto substrates surfaces such as unmodified glass surfaces or polymeric substrates without the need for laborious synthetic steps, with increased surface loading densities, and with greater reproducibility and which avoids the need for pre-surface modifications are disclosed. Molecules such as DNA (either labeled or unlabeled) can be silylated at either the 3' or 5' ends as discussed below and the 3' or 5'-silylated DNA may then be covalently attached directly to a surface such as a pre-cleaned glass surface (Scheme) for use in hybridization assays. Furthermore, thorough the use of certain silylating reagents, it is now possible to further enhance surface loading densities by using modified silylating agents having multiple molecules attached thereto. Moreover, through the use of certain silylating reagents in combination with spacer molecules such as polymers with free amino groups and crosslinker molecules, it is possible to prepare substrates that are surprisingly suitable for use in nanoparticle-based detection systems. Methods for attaching molecules onto a substrate, devices prepared by such methods, and compositions are disclosed. This method provides great advantages over the present technology in terms of simplicity, cost, speed, safety, and reproducibility.

Thus, a method is provided for making a substrate for use in target analyte detection. The method comprises: (a) providing a substrate having a surface; (b) contacting said surface with a isocyanate compound so as to provide a surface comprising free isocyanate groups, the isocyanate compound is a member selected from the group consisting of:

Si(NCY)₄;

[(R₁)(R₂)(R₃)Si-X-Z-CYNH]₂-Si (NCY)₂ vi;

and

(R₁)(R₂)(R₃)Si-X-Z-CYNH-Si (NCY)₃ iv;

wherein R₁, R₂ and R₃ independently represents C₁-C₆ alkoxy, C₁-C₆ alkyl, phenyl, or aryl substituted with one or more groups selected from the group consisting of C₁-C₆ alkyl and C₁-C₆ alkoxy; X represents linear or branched C₁-C₂₀ alkyl or aryl substituted with one or more groups selected from the group consisting of C₁-C₆ alkyl and C₁-C₆ alkoxy, optionally substituted with one or more heteroatoms comprising oxygen, nitrogen, or sulfur; Y represents oxygen or sulfur; and Z represents oxygen or NH, with the proviso that at least one of R₁, R₂, or R₃ represents C₁-C₆ alkoxy.

A method is provided for making a substrate for use in target analyte detection. The method comprises: (a) providing a substrate having a surface; (b) contacting said surface with a isocyanate compound so as to provide a surface comprising free isocyanate groups, the isocyanate compound is a member selected from the group consisting of:

Si(NCY)₄;

[(R₁)(R₂)(R₃)Si-X-Z-CYNH]₂-Si (NCY)₂ vi;

and

(R₁)(R₂)(R₃)Si-X-Z-CYNH-Si (NCY)₃ iv;

wherein R₁, R₂ and R₃ independently represents C₁-C₆ alkoxy, C₁-C₆ alkyl, phenyl, or aryl substituted with one or more groups selected from the group consisting of C₁-C₆ alkyl and C₁-C₆ alkoxy; X represents linear or branched C₁-C₂₀ alkyl or aryl substituted with one or more groups selected from the group consisting of C₁-C₆ alkyl and C₁-C₆ alkoxy, optionally substituted with one or more heteroatoms comprising oxygen, nitrogen, or sulfur; Y represents oxygen or sulfur; and Z represents oxygen or NH, with the proviso that at least one of R₁, R₂, or R₃ represents C₁-C₆ alkoxy; (c) contacting said surface comprising free isocyanate groups with a spacer molecule so as to provide a surface comprising free amino groups; and (d) contacting said surface comprising free amino groups with a linker molecule so as to provide a reactive surface having free reactive groups.

In one aspect, steps (c) and (d) may be repeated one or more times.

In another aspect, the method comprises after step (d): (e) contacting said reactive surface with at least one type of capture probe specific for the target analyte so as to provide a surface comprising immobilized capture probes; and (f) contacting said surface comprising immobilized capture probes with a capping agent so as to block residual unreacted free isocyanate groups on areas of the surface not having immobilized capture probes and produce a substrate having substantially low signal background due to non-specific nanoparticle binding relative to a surface not contacted with a capping agent.

In another aspect, the method further comprises: (i) contacting said reactive surface with at least one type of capture probe specific for the target analyte so as to provide a surface comprising immobilized capture probes; and (ii) contacting said surface comprising immobilized capture probes with a capping agent so as to block residual unreacted free isocyanate groups on areas of the surface not having immobilized capture probes and produce a substrate having substantially low signal background due to non-specific nanoparticle binding relative to a surface not contacted with a capping agent.

A method is provided for making a substrate for use in target analyte detection. The method comprises: (a) providing a substrate having a surface; (b) contacting said surface with a isocyanate compound so as to provide a surface comprising free isocyanate groups, the isocyanate compound is a member selected from the group consisting of:

Si(NCY)₄;

[(R₁)(R₂)(R₃)Si-X-Z-CYNH]₂-Si (NCY)₂ vi;

and

(R₁)(R₂)(R₃)Si-X-Z-CYNH-Si (NCY)₃ iv;

wherein R₁, R₂ and R₃ independently represents C₁-C₆ alkoxy, C₁-C₆ alkyl, phenyl, or aryl substituted with one or more groups selected from the group consisting of C₁-C₆ alkyl and C₁-C₆ alkoxy; X represents linear or branched C₁-C₂₀ alkyl or aryl substituted with one or more groups selected from the group consisting of C₁-C₆ alkyl and C₁-C₆ alkoxy, optionally substituted with one or more heteroatoms comprising oxygen, nitrogen, or sulfur; Y represents oxygen or sulfur; and Z represents oxygen or NH, with the proviso that at least one of R₁, R₂, or R₃ represents C₁-C₆ alkoxy; (c) contacting the surface comprising free isocyanate groups with water so as to provide a surface comprising free amino groups; and (d) contacting said surface comprising free amino groups with a linker molecule so as to provide a reactive surface having free reactive groups.

In one aspect, the method further comprising, after step (d): (e) contacting said surface comprising free isocyanate groups with a spacer molecule so as to provide a surface comprising free amino groups; and (f) contacting said surface comprising free amino groups with a linker molecule so as to provide a reactive surface having free reactive groups.

In one aspect, steps (e) and (f) may be repeated one or more times.

In another aspect, the method further comprises: (i) contacting said reactive surface with at least one type of capture probe specific for the target analyte so as to provide a surface comprising immobilized capture probes; and (ii) contacting said surface comprising immobilized capture probes with a capping agent so as to block residual unreacted free isocyanate groups on areas of the surface not having immobilized capture probes and produce a substrate having substantially low signal background due to non-specific nanoparticle binding relative to a surface not contacted with a capping agent.

A method for making a substrate for use in detection of a target analyte is provided. The method comprises: (a) providing a substrate having a surface; (b) contacting said surface with a isocyanate compound so as to provide a surface comprising free isocyanate groups, the isocyanate compound is a member selected from the group consisting of:

Si(NCY)₄;

[(R₁)(R₂)(R₃)Si-X-Z-CYNH]₂-Si (NCY)₂ vi;

and

(R₁)(R₂)(R₃)Si-X-Z-CYNH-Si (NCY)₃ iv;

wherein R₁, R₂ and R₃ independently represents C₁-C₆ alkoxy, C₁-C₆ alkyl, phenyl, or aryl substituted with one or more groups selected from the group consisting of C₁-C₆ alkyl and C₁-C₆ alkoxy; X represents linear or branched C₁-C₂₀ alkyl or aryl substituted with one or more groups selected from the group consisting of C₁-C₆ alkyl and C₁-C₆ alkoxy, optionally substituted with one or more heteroatoms comprising oxygen, nitrogen, or sulfur; Y represents oxygen or sulfur; and Z represents oxygen or NH, with the proviso that at least one of R₁, R₂, or R₃ represents C₁-C₆ alkoxy; (c) contacting said surface comprising free isocyanate groups with a spacer molecule so as to provide a surface comprising free amino groups; (d) contacting said surface comprising free amino groups with a linker molecule so as to provide a reactive surface having free reactive groups; (e) contacting said reactive surface with at least one type of capture probe specific for the target analyte so as to provide a surface comprising immobilized capture probes; and (f) contacting said surface comprising immobilized capture probes with a capping agent so as to block residual unreactive free isocyanate groups and produce a substrate having substantially low signal background due to non-specific nanoparticle binding relative to a surface not contacted with a capping agent.

In any of the above methods for making a substrate, the isocyanate compound may be is selected from the group consisting of 2-Trimethoxysilane-6-triisocyanatosilanceureabenzene, 3-(triethoxysilyl) propylisocyanate), and tetraisocyanatosilane.

The spacer molecule can be any substance having a molecular structure that provides a plurality of functional groups. The spacer molecule may include at least one first functional group that can react with free isocyanate groups previously attached to the surface; at least one second functional group for attachment to a cross-linker molecule which can then be subsequently attached to another spacer molecule or to a capture probe; and at least one optional third functional groups for providing a negative charge. Both the first and second functional groups are any suitable nucleophilic group that can react with a reactive functional group such as isocyanate. Examples of nucleophilic groups include -OH, -SH, NH-, and -NH₂. The optional third function group includes a carboxylate group. Preferably, the first and second functional groups are free amino groups.

Spacer molecules may include many different types of polymers; preferably those incorporating multiple functional groups. Representative examples of these types of polymers include, without limitation, poly (dimmer acid-co-alkylpolyamine)-95, poly(dimmer acid-co-alkylpolyamine)-140, poly(allylamine), and poly(m-xylendiamine-epichlorohydrin diamine terminated, and PAMAM dendrimer generation 0. Types of polymers also include, without limitation, carbohydrates and polysaccharides. A representative example includes neomycin. Other spacer molecules include low molecular weight compounds that provide the designated functionality; preferred examples include 3,3'-diaminobenzidene, and tris(2-aminoethylamine).

The capping reagents include amino acid, protein, carbohydrate, carboxylate, thiol, alcohol, and amine. A representative, but non-limiting, example includes glycine.

Representative, but non-limiting examples of isocyanate compound include phenylene 1,4-diisocyanate, tolylene-2,6-diisocyanate, tolylene-α,4-diisocyanate, and isophorone diisocyanate.

Non-limiting examples of linker molecules include ethylene glycolbis (succinimidylsuccinate), disuccinimidyl suberate, 1,6-diisocyanatohexane, methylene bis-(4-cyclohexylisocyanate, glutaric dialdehyde, methylene-p-phenyl diisocyanate, and triethyl citrate.

Any suitable substrate may be used in the above methods. Preferably, the substrate includes at least one group that reacts with the isocyanate compound such as hydroxyl, amino, or carboxylate groups.

A substrate for use in target analyte detection is provided. The substrate comprises a surface modified by any of the above methods.

The substrate comprises a surface having a polymeric layer comprising free amino groups capable of binding said capture probes, and negatively charged ionic groups.

In another aspect, the substrate surface produces a background signal upon imaging using visual or fluorescent light having substantially reduced background signal relative to a substrate not having said polymeric layer.

In still yet another aspect, the substrate has a refractive index ranging from 1.400 to_1.900.

A kit is provided for detecting target analytes. The kit comprises any of the above substrates and substrates prepared by the above methods.

A method is provided for detecting one or more target analytes in a sample, the target analyte having at least two binding sites. The method comprises: (a) providing a substrate prepared by any one of the methods of the present invention, said substrate having at least one type of capture probes immobilized on a surface of the substrate, each type of capture probes specific for a target analyte; (b) providing at least one type of detection probe comprising a nanoparticle and a detector probe, the detector probe specific for a target analyte; (c)contacting the capture probes, the detection probes and the sample under conditions that are effective for the binding of the capture probes and detector probes to the specific target analyte to form an immobilized complex onto the surface of the substrate; (d) washing the surface of the substrate to remove unbound nanoparticles; and (e) observing for the presence or absence of the complex as an indicator of the presence or absence of the target molecule.

A method is provided for immobilizing a nanoparticle onto a surface. The method comprises the steps of: (a) providing a substrate having a surface comprising reactive moieties that reacts with amine groups and a nanoparticle having oligonucleotides bound thereto, at least a portion of the oligonucleotides have a amine group at an end not bound to the nanoparticle; and (b)
contacting the reactive moieties with the nanoparticle so as to immobilized the nanoparticles onto said surface.

In one aspect, the surface is a glass surface.

In another aspect, the surface has at least one group that reacts with the reactive intermediate. Representative examples of groups include hydroxyl, amino, or carboxylate group. A non-limiting example of agent includes 3-(isocyanatopropyl) triethoxysilane or 3-(isocyanatopropyl)dimethylmonoethoxysilane.

In another aspect, the oligonucleotides may be bound to the nanoparticle through a functional moiety such as a thiotic acid, alkyl thiol or disulfide group (e.g., epiandrosterone disulfide)

In another aspect, the reactive moieties comprise isocyanates, anhydrides, acyl halides, or aldehydes.

Kits are provided for preparing modified substrates. The kits may include optional reagents for silyating molecules and optional substrtes, buffers for carrying out assays including washing and binding steps.

In one aspect, a method is provided for immobilizing a molecule onto a glass surface.

A method is provided for immobilizing a molecule onto a surface, said method comprising the steps of:
(a) contacting Si(NCY)₄ wherein Y represents oxygen or sulfur with an agent so as to form a first reactive intermediate, said agent having a formula ii:

   (R₁)(R₂)(R₃)Si-X-Z ii

   wherein R₁, R₂ and R₃ independently represents C₁-C₆ alkoxy, C₁-C₆ alkyl, phenyl, or aryl substituted with one or more groups selected from the group consisting of C₁-C₆ alkyl and C₁-C₆ alkoxy; X represents linear or branched C₁-C₂₀ alkyl or aryl substituted with one or more groups selected from the group consisting of C₁-C₆ alkyl and C₁-C₆ alkoxy, optionally substituted with one or more heteroatoms comprising oxygen, nitrogen, or sulfur; and Z represents a hydroxy or amino group, with the proviso that at least one of R₁, R₂ or R₃ represents C₁-C₆ alkoxy;
(b) contacting the first reactive intermediate with a molecule so as to form a second reactive intermediate;
(c) contacting the second reactive intermediate with said surface so as to immobilized the molecule onto said surface. The method allows for the production of branched captured molecules structures such as branched oligonucleotides on a surface which is useful for enhancing detection of target analytes such as nucleic acids.

In one aspect, a method is provided for immobilizing a molecule onto a glass surface.

A compound is provided having the formula iii:

(R₁)(R₂)(R₃)Si-X-NHCYL-M iii

wherein R₁, R₂ and R₃ independently represents C₁-C₆ alkoxy, C₁-C₆ alkyl, phenyl, or aryl substituted with one or more groups selected from the group consisting of C₁-C₆ alkyl and C₁-C₆ alkoxy; X represents linear or branched C₁-C₂₀ alkyl or aryl substituted with one or more groups selected from the group consisting of C₁-C₆ alkyl and C₁-C₆ alkoxy, optionally substituted with one or more heteroatoms comprising oxygen, nitrogen, or sulfur; Y represents oxygen or sulfur; L represents a linking group; and M represents a molecule, with the proviso that at least one of R₁, R₂, or R₃ represent C₁-C₆ alkoxy.

A compound is provided having a formula iv:

(R₁)(R₂)(R₃)Si-X-Z-CYNH-Si (NCY)₃ iv

wherein R₁, R₂ and R₃ independently represents C1-C6 alkoxy, C₁-C₆ alkyl, phenyl, or aryl substituted with one or more groups selected from the group consisting of C₁-C₆ alkyl and C₁-C₆ alkoxy; X represents linear or branched C₁-C₂₀ alkyl or aryl substituted with one or more groups selected from the group consisting of C₁-C₆ alkyl and C₁-C₆ alkoxy, optionally substituted with one or more heteroatoms comprising oxygen, nitrogen, or sulfur; Y represents oxygen or sulfur; and Z represents oxygen or NH, with the proviso that at least one of R₁, R₂, or R₃ represents C₁-C₆ alkoxy.

A compound is provided having a formula v:

(R₁)(R₂)(R₃)Si-X-Z-CYNH-Si (NHCYL-M)₃ v

wherein R₁, R₂ and R₃ independently represents C₁-C₆ alkoxy, C₁-C₆ alkyl, phenyl, or aryl substituted with one or more groups selected from the group consisting of C₁-C₆ alkyl and C₁-C₆ alkoxy; X represents linear or branched C₁-C₂₀ alkyl or aryl substituted with one or more groups selected from the group consisting of C₁-C₆ alkyl and C₁-C₆ alkoxy, optionally substituted with one or more heteroatoms comprising oxygen, nitrogen, or sulfur; Y represents oxygen or sulfur; L represents a linking group; and Z represents oxygen or NH; and M represents a molecule, with the proviso that at least one of R₁, R₂, or R₃ represent C₁-C₆ alkoxy.

A compound is provided having a formula vi:

((R₁)(R₂)(R₃)Si-X-Z-CYNH)₂-Si (NCY)₂ vi

wherein R₁, R₂ and R₃ independently represents C₁-C₆ alkoxy, C₁-C₆ alkyl, phenyl, or aryl substituted with one or more groups selected from the group consisting of C₁-C₆ alkyl and C₁-C₆ alkoxy; X represents linear or branched C₁-C₂₀ alkyl or aryl substituted with one or more groups selected from the group consisting of C₁-C₆ alkyl and C₁-C₆ alkoxy, optionally substituted with one or more heteroatoms comprising oxygen, nitrogen, or sulfur; Y represents oxygen or sulfur; and Z represents oxygen or NH, with the proviso that at least one of R₁, R₂, or R₃ represents C₁-C₆ alkoxy.

A compound is provided having a formula vii:

((R₁)(R₂)(R₃)Si-X-Z-CYNH)₂Si (NHCYL-M)₂ vii

wherein R₁, R₂ and R₃ independently represents C₁-C₆ alkoxy, C₁-C₆ alkyl, phenyl, or aryl substituted with one or more groups selected from the group consisting of C₁-C₆ alkyl and C₁-C₆ alkoxy; L represents a linking group; X represents linear or branched C₁-C₂₀ alkyl or aryl substituted with one or more groups selected from the group consisting of C₁-C₆ alkyl and C₁-C₆ alkoxy, optionally substituted with one or more heteroatoms comprising oxygen, nitrogen, or sulfur; Y represents oxygen or sulfur; and Z represents oxygen or NH; and M represents a molecule, with the proviso that at least one of R₁, R₂, or R₃ represents C₁-C₆ alkoxy.

### DESCRIPTION OF THE DRAWINGS

Figure 1 is a scheme that illustrates one aspect. The scheme shows the modification of a molecule such as an oligonucleotide modified at either a 3'-amino or 5'-amino to produce a silylated DNA intermediate. This silylated intermediate is then spotted onto a surface of a substrate, e.g., glass substrate and washed.
Figure 2 illustrates spot morphology after spotting a substrate with a DMF solution containing a silylated DNA in water or DMF. Branching and spreading of the spot was observed with the aqueous solution.
Figure 3 illustrates spot morphology using a DMF solution containing a silyated DNA spotted on a overhydrated substrate. Branching of the spot was observed with the over hydrated substrate.
Figure 4 illustrates spot morphology with an aqueous solution containing no silylated DNA (blank control) and with silylated DNA (silyl).

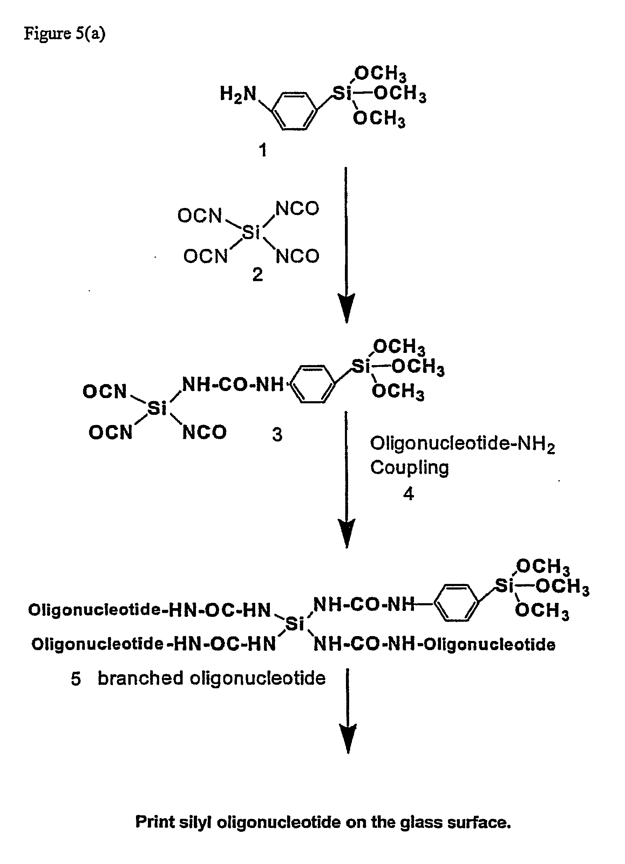

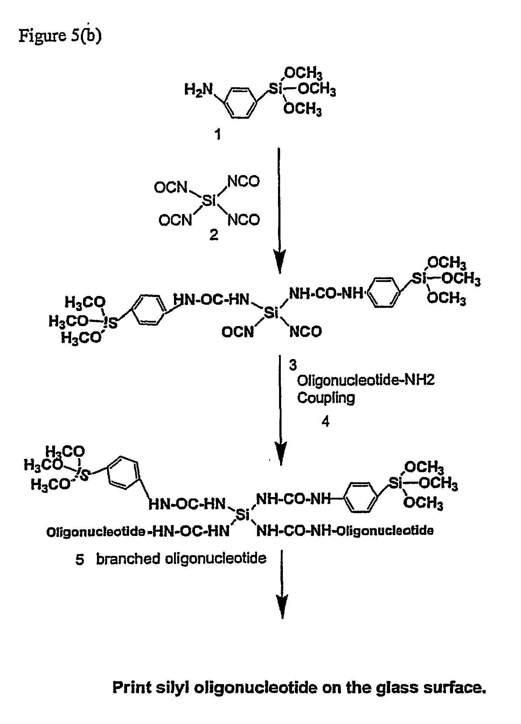

Figure 5 is (a) a scheme that illustrates another aspect. The scheme shows the coupling of a tetraisocyanatosilane with a 1-amino-4-triethoxysilylbenzene to form a first reactive intermediate 4. The reactive intermediate is then coupled to a oligonucleotide having a free 3' or 5'-amino group to silylated DNA intermediate as a second reactive intermediate containing three molecules bound thereto. This silylated intermediate is then spotted onto a surface of a substrate, e.g., glass substrate. In part (b), a scheme is provided that illustrates another embodiment of the invention. The scheme shows the coupling of a tetraisocyanatosilane with a 1-amino-4-triethoxysilylbenzene to form a first reactive intermediate 4. The reactive intermediate is then coupled to a oligonucleotide having a free 3' or 5'-amino group to silylated DNA intermediate as a second reactive intermediate containing two molecules bound thereto.
Figure 6 illustrates the results of detection of M13 capture sequences using a DNA array chip prepared as described in Example 1 (method no. 1)..In plate no. 1, a non-complementary nanoparticle-labeled oligonucleotide probe was used. In plates nos. 2 and 3, a specific complementary nanoparticle-labeled oligonucleotide probe was used. As expected, the plates using the specific complementary probes showed detection events. See Example 3.
Figure 7 illustrates the results of detection of Factor V target sequence using a sandwich hybridization assay. A DNA array chip was prepared as described in Example 1 (method no. 1) using Factor V capture probe. The DNA chip performed as expected. See Example 4.
Figure 8 illustrates the results of detection of MTHFR target sequence using a DNA array chip prepared as described in Example 1 (method no. 1). The DNA chip performed as expected. Plate No. 1 shows that the detection probe does not hybridized above its melting temperature. Plate No. 2 showed detection of a 100mer MTHFR synthetic target. Plate No. 3 showed detection of a MTHFR PCR product. See Example 5. See Example 5.
Figure 9 illustrates the results of detection of Factor V target sequence using a DNA array chip prepared as described in Example 1 (method no. 1). The DNA chip performed as expected. No non-specific background noise was observed. See Example 6.
Figure 10 illustrates the results of detection of Factor V target sequence using a DNA array chip prepared as described in Example 1 (method no. 1). The DNA chip performed as expected. The probes reacted specifically to the target sequence and no cross-hybridization between the probes and targets was observed. See Example 7.
FIG 11: Shows the schematic diagram for making polymer coated slides and DNA printing on EGS linker slides.
FIG 12: Shows the chemistry model picture of polymer and EGS modifications on glass surface.
FIG 13: Shows the stepwise diagram of making polymer coated slides with the different polymers and linkers.
FIG 14: Shows the graphical diagram that compares a polymer coated slide and a dendrimer slide.
FIG 15: Shows arrayed CY3-oligonucleotides intensity comparison on polymer coated slide and dendrimer slide.
FIG 16a: Shows the polymer coated slide performance using positive control and factor V target.
FIG 16b: Shows the performance of tris(2-aminoethylene) modified slide performance using gold nanoparticle probes.
FIG 16c: Shows polymer-95 coated slide performance in an assay format using gold nanoparticle probes
FIG 16d: Shows background after spreading gold nanoparticle probes on dendrimer slide and amplify with silver.
FIG 16e: Shows background after spreading gold nanoparticle probes on polymer-95 slide and amplify with silver.
FIG 16f: Shows the target detection on polymer-95 slide using different target concentrations in each well.
FIG 16g: Shows multiplex detection on polymer-95 modified slide.

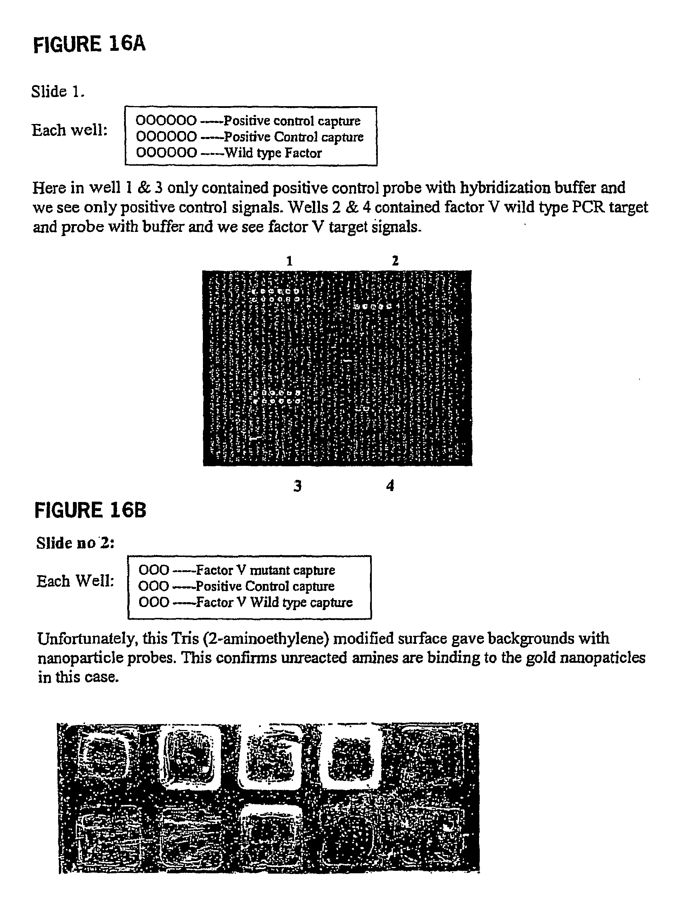

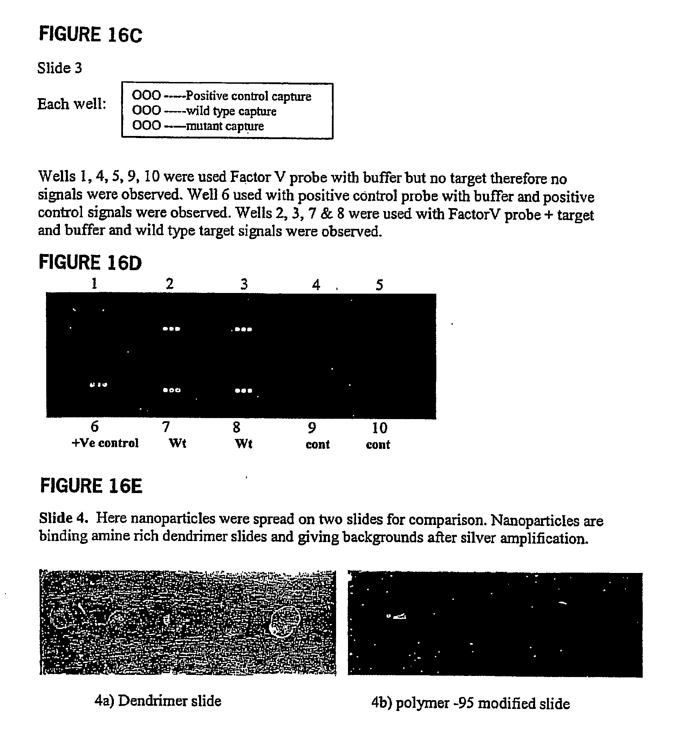

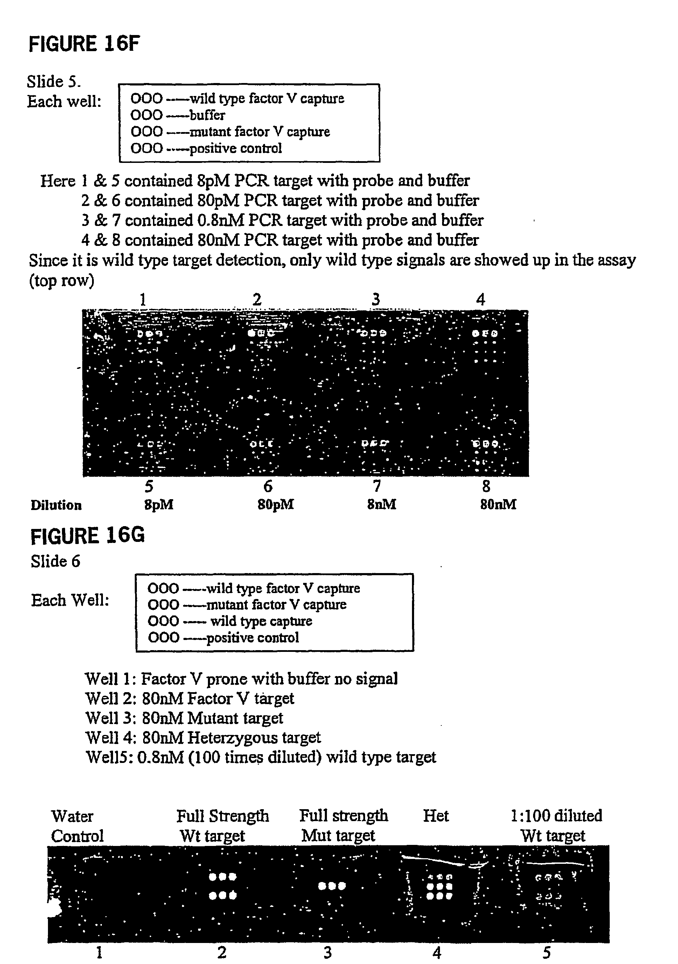

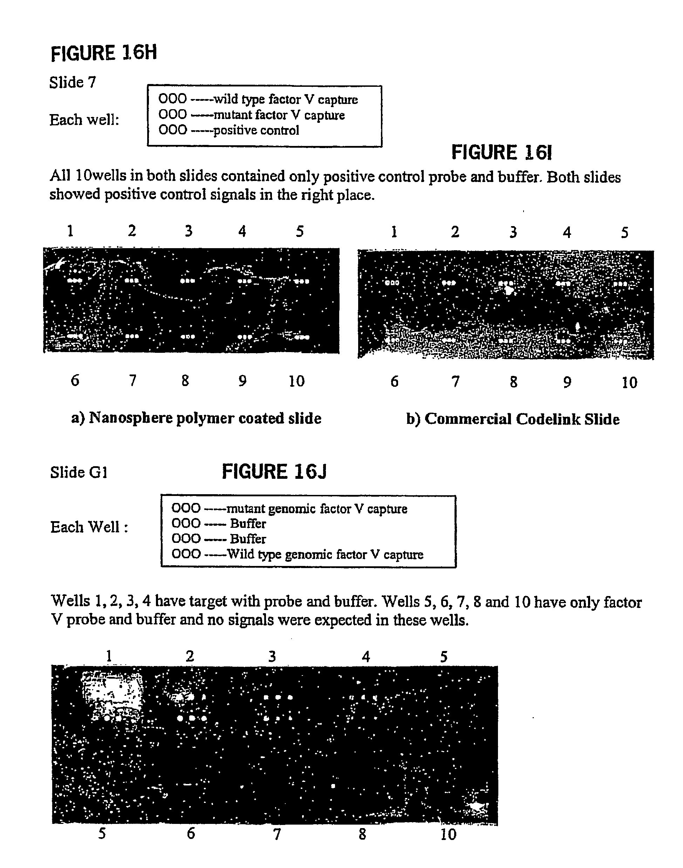

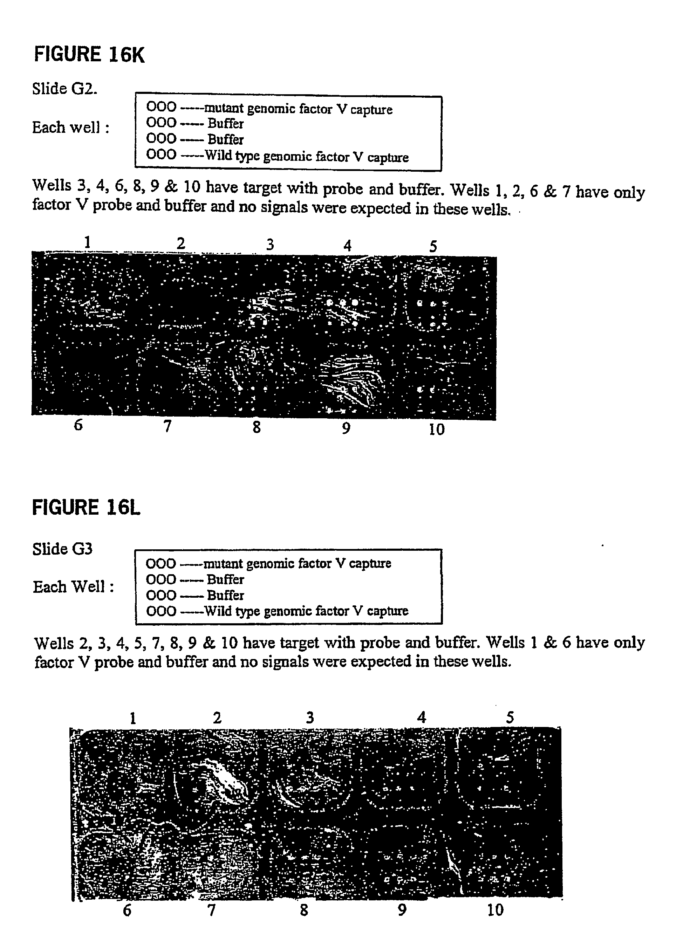

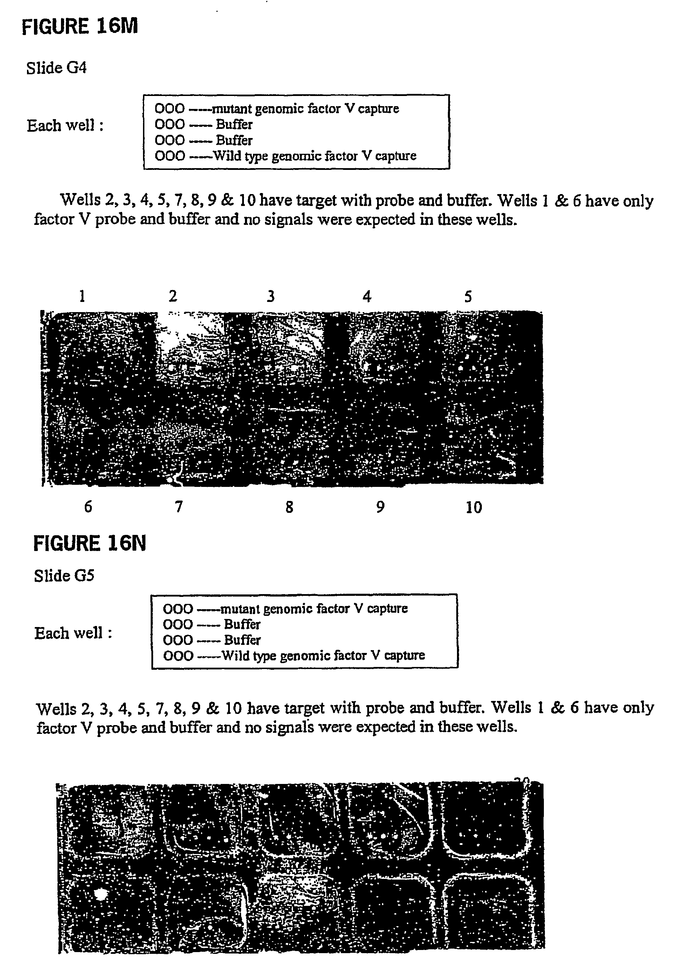

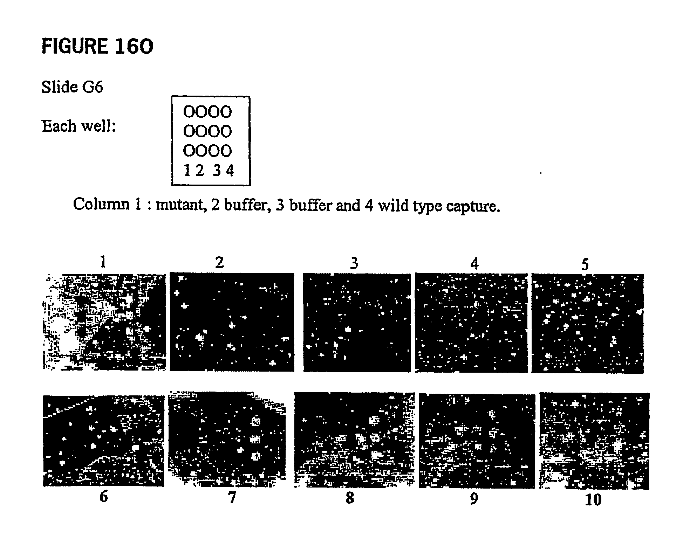

FIG 16 h: Shows positive control detection on polymer-95 slide.
FIG 16i: Shows positive control detection on commercial slide for comparison.
FIG 16j: Shows target detection on polymer-95 slide in top rows and controls in lower rows.
FIG 16k: Shows target detection on polymer-95 slide in wells 2, 3, 4, 5, 7, 8, 9 and 10. All wells have PCR DNA target; wells 1 and 6 do not have target.
FIG 16l: Shows target detection on polymer-95 slide using silver stained gold nanoparticles.
FIG 16m: Shows target detection on polymer-95 slide using silver stained gold nanoparticles.
FIG 16n: Shows target detection on polymer-95 slide using silver stained gold nanoparticles.
FIG 16o: Shows genomic target detection on polymer-95 slide. Signal was observed in wells 1, 7, 8, 9 clearly signals were observed.

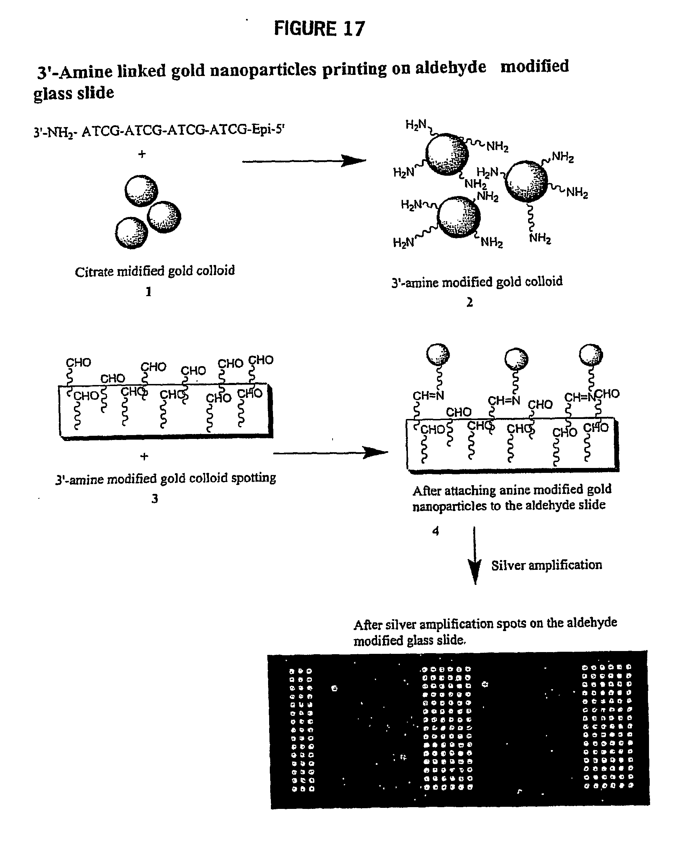

FIG 17: Shows the immobilization of gold nanoparticles on aldehyde modified surface using silver amplification.

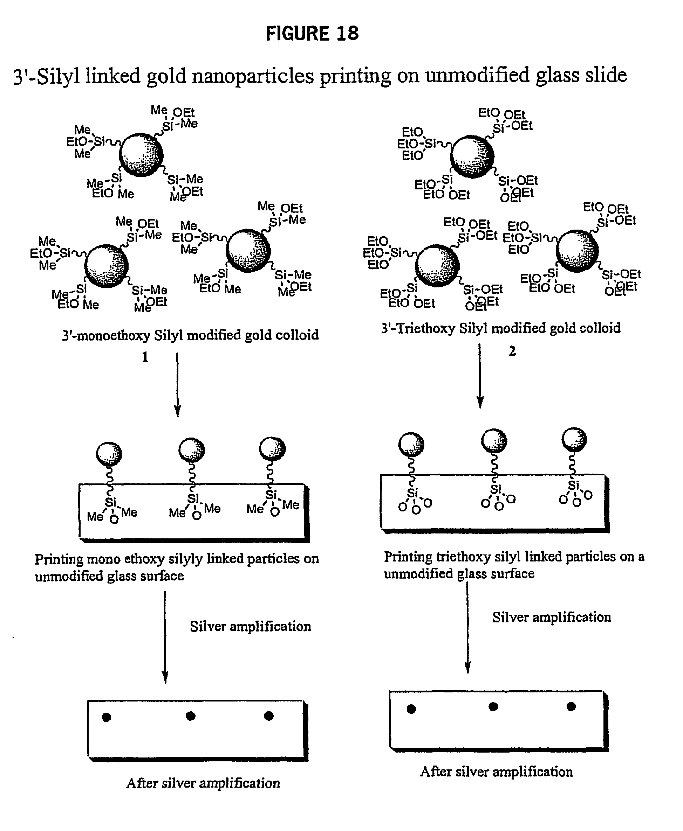

FIG 18: Shows the schematic diagram of attaching silyl linked gold nanoparticles to unmodified surface and silver amplification.

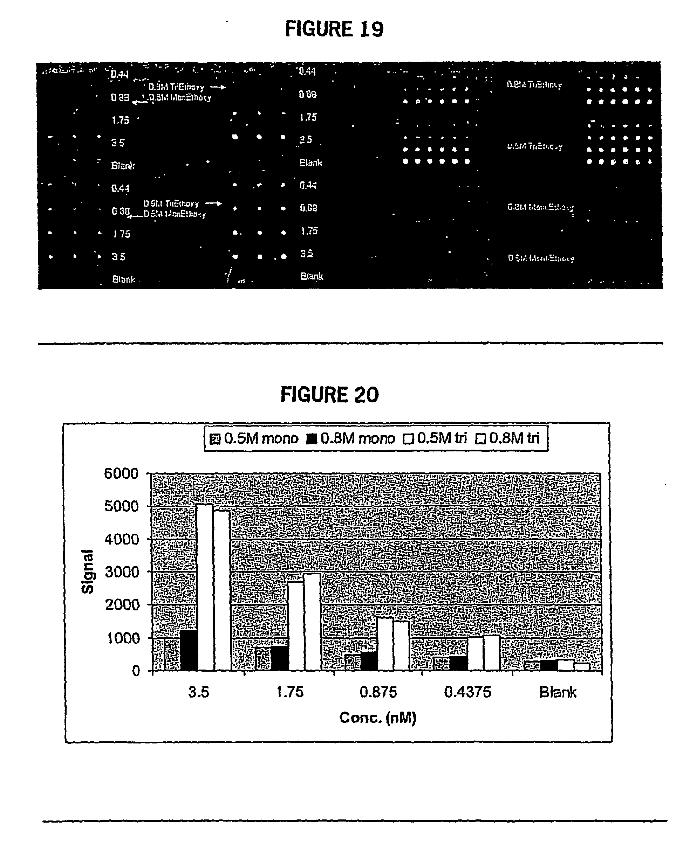

FIG 19: Shows attaching different types of silyl linked gold nanoparticles printed on an unmodified surface using silver amplification.
FIG 20: Shows the difference in signal intensity based on salt content in gold nanoparticle preparation.

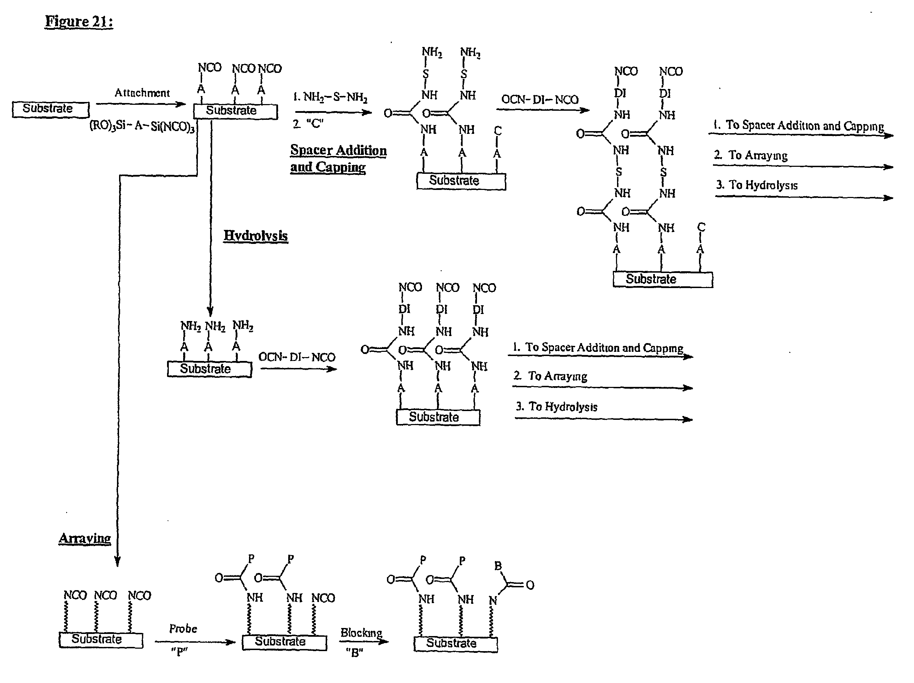

Figure **21** shows schematically the chemical reactions involved in the production of a layer on top of a substrate surface. The first step of the scheme shows the attachment of the bifunctional disilyl moiety to the surface of the substrate which is labeled "A" on top of the surface. Once the surface is functionalized with "A" the available reactive isocyanate groups can subsequently be reacted with a nucleophile for printing of a biomolecule "P", adding a spacer group "S" or hydrolysis to produce a primary amine on the surface. Once the isocyanate groups are printed with a biomolecule "P", the surface is then blocked with a nucleophilic molecule "B". After the isocyanate groups of "A" are reacted with a multi-functional spacer group "S", such as a di or tetraamine, any residual isocyanate groups are capped "C" with a nucleophilic molecule. After capping, another multifunctional molecule such as a diisocyanate "DI" can be added to produce available reactive isocyanates on the surface. If the isocyanate is on the surface it is considered the linker group "L". The new surface "Substrate-A-L-DI" has multiple pathways again and can be printed, hydrolyzed or an additional linker added followed by a diisocyanate. After the last spacer "S" is added and reacted with a diisocyanate or other bifunctional electrophile, this produces the final surface having free isocyanates. This last bifunctional electrophile or mixture of bifunctional molecules to be added is a linker "L" which can then be used for printing of biomolecules. An isocyanate surface which has been hydrolyzed to produce the primary amine groups on the surface is now open for addition of a bifunctional electrophile "DI" and can then go through all the combinations. See Exmple 14.

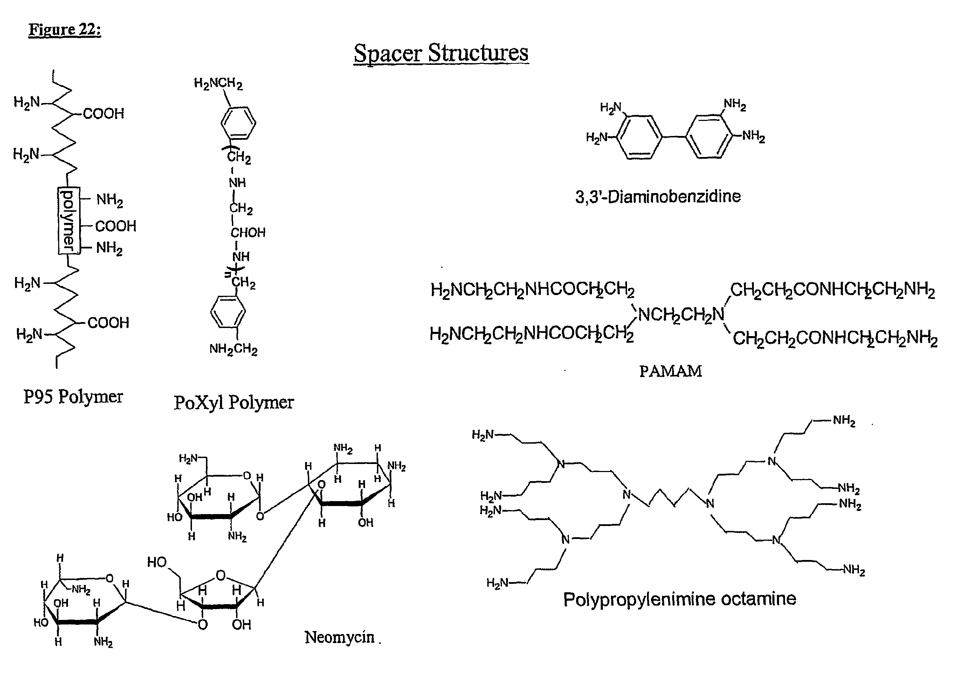

Figure **22****.** shows the chemical structures for the spacer molecules.

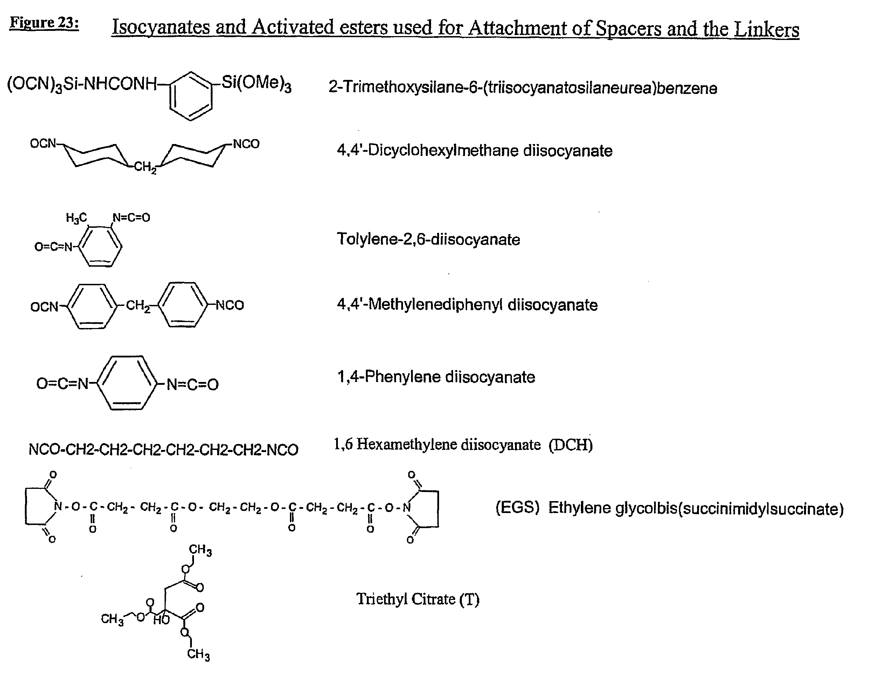

Figure **23** shows the isocyanate and activate ester chemical structures.

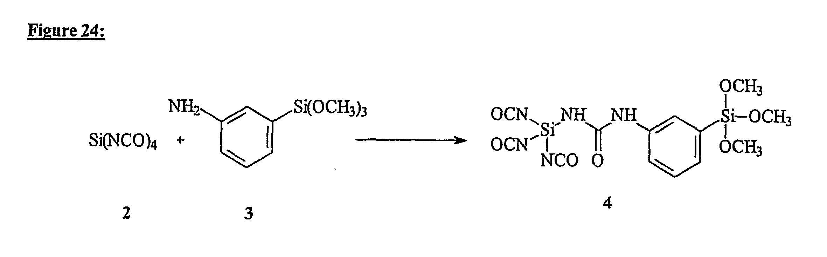

Figure **24****.** shows the synthesis of 2-trimethoxy-6-(triisocyanatosilaneurea)benzene **(4)**

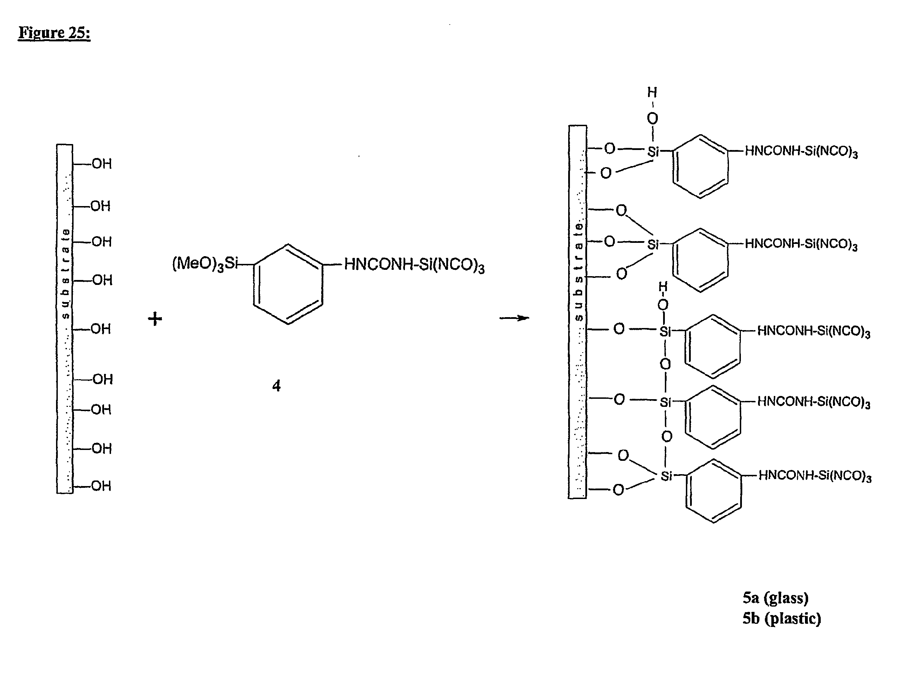

Figure **25****.** shows addition of the bifunctional disilyl **4** to the substrate surface to produce a coated substrate with free isocyanate groups on glass **(5a)** or plastic **(5b).**

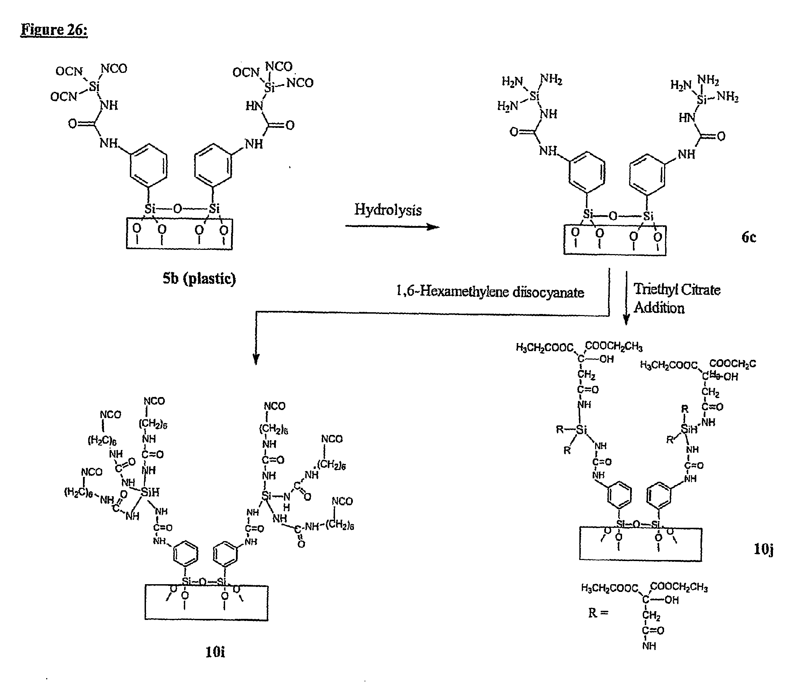

Figure **26****.** shows the hydrolysis of the isocyanate groups on the surface to give primary amine groups which then is attached a linker group "L". The first example is with 1,6-hexamethylene diisocyanate to give a surface with free isocyanate groups, and the second example is with the triester of citric acid to give an activated ester group on the surface available for printing.

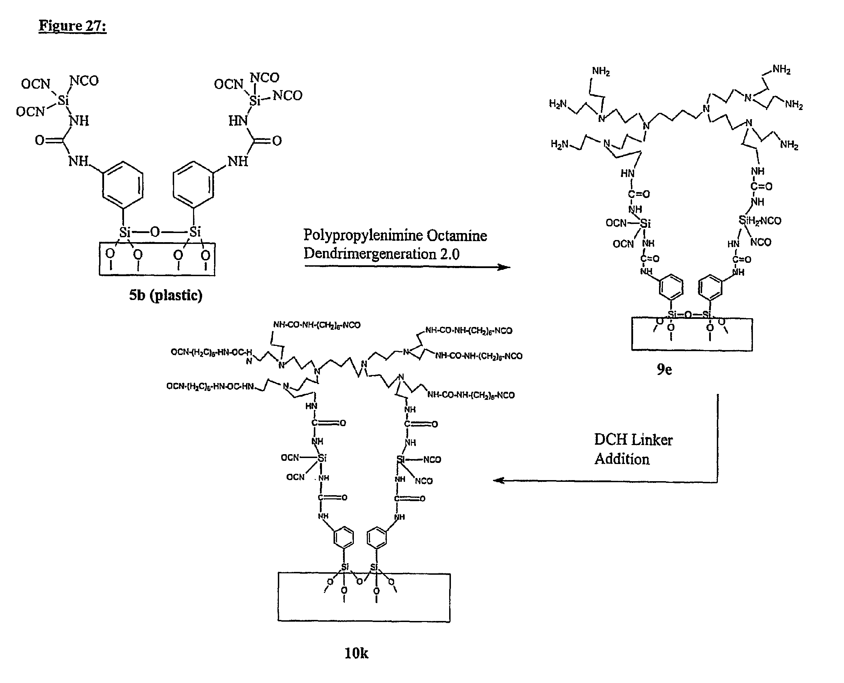

Figure **27****.** shows the addition of a spacer dendrimer followed by the addition of the linker 1,6-hexamethylene diisocyanate.

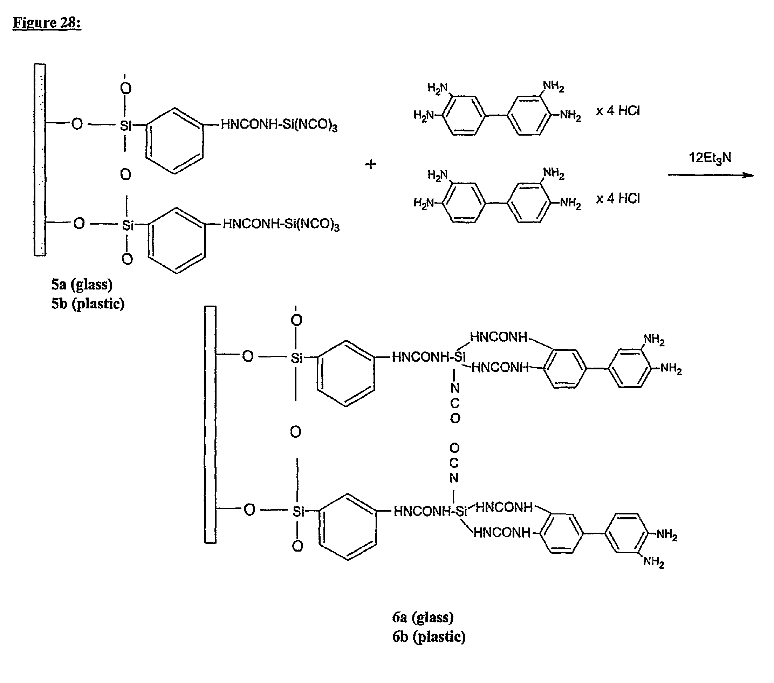

Figure **28****.** shows the addition of the spacer 3,3'-Diaminobenzidine.

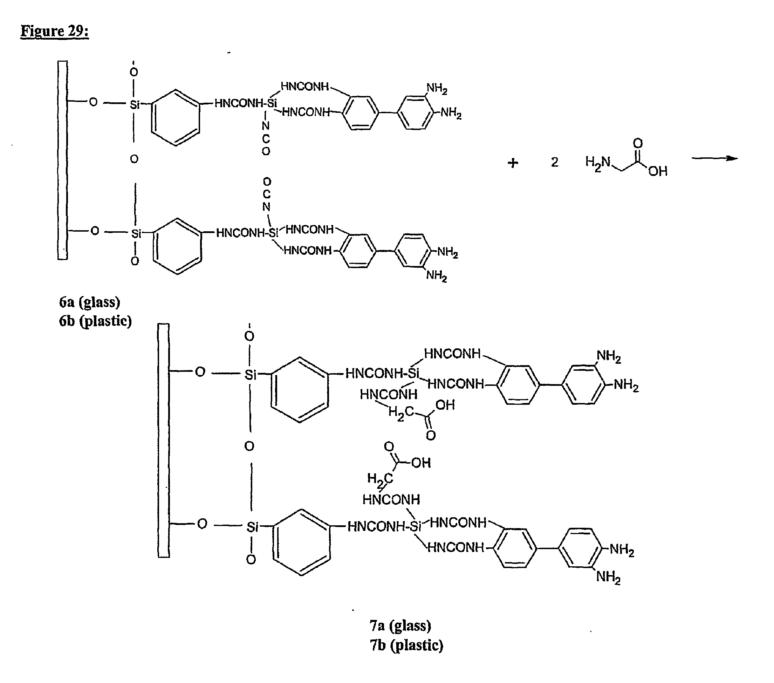

Figure **29****.** continued from Figure 28. shows the capping of isocyanates after the addition of the spacer.

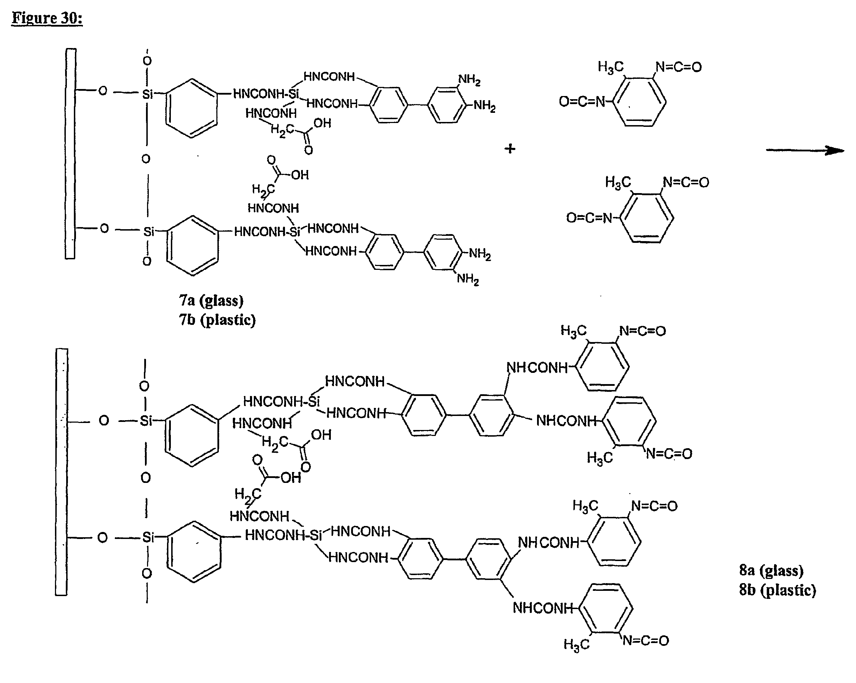

Figure **30****.** continued from Figure 29. shows the addition of diisocyanate, which could be arrayed or the process continued with the addition of another spacer.

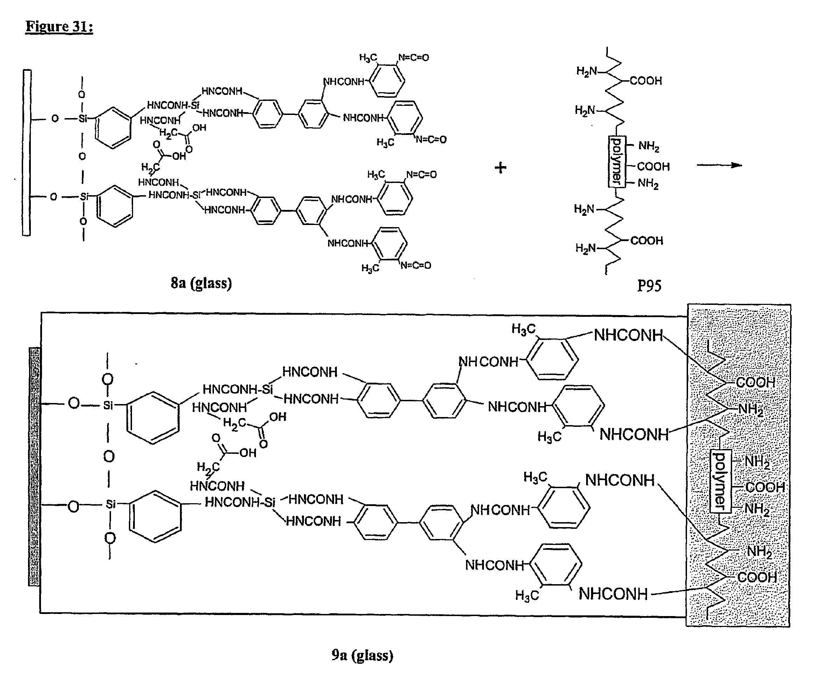

Figure **31****.** continued from Figure 30. shows the addition of P95 polymer to free isocyanates on glass substrate.

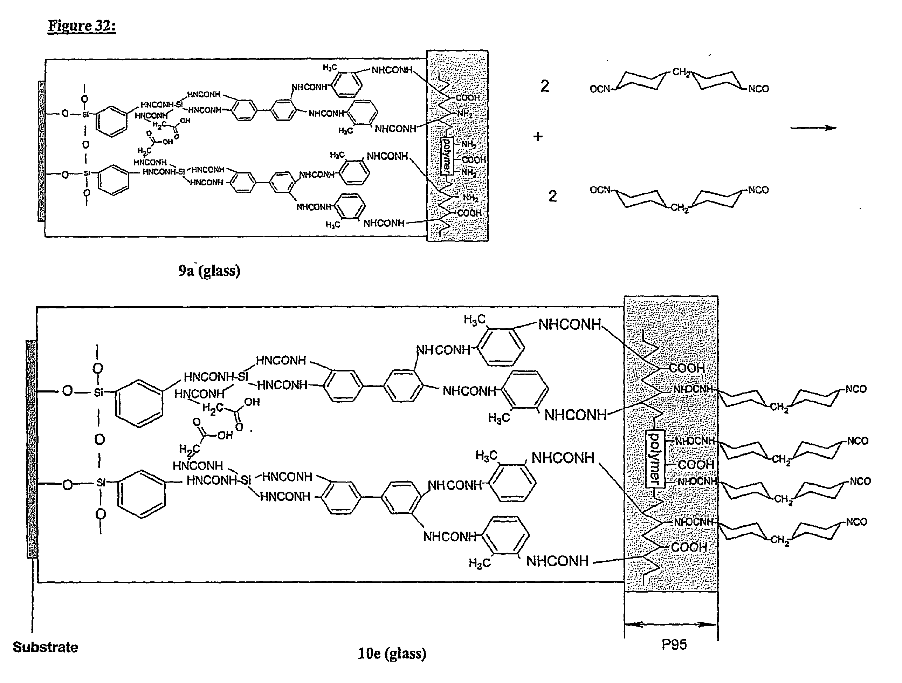

Figure **32****.** continued from Figure 31. shows the addition of the linker group 4,4'-Dicyclohexylmethane diisocyanate which can be printed or an additional spacer added.

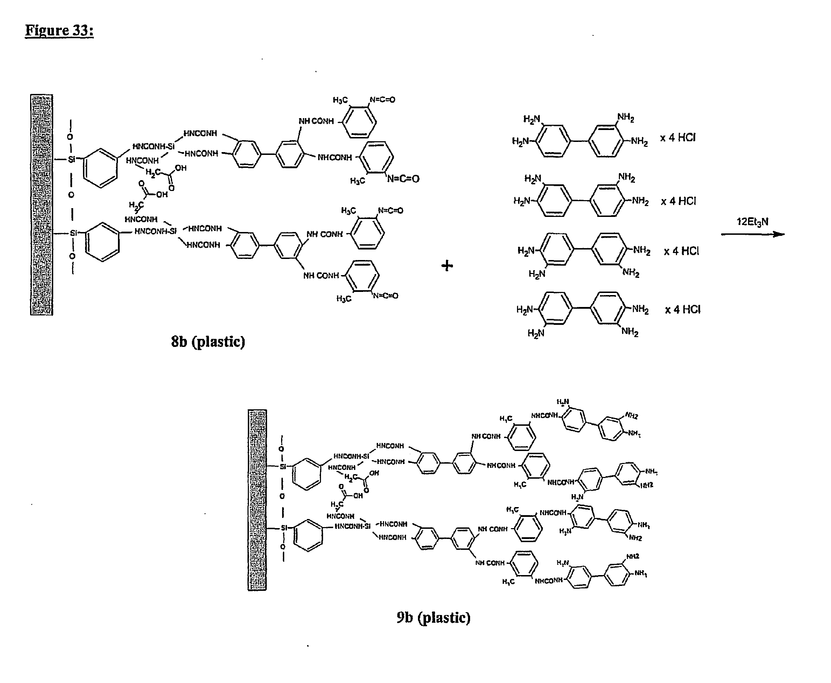

Figure **33****.** continued from Figure 29. shows the addition of the spacer 3,3'-Diaminobenzidine.

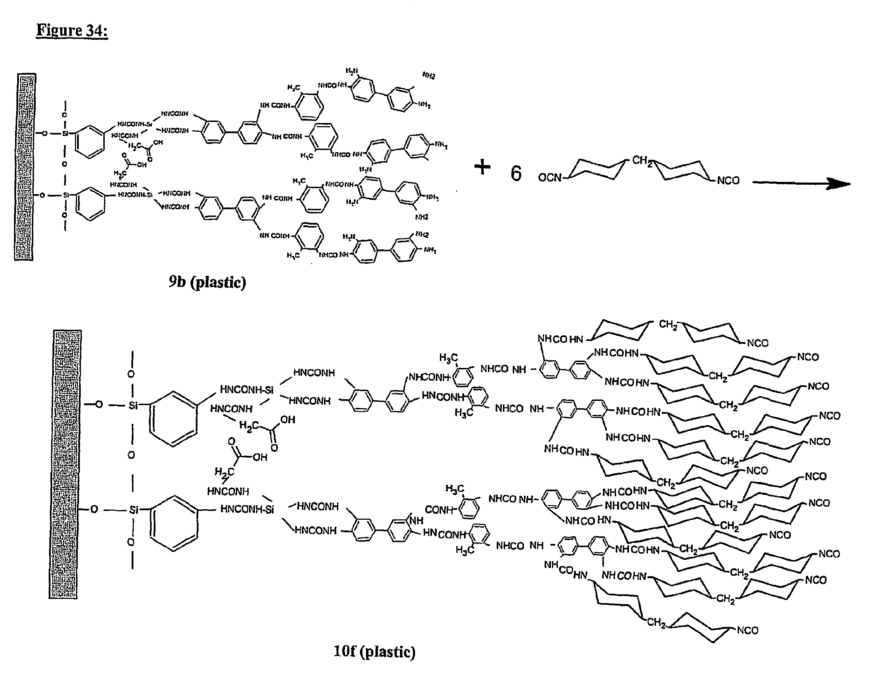

Figure **34****.** continued from Figure 31. shows the addition of the linker group 4,4'-Dicyclohexylmethane diisocyanate which can be printed or an additional spacer added.

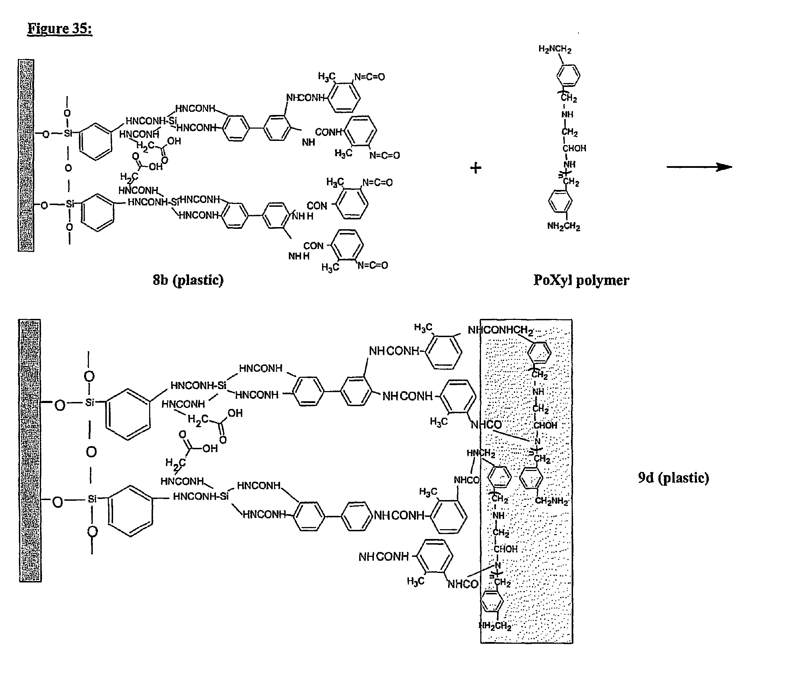

Figure **35****.** continued from Figure 29. shows the addition of PoXyl polymer as a spacer.

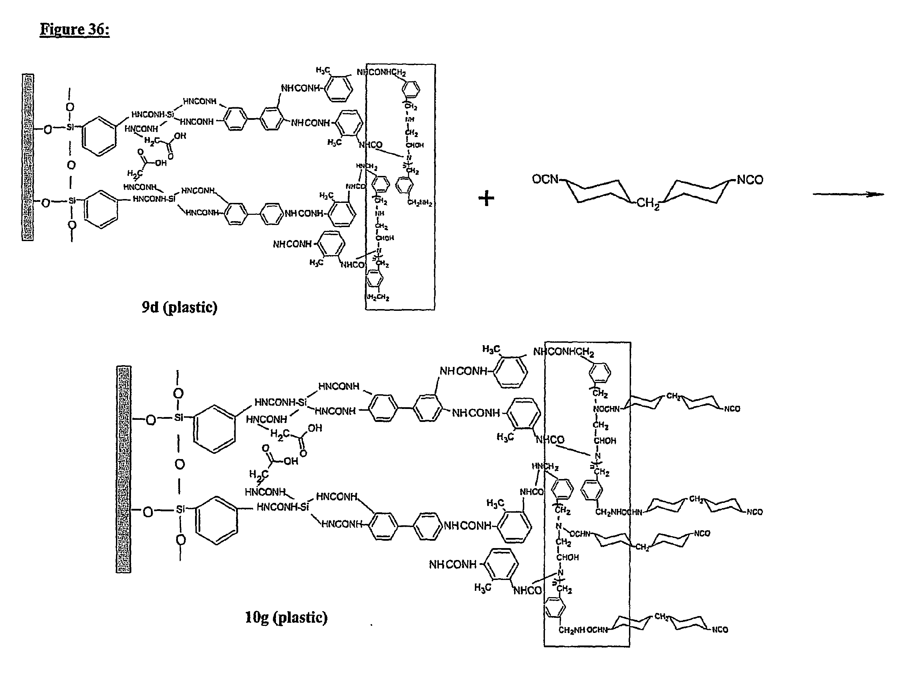

Figure **36****.** continued from Figure 35. shows the addition of 4,4'-Dicyclohexylmethane diisocyanate as a linker which can be printed or an additional spacer added.

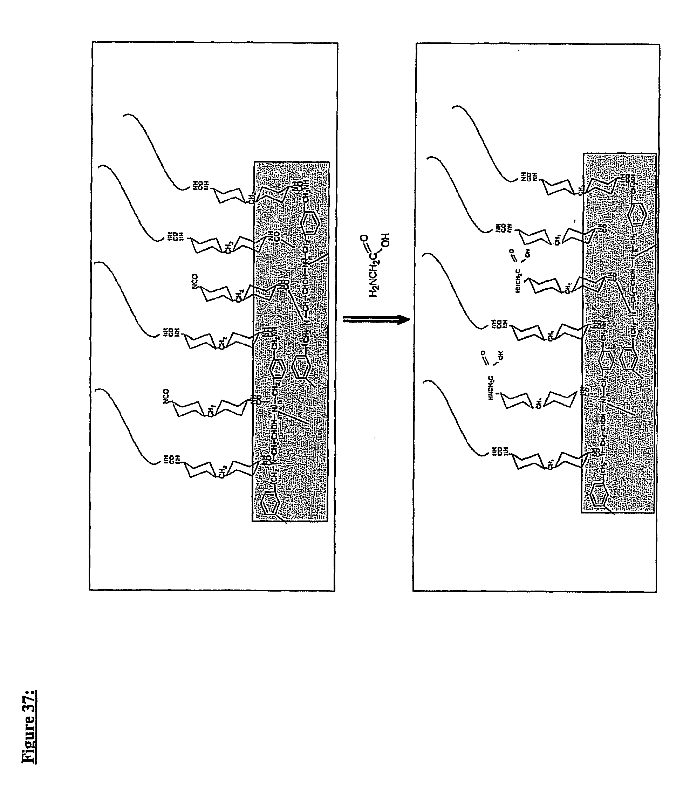

Figure **37****.** shows the addition of glycine to block any isocyanate on the surface after the substrate is arrayed with a biomolecule.

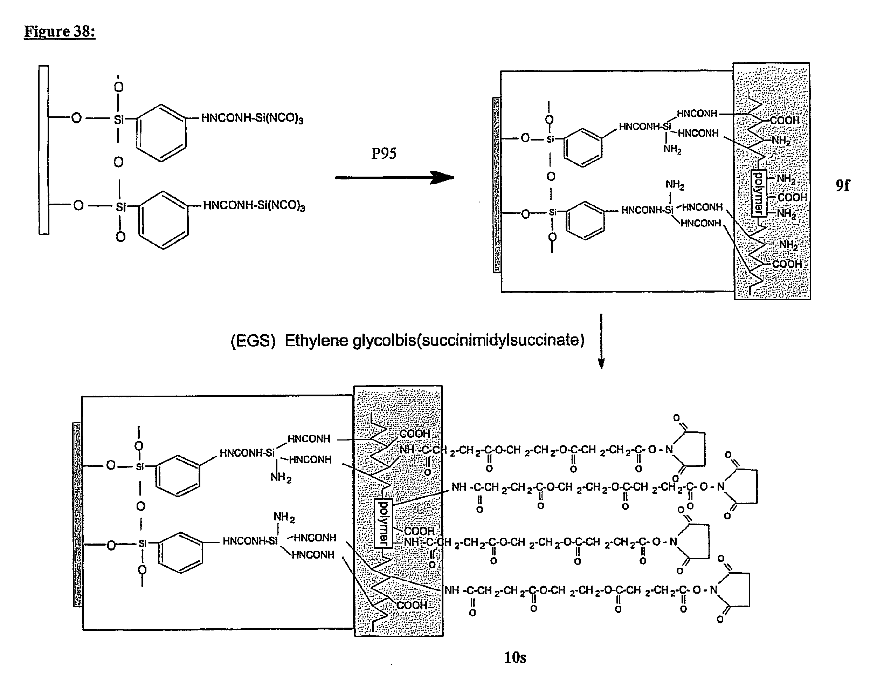

Figure **38****.** continued from Figure 25. shows the addition of the spacer P95 to the anchor group, followed by the addition of ethylene glycolbis(succinimidylsuccinate) as a linker.

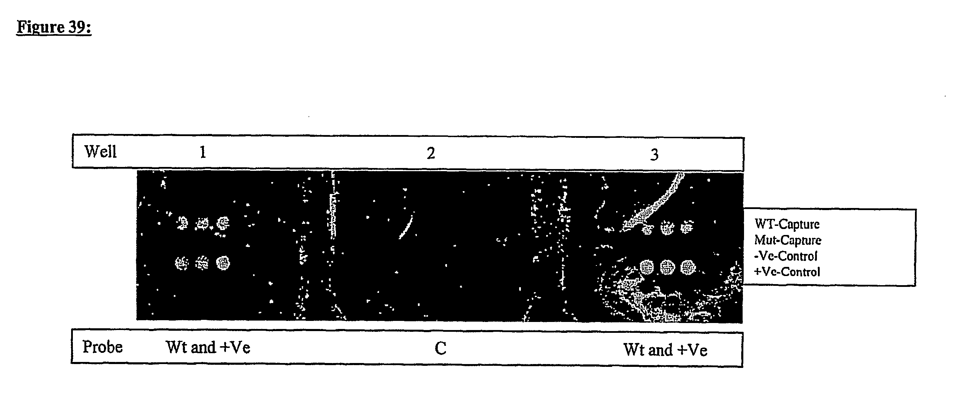

Figure **39****.** shows image of glass substrate **(10i)** with 1,6-hexamethylene linker: Substrate **10i** is arrayed with three columns and four rows in each of the three wells. Each of the three spots in any row has the same capture arrayed on the slide. The type of capture by row is wild type capture, mutant capture, negative control and positive control capture DNA sequences from top to bottom. Wells 1 and 3 show genomic wild type target with positive and wild type nanoparticle probes binding to both the positive and genomic captures. Well 2 shows nanoparticle probes without any genomic DNA, demonstrating that there is no nonspecific binding to the substrate.

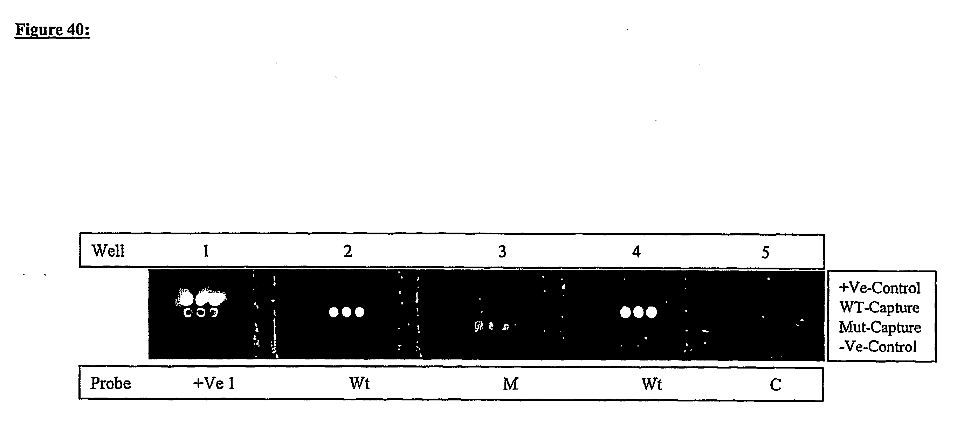

Figure **40****.** shows image of glass substrate with triethyl citrate and 1,6-hexamethylene diisocyanate linker mixture **(10p):** Substrate **10p** is arrayed with three columns and four rows in each of the five wells. Each of the three spots in any row has the same capture arrayed on the slide. The type of capture by row is positive control, wild type capture, mutant capture, and negative control capture DNA sequences from top to bottom. Well 1 shows positive nanoparticle control probes, with no DNA target, binding to positive control captures. Wells 2 and 4 show PCR wild type target with wild type nanoparticle probes binding to the wild type captures. Well 3 shows mutant nanoparticle probes with mutant DNA target binding to the mutant captures only. The control, well 5 contained wild type and mutant nanoparticle probes with no DNA targets and demonstrate no non-specific binding of the nanoparticle probes.

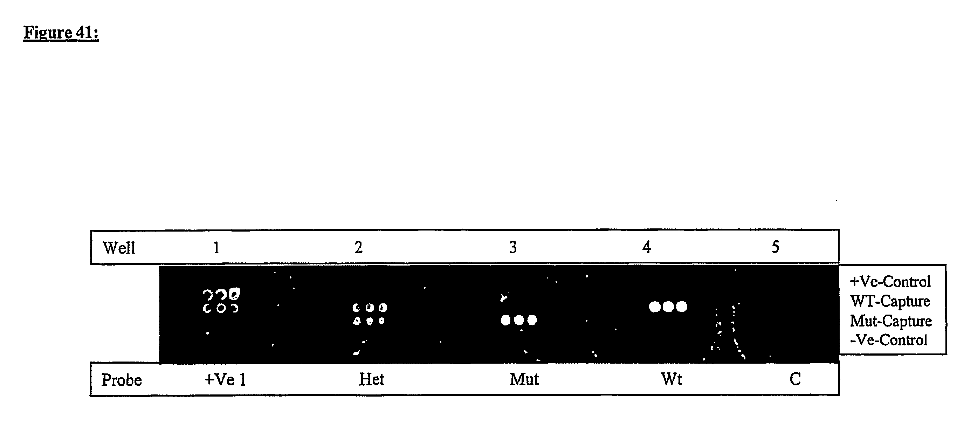

Figure **41****.** shows image of glass substrate with triethyl citrate linker **(10b):** Substrate **10b** is arrayed with three columns and four rows in each of the five wells. Each of the three spots in any row has the same capture arrayed on the slide. The type of capture by row is positive control, wild type capture, mutant capture, and negative control capture DNA sequences from top to bottom. Well 1 shows positive nanoparticle control probes, with no PCR target, binding to positive control captures. Well 2 shows PCR heterozygous DNA type target with wild type and mutant nanoparticle probes binding to both the wild type and mutant captures. Well 3 shows mutant nanoparticle probes with mutant DNA target binding to the mutant captures only. Well 4 shows wild type nanoparticle probes with wild type DNA target binding to the wild captures only. The control, well 5 contained wild type and mutant nanoparticle probes with no DNA targets and demonstrate no non-specific binding of probes.

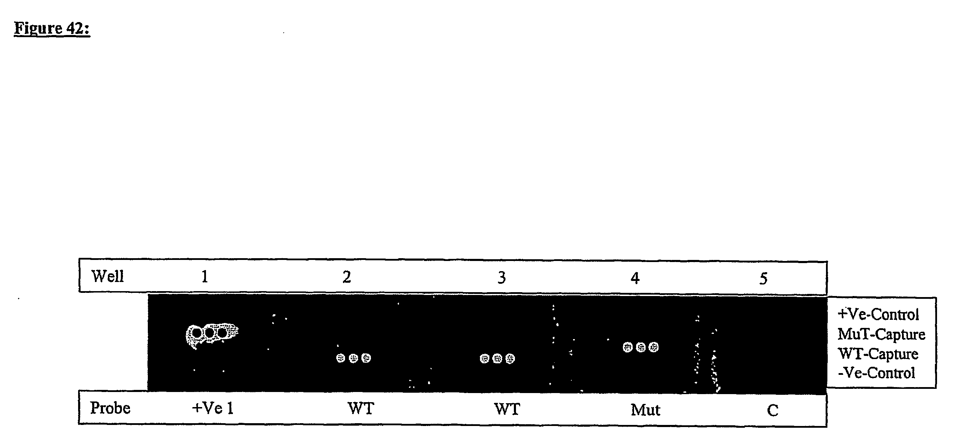

Figure **42****.** shows glass substrate with Methylenediphenyl diisocyanate linker **(10n):** Substrate **10n** is arrayed with three columns and four rows in each of the five wells. Each of the three spots in any row has the same capture arrayed on the slide. The type of capture by row is positive control, mutant type capture, wild type capture, and negative control capture DNA sequences from top to bottom. Well 1 shows positive nanoparticle control probes, with no DNA target, binding to positive control captures. Wells 2 and 3 show genomic wild type DNA target with wild type nanoparticle probes binding to the wild type and mutant captures. Well 4 shows mutant type nanoparticle probes with mutant type DNA target binding to the mutant captures only. The control, well 5 contained wild type and mutant nanoparticle probes with no DNA targets and demonstrate no non-specific binding of probes.

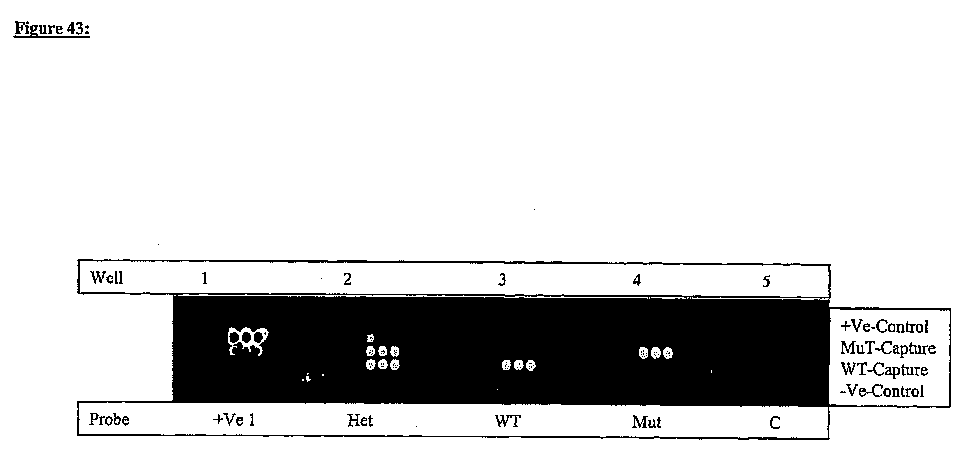

Figure **43****.** shows glass substrate with triethyl citrate and methylenediphenyl diisocyanate linkers **(10r):** Substrate **10r** is arrayed with three columns and four rows in each of the five wells. Each of the three spots in any row has the same capture arrayed on the slide. The type of capture by row is positive control, mutant type capture, wild type capture, and negative control capture DNA sequences from top to bottom. Well 1 shows positive nanoparticle control probes, with no DNA target, binding to positive control captures. Well 2 shows PCR heterozygous type DNA target with wild type and mutant nanoparticle probes binding to both the wild type and mutant captures. Well 3 shows wild type nanoparticle probes with wild type DNA target binding to the wild type captures only. Well 4 shows mutant type nanoparticle probes with mutant type DNA target binding to the mutant captures only. The control, well 5 contained wild type and mutant nanoparticle probes with no DNA targets and demonstrate no non-specific binding of probes.

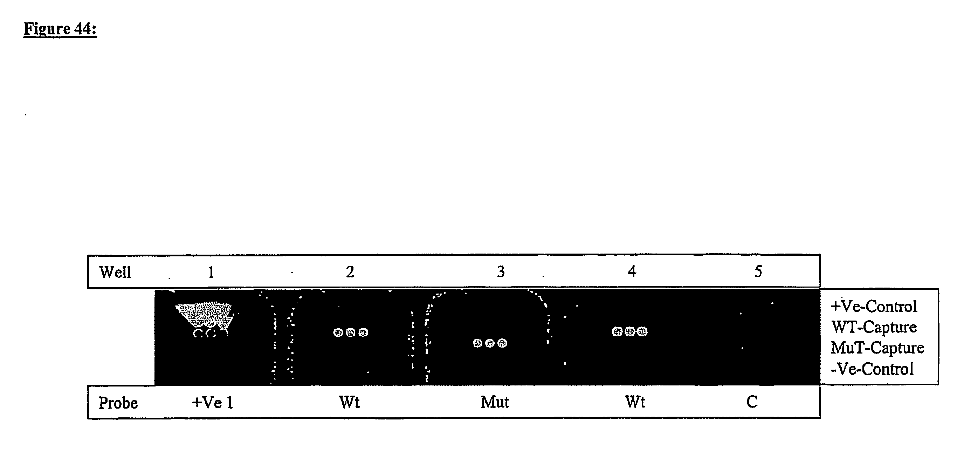

Figure **44****.** shows glass substrate with 4,4'-Dicyclohexyl methane diisocyanate linker **(10m):** Substrate **10m** is arrayed with three columns and four rows in each of the five wells. Each of the three spots in any row has the same capture arrayed on the slide. The type of capture by row is positive control, wild type capture, mutant capture, and negative control capture DNA sequences from top to bottom. Well 1 shows positive nanoparticle control probes, with no DNA target, binding to positive control captures only. Wells 2 and 4 show PCR wild type target with wild type nanoparticle probes binding to the wild type captures only. Well 3 shows mutant nanoparticle probes with mutant DNA target binding to the mutant captures only. The control, well 5 contained wild type and mutant nanoparticle probes with no DNA targets and demonstrates no non-specific binding of the probes

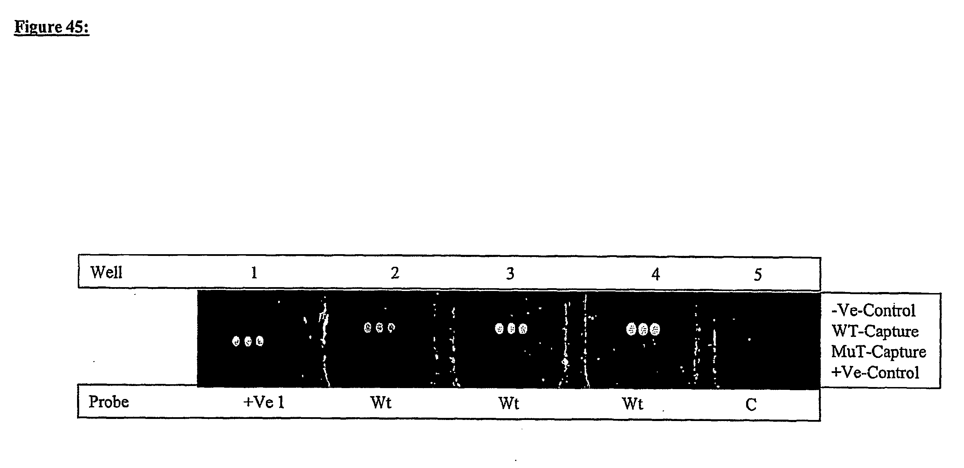

Figure **45****.** shows glass substrate with 1,6-Hexamethylene diisocyanate linker **(10L):** Substrate **10L** is arrayed with three columns and four rows in each of the five wells. Each of the three spots in any row has the same capture arrayed on the slide. The type of capture by row is negative control, mutant type capture, wild type capture, and positive control capture DNA sequences from top to bottom. Well 1 shows positive nanoparticle control probes diluted 2 logs compared to positive control probe concentration in Figure 40. DNA target was not used and binding to the positive control captures was demonstrated. Wells 2, 3 and 4 show PCR wild type target with wild type nanoparticle probes binding to the wild type captures only. Well 4 contained the highest concentration of target with 3 and 2 each containing a log reduction in target respectively. The control, well 5 contained wild type and mutant nanoparticle probes with no DNA targets and demonstrates no non-specific binding of the probes.

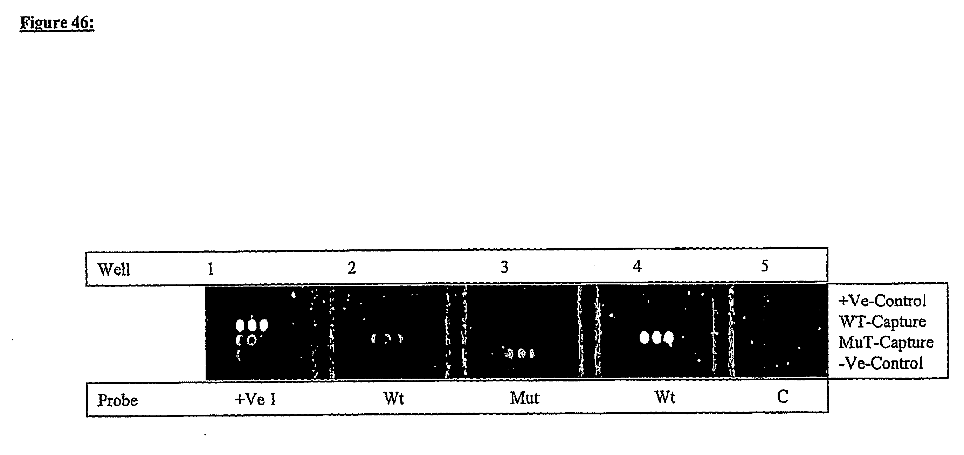

Figure **46****.** shows glass substrate with methylenediphenyl diisocyanate linker. **(10n):** Substrate **10n** is arrayed with three columns and four rows in each of the five wells. Each of the three spots in any row has the same capture arrayed on the slide. The type of capture by row is positive control, wild type capture, mutant capture, and negative control capture DNA sequences from top to bottom. Well 1 shows positive nanoparticle control probes, with no DNA target, binding to positive control captures only. Wells 2 and 4 show PCR wild type target with wild type nanoparticle probes binding to the wild type captures only. Well 3 shows mutant nanoparticle probes with mutant DNA target binding to the mutant captures only. The control, well 5 contained wild type and mutant nanoparticle probes with no DNA targets and demonstrates no non-specific binding of the probes.

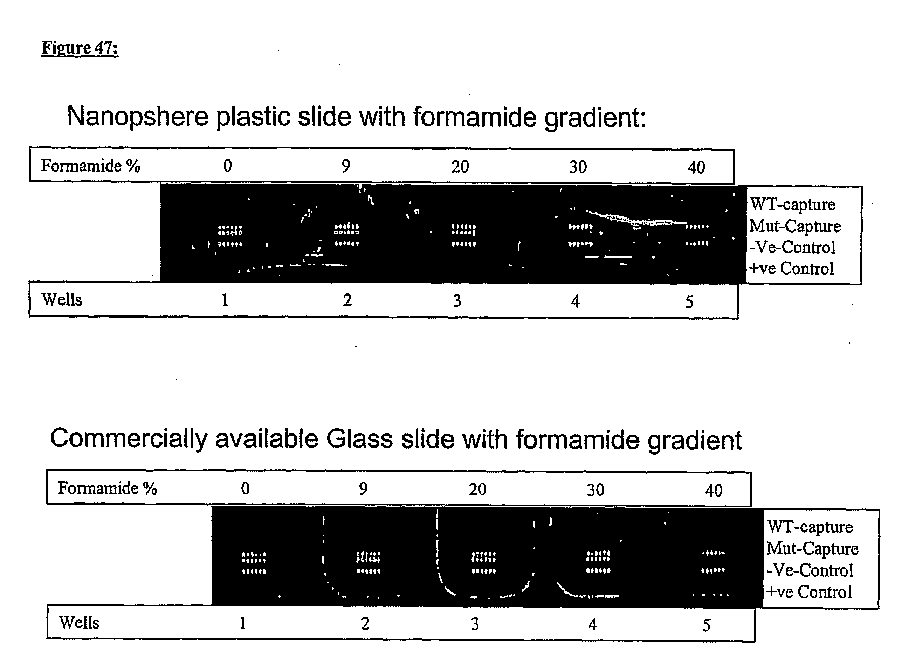

Figure **47****.** shows two separate slides for a comparison of a Nanosphere plastic substrate **(10h)** with a commercially available glass substrate. The top slide (plastic) shows the wild type and mutant differentiation using formamide gradient in different wells on a HMDI surface. A similar experiment was conducted on the commercially available glass slide to compare with Nanosphere modified slide. The modified plastic slide demonstrated higher intensity and discrimination at 30% formamide concentration between wild type and mutant capture sequences and synthetic DNA targets. This is compared to the commercial slide at 40% formamide to obtain discrimination between wild type and mutant synthetic DNA targets. Assay conditions: To each well 100 µL of aliquot was prepared using hybridization buffer, wild type, mutant and control gold nanoparticle probes, and increasing concentrations of formamide. Assay was developed on modified slide following general assay conditions and imaged on Verigene® instrument.

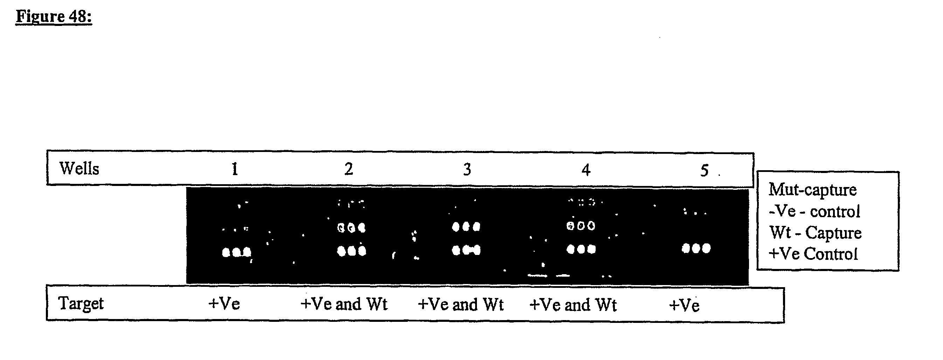

Figure **48****.** shows the plastic substrate with a 1,6 hexamethylene diisocyanate linker on the surface **(10K).** Substrate **10K** is arrayed with three columns and four rows in each of the five wells. Each of the three spots in any row has the same capture arrayed on the slide. The type of capture by row is mutant type capture, negative control, wild type capture, and positive control capture DNA sequences from top to bottom. Wells 1 and 5 show positive nanoparticle control probes, with no DNA target, binding to positive control captures only. Wells 2, 3 and 4 show genomic wild type target with wild type and positive control nanoparticle probes binding to the wild type and positive control captures only.

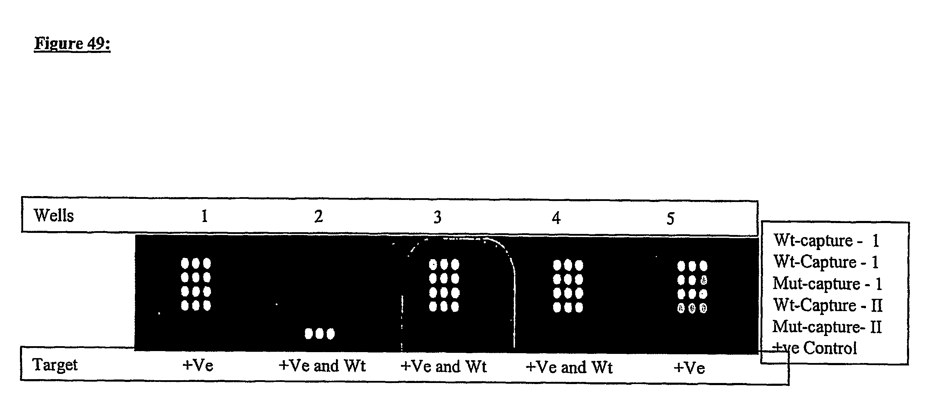

Figure **49****.** shows the plastic substrate with 4,4'-dicyclohexylmethane diisocyanate linker on the surface **(10e).** Substrate **10e** is arrayed with three columns and four rows in each of the five wells. Each of the three spots in any row has the same capture arrayed on the slide. The type of capture by row is wild type -1, wild type -1, mutant -1, wild type - II, mutant -II and positive control captures. All the targets were hybridized to respective captures at room ambient temperature folowing regular assay protocol to check the capture presence on the modified slide. Wells-1,3,4,5 are showing all the genomic captures and well 2 is showing positive control capture.

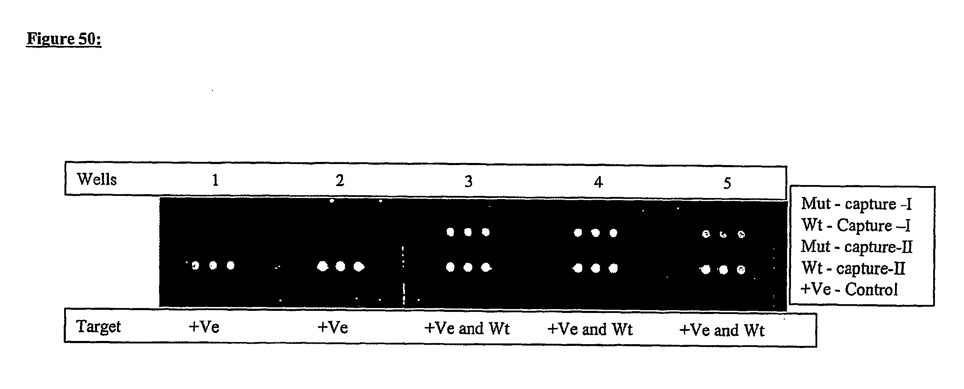

Figure **50****.** shows the plastic substrate with 4,4'-dicyclohexylmethane diisocyanate linker on the surface **(10e).** Substrate **10e** is arrayed with three columns and four rows in each of the five wells. Each of the three spots in any row has the same capture arrayed on the slide. The type of capture by row is mutant - I, wild type I, mutant - II, wild type - II, and positive control capture DNA sequences from top to bottom. Wells 1 and 2 were used for positive controls and 3, 4 and 5 were used for checking to differentiate genomic wild type target from mutant. In wells 3, 4 and 5 both positive control probe and gene specific probe were used to see the signal alignment.

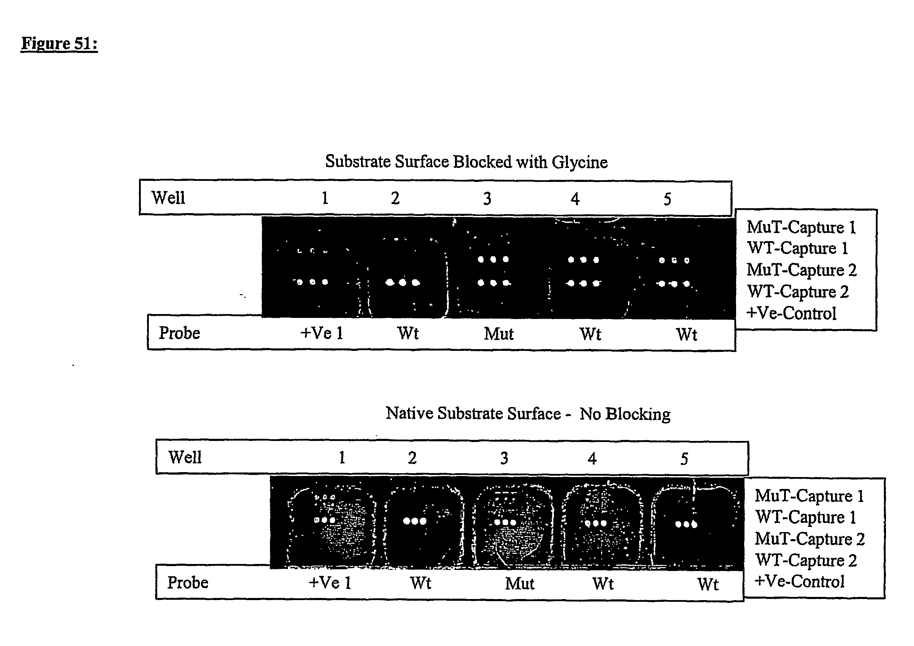

Figure **51****.** shows the plastic substrate with 4,4'-dicyclohexylmethane diisocyanate linker on the surface **(10e).** The sample on the top was blocked with glycine after being arrayed with DNA captures, while the substrate on the bottom was not modified after arraying. Both samples tested with genomic DNA mutant and wild type. Each was arrayed and assayed and developed the same. The substrate (bottom) that was not block visually shows higher background and is approximately 2.5 to 3 times higher in signal response. Substrate **10e** is arrayed with three columns and four rows in each of the five wells. Each of the three spots in any row has the same capture arrayed on the slide. The type of capture by row is wild type -1, wild type -1, mutant -1, wild type - II, mutant -II and positive control captures.

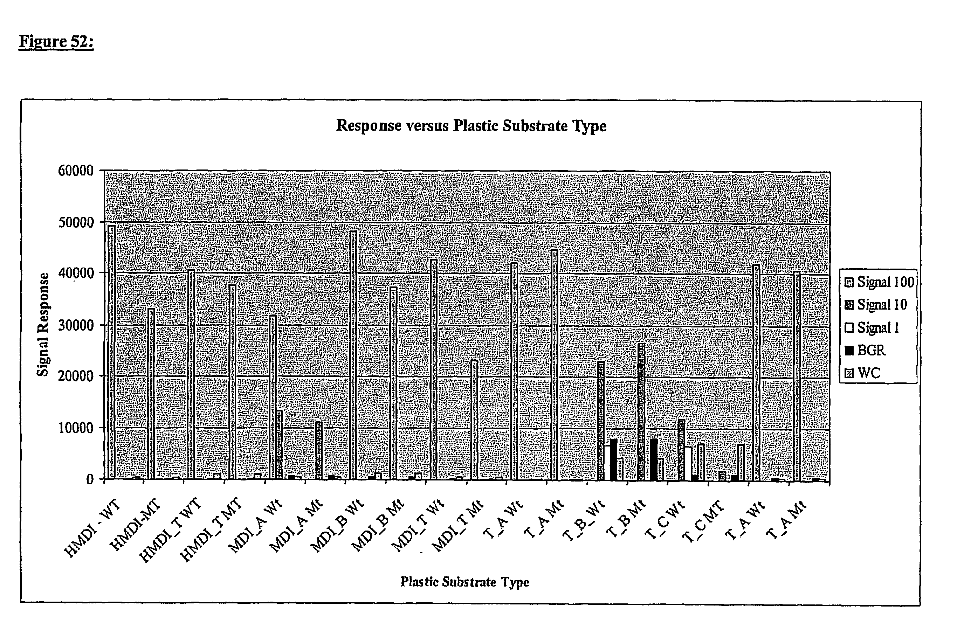

Figure **52****.** shows several different surfaces prepared on plastic. Surfaces contain 3,3'-diaminobenzidene (DAB) linker with, 1,6-hexamethylene diisocyanate (HMDI), 4,4'-methylenediphenyl diisocyanate (MDI), or triethyl citrate (T) linker on the surface. Substrates were arrayed with wild type (WT), mutant (MT), and positive control (PC) captures. Background (BGR) wells were developed without probes or DNA, and water control (WC), wells were exposed to water. Each slide was developed using wild type or mutant DNA targets at 1.9 nM (Signal 100), 190 pM (Signal 10) and 19 pM (Signal 1) and the appropriate nanoparticle probes. After silver enhancement all slides were scanned on the Verigene® detector system. The signal response was plotted and compared across different modified substrates.

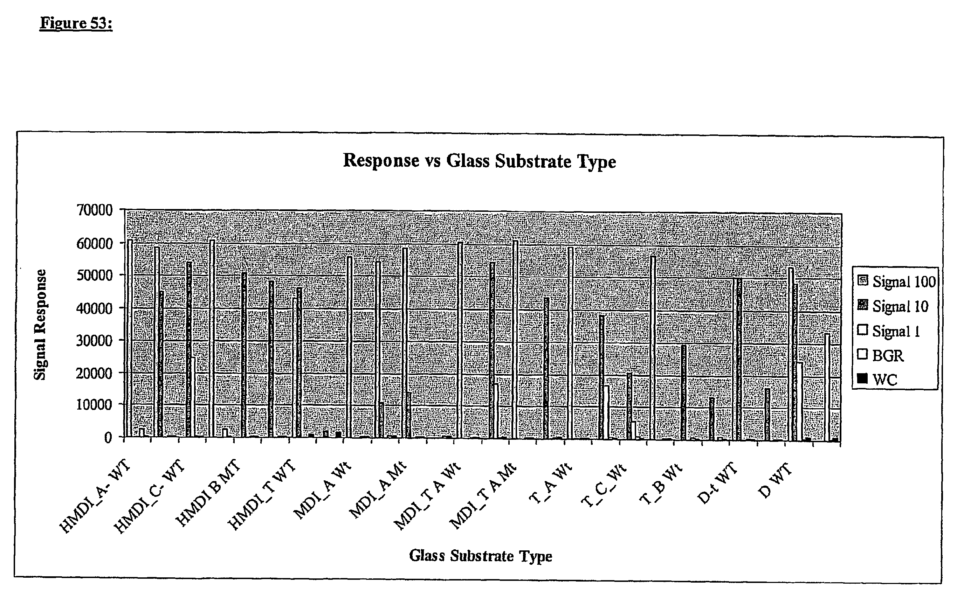

Figure **53****.** shows several different surfaces prepared on glass. Surfaces labeled "HMDI" are from the **10m** preparation, "MDI" from the **10n** preparation, "T" from the **10o** preparation, "D-t" from the **10p** preparation and "D" from the **10a** preparation. Iterations are from different samples. Susbstrates were arrayed with wild type (WT), mutant (MT), and positive control (PC) captures. Background (BGR) wells were developed without nanoparticle probes or target DNA. Water control (WC) wells were exposed to water. Each slide was developed using wild type or mutant DNA targets at 1.9 nM (Signal 100), 190 pM (Signal 10) and 19 pM (Signal 1) and the appropriate nanoparticle probes. After silver enhancement all slides were scanned on the Verigene® detector system. The signal response was plotted and compared across different modified

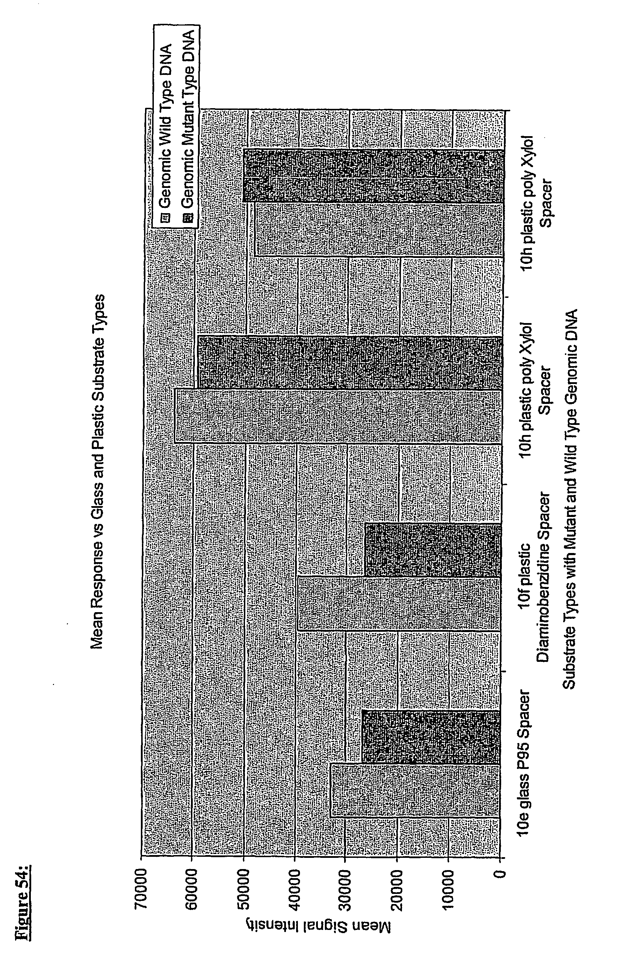

Figure **54****.** shows the mean signal intensities when comparing glass and plastic substrates and also comparing different spacer groups on plastic. Slides were developed with positive, negative, mutant, and wild type DNA captures and developed utilizing nanoparticle probes as previously described herein. PoXyl was completed induplicate.

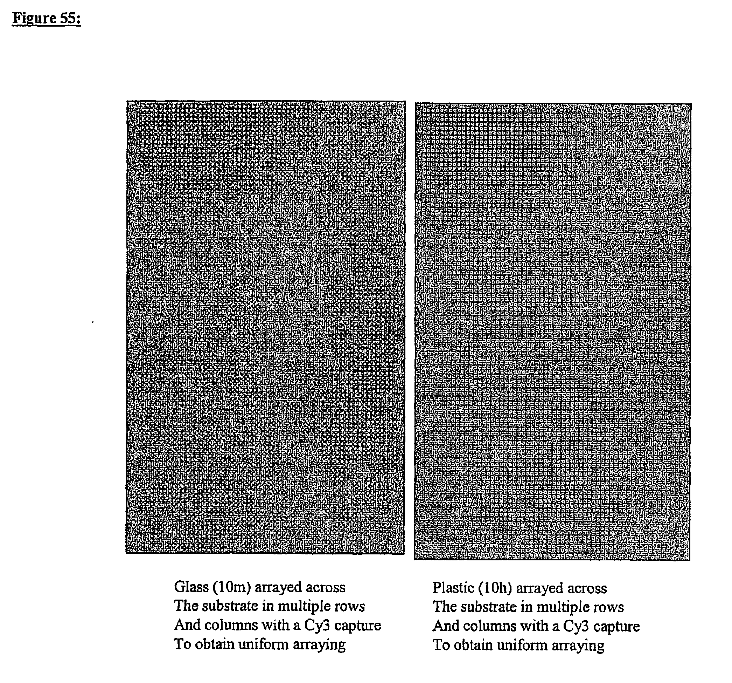

Figure **55****.** shows that glass **(10h)** and plastic **(10m)** substrates were arrayed uniformly with Cy3 capture probes.

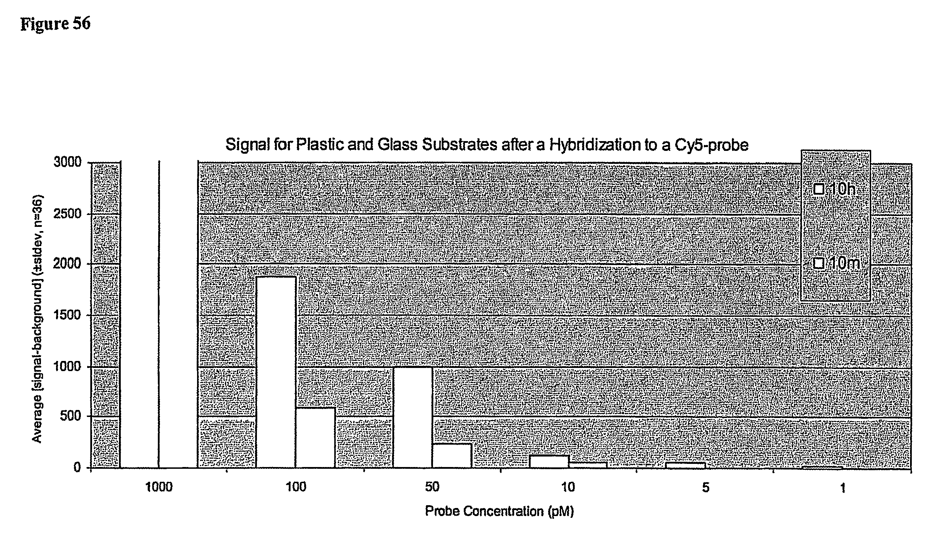

Figure **56****.** shows a one step hybridization of a Cy5 probe to glass **(10m)** and plastic **(10h)** substrates with increasing probe concentrations. As the probe concentration increases so does the fluorescence response from the detector.

### DESCRIPTION OF THE INVENTION

As defined herein, the term "molecule" refers to any desired substance, such as a desired specific binding member, that may be immobilized onto the surface of the substrate. The "specific binding member," as defined herein, means either member of a cognate binding pair. A "cognate binding pair, " as defined herein, is any ligand-receptor combination that will specifically bind to one another, generally through non-covalent interactions such as ionic attractions, hydrogen bonding, Vanderwaals forces, hydrophobic interactions and the like. Exemplary cognate pairs and interactions are well known in the art and include, by way of example and not limitation: immunological interactions between an antibody or Fab fragment and its antigen, hapten or epitope; biochemical interactions between a protein (e.g. hormone or enzyme) and its receptor (for example, avidin or streptavidin and biotin), or between a carbohydrate and a lectin; chemical interactions, such as between a metal and a chelating agent; and nucleic acid base pairing between complementary nucleic acid strands; a peptide nucleic acid analog which forms a cognate binding pair with nucleic acids or other PNAs. Thus, a molecule may be a specific binding member selected from the group consisting of antigen and antibody-specific binding pairs, biotin and avidin binding pairs, carbohydrate and lectin bind pairs, complementary nucleotide sequences, complementary peptide sequences, effector and receptor molecules, enzyme cofactor and enzymes, and enzyme inhibitors and enzymes. Other specific binding members include, without limitation, DNA, RNA, polypeptide, antibody, antigen, carbohydrate, protein, peptide, amino acid, carbohydrate, hormone, steroid, vitamin, drug, virus, polysaccharides, lipids, lipopolysaccharides, glycoproteins, lipoproteins, nucleoproteins, oligonucleotides, antibodies, immunoglobulins, albumin, hemoglobin, coagulation factors, peptide and protein hormones, non-peptide hormones, interleukins, interferons, cytokines, peptides comprising a tumor-specific epitope, cells, cell-surface molecules, microorganisms, fragments, portions, components or products of microorganisms, small organic molecules, nucleic acids and oligonucleotides, metabolites of or antibodies to any of the above substances. Nucleic acids and oligonucleotides comprise genes, viral RNA and DNA, bacterial DNA, fungal DNA, mammalian DNA, cDNA, mRNA, RNA and DNA fragments, oligonucleotides, synthetic oligonucleotides, modified oligonucleotides, single-stranded and double-stranded nucleic acids, natural and synthetic nucleic acids, and aptamers. Preparation of antibody and oligonucleotide specific binding members is well known in the art. Molecules may be immobilized onto substrates and serve as capture probes for target analytes. Molecules may also include a detection label such as a fluorophore or nanoparticle. The molecules (M) have at least one or more nucleophilic groups, e.g., amino, carboxylate, or hydroxyl, that are capable of linking or reacting with the silylating agents to form a reactive silylated molecule which is useful for modifying the surfaces of substrates. These nucleophilic groups are also capable of reacting with reactive moieties such as isocyanate groups and crosslinker molecules on the surfaces of substrates. These nucleophilic groups are either already on the molecules or are introduced by known chemical procedures.

The terms "label" or "detection label" refers to a detectable marker that may be detected by photonic, electronic, opto-electronic, magnetic, gravity, acoustic, enzymatic, or other physical or chemical means.

The phrase "spacer molecule" refers can be any substance having a molecular structure that provides a plurality of functional groups. The spacer may molecule include at least one first functional group that can react with free isocyanate groups previously attached to the surface; at least one second functional group for attachment to a cross-linker molecule which can then be subsequently attached to another spacer molecule or to a capture probe; and at least one optional third functional groups for providing a negative charge. Both the first and second functional groups are any suitable nucleophilic group that can react with a reactive functional group such as isocyanate. Examples of nucleophilic groups include -OH, -SH, -NH-, and -NH₂. The optional third function group includes a carboxylate group. Preferably, the first and second functional groups are free amino groups. Spacer molecules may relate to substances such polymers, carbohydrates, antibiotics having a plurality of one or more types of nucleophic groups, e.g., amino, carboxylate, or hydroxyl groups that are capable of linking or reacting with reactive moieties such as isocyanate groups on the surface of substrates or with linker molecules.

Spacer molecules may include many different types of polymers; preferably those incorporating multiple functional groups. Representative examples of these types of polymers include, without limitation, poly (dimmer acid-co-alkylpolyamine)-95, poly(dimmer acid-co-alkylpolyamine)-140, poly(allylamine), and poly(m-xylendiamine-epichlorohydrin diamine terminated, and PAMAM dendrimer generation 0. Types of polymers also include, without limitation, carbohydrates and polysaccharides. A representative example includes neomycin. Other spacer molecules include low molecular weight compounds that provide the designated functionality; preferred examples include 3,3'-diaminobenzidene, and tris(2-aminoethylamine). The term "capping reagent" refers to a substance that deactivates reactive moieties that may be present on regions of a substrate surface after printing or attachment of capture probes onto the substrate surface. Just prior to capture probe attachment, a surface having free amino groups may be treated with a bifunctional crosslinker molecule of which one functional group reacts with the free amino groups. Another functional group of the crosslinker molecule is available to bind directly or indirectly to the capture probe. The presence of any remaining reactive functional groups after capture probe attachment is generally undesirable as these moieties may react and bind target molecules or detection labels and produce substantial background noise. Thus, any remaining unreacted reactive moieties are generally deactivated prior to use of the substrate for target detection. Examples of capping reagents include amino acid, protein, carbohydrate, carboxylate, thiol, alcohol, and amine. A representative, but non-limiting, example includes glycine

The term "crosslinker molecules," "linker molecules," or "linker compound" refers to a molecule that serves as a bridge or link between different substances or a substance and a surface of a substrate. In one embodiment, the linker molecule is capable of forming at least two covalent bonds such as a bond between a spacer molecule and a free hydroxyl, amino, or carboxylate group on a substrate. Non-limiting examples of linker molecules include ethylene glycolbis (succinimidylsuccinate), disuccinimidyl suberate, 1,6-diisocyanatohexane, methylene bis-(4-cyclohexylisocyanate, glutaric dialdehyde, methylene-p-phenyl diisocyanate, and triethyl citrate.

As defined herein, the term "substrate" refers any solid support suitable for immobilizing oligonucleotides and other molecules are known in the art. These include nylon, nitrocelluose, activated agarose, diazotized cellulose, latex particles, plastic, polystyrene, glass and polymer coated surfaces. These solid supports are used in many formats such as membranes, microtiter plates, beads, probes, dipsticks, optical fibers, etc. Of particular interest as background to the present invention is the use of glass and nylon surfaces in the preparation of DNA microarrays which have been described in recent years (Ramsay, Nat. Biotechnol., 16: 40-4 (1998)). The journal Nature Genetics has published a special supplement describing the utility and limitations of microarrays (Nat.Genet., 21(1): 1-60 (1999). Also of interest are optical substrates such as the ones described in U.S. Patent No. 6,807,352. Typically the use of any solid support requires the presence of a nucleophilic group to react with the silylated molecules of the invention that contain a "reactive group" capable of reacting with the nucleophilic group. Suitable nucleophilic groups or moieties include hydroxyl, sulfhydryl, and amino groups or any moiety that is capable of coupling with the silyated molecules of the invention. Chemical procedures to introduce the nucleophilic or the reactive groups onto solid support are known in the art, they include procedures to activate nylon (U.S. Pat. No. 5,514,785), glass (Rodgers et al., Anal. Biochem., 23-30 (1999)), agarose (Highsmith et al., J., Biotechniques 12: 418-23 (1992) and polystyrene (Gosh et al., Nuc. Acid Res., 15: 5353-5372 (1987)). The preferred substrate is glass.

The substrates may have surfaces that are porous or non-porous. As defined herein, the term "porous" means surface means that the surface permits diffusion to occur. The term "non-porous" surface means that the surface does not permit diffusion to occur.

The term "analyte," or "target analyte", as used herein, is the substance to be quantitated or detected in the test sample using substrates or devices prepared by the method of the present invention. The analyte can be any substance for which there exists a naturally occurring specific binding member (e.g., an antibody, polypeptide, DNA, RNA, cell, virus, etc.) or for which a specific binding member can be prepared, and the analyte can bind to one or more specific binding members in an assay.

In practice, the molecule is contacted with the agent in solution. Generally, the molecule is dissolved in a solution and agent is added drop-wise to the molecule solution. Suitable, but non-limiting, examples of solvents used in preparing the solution include DMF, DMSO, ethanol and solvent mixtures such as DMSO/ethanol. The preferred solvent is ethanol. Water is preferably excluded from the reaction solvent because water may interfere with the efficient modification of the molecule. However, if water is necessary to increase solubility of the molecule in the solution, the amount of water generally ranges from about 0.1 % to about 1 %, usually no greater than 1 %.

The amount of molecule to agent generally ranges from about 1 to about 1.5 typically from about 1 to about 1.1, preferably from about 1 to about 1 molar equivalents. The reaction may be performed in any suitable temperature. Generally, the temperature ranges between about 0 °C and about 40 °C, preferably from about 20 °C to about 25 °C. The reaction is stirred for a period of time until sufficient amount of molecule and agent reacts to form a reactive intermediate. The reactive intermediate has a structure defined by formula iii.

Thereafter, the reaction solution containing the reactive intermediate is then concentrated and dissolved in desired solvent to provide a spotting solution which is then applied to the surface of a substrate. The reactive intermediate is applied as a spotting solution. Any suitable solvent may be used to prepare the spotting solution. Suitable, but non-limiting, examples of solvents used in preparing the spotting solution include DMF, DMSO, and ethanol as well as any suitable solvent mixtures such as DMF/pyridine. Any suitable concentration of the spotting solution may be prepared, generally the concentration of the spotting solution is about 1 mM. Any suitable spotting technique may be used to produce spots. Representative techniques include, without limitation, manual spotting, ink-jet technology such as the ones described in U.S. Patent nos. 5,233,369 and 5,486,855;, array pins or capillary tubes such as the ones described in U.S. patent nos. 5,567,294 and 5,527, 673; microspotting robots (e.g., available from Cartesian); chipmaker micro-spotting device (e.g., as available from TeleChem Interational). Suitable spotting equipment and protocols are commercially available such as the ArrayIt® chipmaker 3 spotting device. The spotting technique can be used to produce single spots or a plurality of spots in any suitable discrete pattern or array.

The agent may be triethoxysilylisocyanate. The molecule may be a nucleic acid.

A method is provided for immobilizing a molecule onto a substrate surface, said method comprising the steps of contacting Si(NCY)₄ with an agent so as to form a first reactive intermediate, said agent having a formula ii:

(R₁)(R₂)(R₃)Si-X-Z ii

wherein R₁, R₂ and R₃ independently represents C₁-C₆ alkoxy, C₁-C₆ alkyl, phenyl, or aryl substituted with one or more groups selected from the group consisting of C₁-C₆ alkyl and C₁-C₆ alkoxy; X represents linear or branched C₁-C₂₀ alkyl or aryl substituted with one or more groups selected from the group consisting of C₁-C₆ alkyl and C₁-C₆ alkoxy, optionally substituted with one or more heteroatoms comprising oxygen, nitrogen, or sulfur; wherein Y represents oxygen or sulfur; and Z represents a hydroxy or amino group, with the proviso that at least one of R₁, R₂ or R₃ represents C₁-C₆ alkoxy; contacting the first reactive intermediate with a molecule so as to form a second reactive intermediate; and contacting the second reactive intermediate with said surface so as to immobilized the molecule onto said surface.

The method may provide for a modification of substrate surfaces with branched molecules so as to increase molecule loading on the substrate surface. These branched molecules behave like dendrimers to enhance sensitivity in assay performance. In practice, either Si(NCO)₄ or Si(NCS)₄ are reacted with a compound of formula ii to form a first reactive intermediate having the formula iv:

(R₁)(R₂)(R₃)Si-X-Z-CYNH-Si (NCY)₃ iv

wherein R₁, R₂ and R₃ independently represents C₁-C₆ alkoxy, C₁-C₆ alkyl, phenyl, or aryl substituted with one or more groups selected from the group consisting of C₁-C₆ alkyl and C₁-C₆ alkoxy; X represents linear or branched C₁-C₂₀ alkyl or aryl substituted with one or more groups selected from the group consisting of C₁-C₆ alkyl and C₁-C₆ alkoxy, optionally substituted with one or more heteroatoms comprising oxygen, nitrogen, or sulfur; Y represents oxygen or sulfur; and Z represents oxygen or NH, with the proviso that at least one of R₁, R₂, or R₃ represents C₁-C₆ alkoxy.

Generally, Si(NCO)₄ or Si(NCS)₄ is dissolved in a suitable dry solvent as described above. In practice, ethanol is the preferred solvent. The resulting ethanol solution is contained in a reaction flask and a solution of formula ii compound is added to the reaction flask. The formula ii solution may include any of the dried solvents described above. In practice, ethanol is the preferred solvent. The reaction temperature generally ranges from about 0 °C to about 40 °C, preferably about 22°C. The reaction mixture is allowed to stir from about 1min to about 60min, usually about 5min to about 10min, until it reaches completion. The molar amount of Si(NCO)₄ or Si(NCS)₄ to formula ii compound generally ranges from about 3:1 to 1:1, preferably about 1:1.

Thereafter, the molecule is contacted with the first reactive intermediate to form a second reactive intermediate having the formula v:

(R₁)(R₂)(R₃)Si-X-Z-CYNH-Si (NHCYL-M)₃ v

wherein R₁, R₂ and R₃ independently represents C₁-C₆ alkoxy, C₁-C₆ alkyl, phenyl, or aryl substituted with one or more groups selected from the group consisting of C₁-C₆ alkyl and C₁-C₆ alkoxy; X represents linear or branched C₁-C₂₀ alkyl or aryl substituted with one or more groups selected from the group consisting of C₁-C₆ alkyl and C₁-C₆ alkoxy, optionally substituted with one or more heteroatoms comprising oxygen, nitrogen, or sulfur; L represents a linking group; Y represents oxygen or sulfur; and Z represents oxygen or NH; and M represents a molecule, with the proviso that at least one of R₁, R₂, or R₃ represent C₁-C₆ alkoxy. The linking group L may be a nucleophile that is naturally present or chemically added to the molecule such as an amino, sulfhydryl group, hydroxy group, carboxylate group, or any suitable moiety. L may represent -NH, -S-, - O-, or -OOC-.

The molecule is contacted with the first reactive intermediate in solution. Generally, the molecule is dissolved in a solvent and added dropwise to the reaction flask containing the first reactive intermediate. The molecule is generally mixed in any suitable solvent as described above. The molar amount of molecule to first reactive intermediate generally ranges from about 1 to about 10 typically from about 1 to about 3, preferably from about 1 to about 4. The reaction may be performed in any suitable temperature. Generally, the temperature ranges between about 0 °C and about 40 °C, preferably from about 20 °C to about 25 °C. The reaction is stirred for a period of time until sufficient amount of molecule and first reactive intermediate reacts to form a second reactive intermediate. Generally, an excess amount of molecule is used to react with the first reactive intermediate. In practice, typically at least 3 equivalents of molecule to 1 equivalent of first reactive intermediate is used.

Thereafter, the second reactive intermediate is then applied to the surface of a substrate using techniques described above.

In another aspect, if the ratio of Si(NCO)₄ or Si(NCS)₄ to formula ii compound is about 1:2 equiv./equiv., a first reactive intermediate is formed having the formula vi:

((R₁)(R₂)(R₃)Si-X-Z-CYNH)₂-Si (NCY)₂ vi

wherein R₁, R₂ and R₃ independently represents C₁-C₆ alkoxy, C₁-C₆ alkyl, phenyl, or aryl substituted with one or more groups selected from the group consisting of C₁-C₆ alkyl and C₁-C₆ alkoxy; X represents linear or branched C₁-C₂₀ alkyl or aryl substituted with one or more groups selected from the group consisting of C₁-C₆ alkyl and C₁-C₆ alkoxy, optionally substituted with one or more heteroatoms comprising oxygen, nitrogen, or sulfur; Y represents oxygen or sulfur; and Z represents oxygen or NH, with the proviso that at least one of R₁, R₂, or R₃ represents C₁-C₆ alkoxy. Preferably, R₁, R₂ and R₃ represent methoxy, X represents phenyl, Y represents oxygen, and Z represents NH.

Thereafter, the molecule is contacted with the first reactive intermediate of formula vi as described above to produce a second reactive intermediate having the formula vii:

((R₁)(R₂)(R₃)Si-X-Z-CYNH)₂Si(NHCYL-M)₂ vii

wherein R₁, R₂ and R₃ independently represents C₁-C₆ alkoxy, C₁-C₆ alkyl, phenyl, or aryl substituted with one or more groups selected from the group consisting of C₁-C₆ alkyl and C₁-C₆ alkoxy; L represents a linking group; X represents linear or branched C₁-C₂₀ alkyl or aryl substituted with one or more groups selected from the group consisting of C₁-C₆ alkyl and C₁-C₆ alkoxy, optionally substituted with one or more heteroatoms comprising oxygen, nitrogen, or sulfur; Y represents oxygen or sulfur; and Z represents oxygen or NH; and M represents a molecule, with the proviso that at least one of R₁, R₂, or R₃ represent C₁-C₆ alkoxy. The linking group L may be a nucleophile that is naturally present or chemically added to the molecule such as an amino, sulfhydryl group, hydroxy group, carboxylate group, or any suitable moiety. L may represent -NH, -S-, -O-, or -OOC-. Generally, an excess amount of molecule is used to react with the first reactive intermediate. In practice, typically at least 3 equivalents of molecule to 1 equivalent of first reactive intermediate is used.

Thereafter, the second reactive intermediate is then applied to the surface of a substrate using the techniques described above.

A compound is provided having the formula iii:

(R₁)(R₂)(R₃)Si-X-NHCYL-M iii

wherein R₁, R₂ and R₃ independently represents C₁-C₆ alkoxy, C₁-C₆ alkyl, phenyl, or aryl substituted with one or more groups selected from the group consisting of C₁-C₆ alkyl and C₁-C₆ alkoxy; L represents a linking group; X represents linear or branched C₁-C₂₀ alkyl or aryl substituted with one or more groups selected from the group consisting of C₁-C₆ alkyl and C₁-C₆ alkoxy, optionally substituted with one or more heteroatoms comprising oxygen, nitrogen, or sulfur; Y represents oxygen or sulfur; and M represents a molecule, with the proviso that at least one of R₁, R₂, or R₃ represent C₁-C₆ alkoxy. The linking group L may be a nucleophile that is naturally present or chemically added to the molecule such as an amino, sulfhydryl group, hydroxy group, carboxylate group, or any suitable moiety. L may represent -NH, -S-, -O-, or -OOC-. In the preferred embodiment, R₁, R₂, and R₃ represent alkoxy, L represents -NH-, X represents propyl, and Y represents O. The compound is useful for modifying substrate surfaces with a desired molecule. A

A compound is provided having a formula iv:

(R₁)(R₂)(R₃)Si-X-Z-CYNH-Si (NCY)₃ iv

wherein R₁, R₂ and R₃ independently represents C₁-C₆ alkoxy, C₁-C₆ alkyl, phenyl, or aryl substituted with one or more groups selected from the group consisting of C₁-C₆ alkyl and C₁-C₆ alkoxy; X represents linear or branched C₁-C₂₀ alkyl or aryl substituted with one or more groups selected from the group consisting of C₁-C₆ alkyl and C₁-C₆ alkoxy, optionally substituted with one or more heteroatoms comprising oxygen, nitrogen, or sulfur; Y represents oxygen or sulfur; and Z represents oxygen or NH, with the proviso that at least one of R₁, R₂, or R₃ represents C₁-C₆ alkoxy. In the preferred embodiment, R₁, R₂, and R₃ represent ethoxy or methoxy, X represents benzyl, Y represents oxygen, and Z represents NH. The compound is useful for modifying molecules so that they can be attached to substrate surfaces.

A compound is provided having a formula v:

(R₁)(R₂)(R₃)Si-X-Z-CYNH-Si (NHCYL-M)₃ v

wherein R₁, R₂ and R₃ independently represents C₁-C₆ alkoxy, C₁-C₆ alkyl, phenyl, or aryl substituted with one or more groups selected from the group consisting of C₁-C₆ alkyl and C₁-C₆ alkoxy; L represents a linking group; X represents linear or branched C₁-C₂₀ alkyl or aryl substituted with one or more groups selected from the group consisting of C₁-C₆ alkyl and C₁-C₆ alkoxy, optionally substituted with one or more heteroatoms comprising oxygen, nitrogen, or sulfur; Y represents oxygen or sulfur; and Z represents oxygen or NH; and M represents a molecule, with the proviso that at least one of R₁, R₂, or R₃ represent C₁-C₆ alkoxy. The linking group L may be a nucleophile that is naturally present or chemically added to the molecule such as an amino, sulfhydryl group, hydroxy group, carboxylate group, or any suitable moiety. L may represent -NH, -S-, -O-, or - OOC-. In the preferred embodiment, R₁, R₂, and R₃ represent methoxy or ethoxy, X represents 3- or 4- phenyl, Y represents oxygen, and Z represents NH. The compound is useful for modifying molecules so that they can be attached to substrate surfaces.

A device is provided for the detection of target analytes in a sample. The device comprises a surface having an immobilized molecule as a specific binding member to the target analyte, wherein said surface is prepared by any of the above methods. The surface may be a glass surface. The surface may have one or more different specific binding members attached thereto in an array to allow for the detection of different portions of a target analyte or multiple different types of target analytes.

A kit is provided. The kit may comprise one or more containers containing any of the silylating agents mentioned above with an optional substrate, and a set of instructions.

The present description also discloses derivatizing substrate surfaces (e.g, glass, plastic, metal) have a variety of surface properties (i.e. porous and nonporous, impermeable and nonimpermeable) for use in nanoparticle-based detection of a target analyte. In particular, the substrate will typically have a surface comprising bound free hydroxyl, amino, or carboxylate groups, or a combination thereof. The substrate surface is then contacted with a disilyl compound having both silyl alkoxy and silyl isocyanate groups. Contacting the disilyl compound is such that the silyl alkoxy group preferentially contacts to the surface of the substrate so as to provide an anchor molecule having free isocyanate groups. The subsequent attachment of spacer molecules is made possible by reacting to the free isocyanate groups.

The substrate surface anchor group providing the free isocyanate groups can then be reacted with a spacer molecule; addition of a suitable spacer molecule provides free amino groups for attachment of an additional spacer molecule while also providing the desired surface characteristics. Suitable spacer molecules can be comprised of multiple free amino groups; some free amino groups react with the free isocyanate group provided by the anchor molecule whereas the other free amino groups provide a surface for attachment of an additional spacer molecule. A preferred spacer of this type is 3,3'-diaminobenzidene. Other suitable spacer molecules can generally be comprised of multifunctional groups, in particular, amino groups as well as functional groups providing a negatively charged moiety. Examples of suitable spacer molecules are polymers; examples of polymers include, but are not limited to, poly (dimmer acid-co-alkylpolyamine)-95, poly(dimmer acid-co-alkylpolyamine)-140, poly(allylamine), poly(m-xylendiamine-epichlorohydrin diamine terminated, tris(2-aminoethylamine), and PAMAM dendrimer generation 0. A preferred polymer is one which is also comprised of negatively charged functional groups, an example of which is a carboxylic acid. An example of a preferred polymer includes poly (dimmer acid-co-alkylpolyamine)-95 and poly(dimmer acid-co-alkylpolyamine)-140. Other suitable spacers include carbohydrate polymers, and antibiotics such as neomycin.

Alternately, the anchor group providing free isocyanate groups can optionally be reacted with water without attaching a spacer molecule; the reaction with water is such that the free isocyanate groups undergo hydrolysis to produce free amino groups. Attachment of spacer molecules can then be carried out as described.

Linker molecules comprise functional groups capable of reacting with the free amino groups provided on the surface, and can be attached in any step where free amino groups are provided. According to the present invention, the linker molecules provide suitable functional groups (e.g. carboxylic acids, ester groups, and isocyanates) for attaching capture probes (i.e during arraying) that are specific for a target analyte. Preferred linker molecules include ethylene glycolbis (succinimidylsuccinate), disuccinimidyl suberate, 1,6-diisocyanatohexane, methylene bis-(4-cyclohexylisocyanate), glutaric dialdehyde, methylene-p-phenyl diisocyanate, and triethyl citrate.

A linker group can be attached directly to the hydrolyzed anchor group. Preferably, additional spacer molecules are attached to provide a substrate surface having the desired surface characteristics. Additional spacer molecules can be attached to any surface comprised of free amino groups by first reacting a diisocyanate compound to the free amino group surface to provide free isocyanate groups. Preferred diisocyanate compounds include phenylene 1,4-diisocyanate, tolylene-2,6-diisocyanate, tolylene-α,4-diisocyanate, and isophorone diisocyanate. The additional spacer molecule can then be attached via the reaction between the free isocyanate group on the surface and free amino group of the spacer molecule; attachment of any number of spacer molecules that result in an amenable surface according to this invention is contemplated, however, the preferred number of spacer molecules is between 2 and 7.

After the linker molecule is attached to the surface, the substrate is arrayed in discrete predetermined areas on the surface to attach a capture probe. More than one type of capture probes can be contacted with the surface; each type of capture probes is specific for a particular target analyte. A preferred capture probe is a nucleic acid.

A principle advantage of the method is that many types of amine-linked compounds can be coupled in a three-dimensional way to the polymer layer, thus maximizing availability for hybridizing target DNA and RNA biomolecules on the surface for detection purposes. The immobilizing amine molecules do not directly contact the substrate surface but rather contacting the polymer coated on the surface of the substrate. The copolymer layer on the glass surface contains both amine and acid groups with amide bond linkage through out the molecular chain. Without wishing to be bound by any particular theory, it is believed that the copolymer coating of the invention provides good results because the acid groups in the chain contribute to controlling the nonspecific binding of gold nanoparticle probes to the surface which in turn leads to minimization of background noise. Example 1 provides a general protocol for preparing polymer-coated substrates.

Any substrate can be used**.** Suitable substrates include transparent solid surfaces (e.g., glass, quartz, plastics and other polymers), opaque solid surface, and conducting solid surfaces. The substrate can be any shape or thickness, but generally will be flat and thin. Preferred are glass substrates, such as glass slides.

A method is provided for making a substrate for use in target analyte detection. The method comprises: (a) providing a substrate having a surface; (b) contacting said surface with a isocyanate compound so as to provide a surface comprising free isocyanate groups, the isocyanate compound is a member selected from the group consisting of:

Si(NCY)₄;

[(R₁)(R₂)(R₃)Si-X-Z-CYNH]₂-Si (NCY)₂ vi;

and

(R₁)(R₂)(R₃)Si-X-Z-CYNH-Si (NCY)₃ iv;

wherein R₁, R₂ and R₃ independently represents C₁-C₆ alkoxy, C₁-C₆ alkyl, phenyl, or aryl substituted with one or more groups selected from the group consisting of C₁-C₆ alkyl and C₁-C₆ alkoxy; X represents linear or branched C₁-C₂₀ alkyl or aryl substituted with one or more groups selected from the group consisting of C₁-C₆ alkyl and C₁-C₆ alkoxy, optionally substituted with one or more heteroatoms comprising oxygen, nitrogen, or sulfur; Y represents oxygen or sulfur; and Z represents oxygen or NH, with the proviso that at least one of R₁, R₂, or R₃ represents C₁-C₆ alkoxy.In another embodiment of the invention, a method is provided for making a substrate for use in target analyte detection. The method comprises: (a) providing a substrate having a surface; (b) contacting said surface with a isocyanate compound so as to provide a surface comprising free isocyanate groups, the isocyanate compound is a member selected from the group consisting of:

Si(NCY)₄;

[(R₁)(R₂)(R₃)Si-X-Z-GYNH]₂-Si (NCY)₂ vi;

and

(R₁)(R₂)(R₃)Si-X-Z-CYNH-Si (NCY)₃ iv;

wherein R₁, R₂ and R₃ independently represents C₁-C₆ alkoxy, C₁-C₆ alkyl, phenyl, or aryl substituted with one or more groups selected from the group consisting of C₁-C₆ alkyl and C₁-C₆ alkoxy; X represent linear or branched C₁-C₂₀ alkyl or aryl substituted with one or more groups selected from the group consisting of C₁-C₆ alkyl and C₁-C₆ alkoxy, optionally substituted with one or more heteroatoms comprising oxygen, nitrogen, or sulfur; Y represents oxygen or sulfur; and Z represents oxygen or NH, with the proviso that at least one of R₁, R₂, or R₃ represents C₁-C₆ alkoxy; (c) contacting said surface comprising free isocyanate groups with a spacer molecule so as to provide a surface comprising free amino groups; and (d) contacting said surface comprising free amino groups with a linker molecule so as to provide a reactive surface having free reactive groups.

In one aspect, steps (c) and (d) may be repeated one or more times.

In another aspect, the method comprises after step (d): (e) contacting said reactive surface with at least one type of capture probe specific for the target analyte so as to provide a surface comprising immobilized capture probes; and (f) contacting said surface comprising immobilized capture probes with a capping agent so as to block residual unreacted free isocyanate groups on areas of the surface not having immobilized capture probes and produce a substrate having substantially low signal background due to non-specific nanoparticle binding relative to a surface not contacted with a capping agent.

In another aspect, the method further comprises: (i) contacting said reactive surface with at least one type of capture probe specific for the target analyte so as to provide a surface comprising immobilized capture probes; and (ii) contacting said surface comprising immobilized capture probes with a capping agent so as to block residual unreacted free isocyanate groups on areas of the surface not having immobilized capture probes and produce a substrate having substantially low signal background due to non-specific nanoparticle binding relative to a surface not contacted with a capping agent.

A method is provided for making a substrate for use in target analyte detection. The method comprises: (a) providing a substrate having a surface; (b) contacting said surface with a isocyanate compound so as to provide a surface comprising free isocyanates groups, the isocyanate compound is a member selected from the group consisting of:

Si(NCY)₄;

[(R₁)(R₂)(R₃)Si-X-Z-CYNH]₂-Si (NCY)₂ vi;

and

(R₁)(R₂)(R₃)Si-X-Z-CYNH-Si (NCY)₃ iv;

wherein R₁, R₂ and R₃ independently represents C₁-C₆ alkoxy, C₁-C₆ alkyl, phenyl, or aryl substituted with one or more groups selected from the group consisting of C₁-C₆ alkyl and C₁-C₆ alkoxy; X represents linear or branched C₁-C₂₀ alkyl or aryl substituted with one or more groups selected from the group consisting of C₁-C₆ alkyl and C₁-C₆ alkoxy, optionally substituted with one or more heteroatoms comprising oxygen, nitrogen, or sulfur; Y represents oxygen or sulfur; and Z represents oxygen or NH, with the proviso that at least one of R₁, R₂, or R₃ represents C₁-C₆ alkoxy; (c) contacting the surface comprising free isocyanate groups with water so as to provide a surface comprising free amino groups; and (d) contacting said surface comprising free amino groups with a linker molecule so as to provide a reactive surface having free reactive groups.

In one aspect, the method further comprising, after step (d): (e) contacting said surface comprising free isocyanate groups with a spacer molecule so as to provide a surface comprising free amino groups; and (f) contacting said surface comprising free amino groups with a linker molecule so as to provide a reactive surface having free reactive groups.

In one aspect, steps (e) and (f) may be repeated one or more times.

In another aspect, the method further comprises: (i) contacting said reactive surface with at least one type of capture probe specific for the target analyte so as to provide a surface comprising immobilized capture probes; and (ii) contacting said surface comprising immobilized capture probes with a capping agent so as to block residual unreacted free isocyanate groups on areas of the surface not having immobilized capture probes and produce a substrate having substantially low signal background due to non-specific nanoparticle binding relative to a surface not contacted with a capping agent.

A method for making a substrate for use in detection of a target analyte is provided. The method comprises: (a) providing a substrate having a surface; (b) contacting said surface with a isocyanate compound so as to provide a surface comprising free isocyanate groups, the isocyanate compound is a member selected from the group consisting of:

Si(NCY)₄;

[(R₁)(R₂)(R₃)Si-X-Z-CYNH]₂-Si (NCY)₂ vi;

and

(R₁)(R₂)(R₃)Si-X-Z-CYNH-Si (NCY)₃ iv;

wherein R₁, R₂ and R₃ independently represents C₁-C₆ alkoxy, C₁-C₆ alkyl, phenyl, or aryl substituted with one or more groups selected from the group consisting of C₁-C₆ alkyl and C₁-C₆ alkoxy; X represents linear or branched C₁-C₂₀ alkyl or aryl substituted with one or more groups selected from the group consisting of C₁-C₆ alkyl and C₁-C₆ alkoxy, optionally substituted with one or more heteroatoms comprising oxygen, nitrogen, or sulfur; Y represents oxygen or sulfur; and Z represents oxygen or NH, with the proviso that at least one of R₁, R₂, or R₃ represents C₁-C₆ alkoxy; (c) contacting said surface comprising free isocyanate groups with a spacer molecule so as to provide a surface comprising free amino groups; (d) contacting said surface comprising free amino groups with a linker molecule so as to provide a reactive surface having free reactive groups; (e) contacting said reactive surface with at least one type of capture probe specific for the target analyte so as to provide a surface comprising immobilized capture probes; and (f) contacting said surface comprising immobilized capture probes with a capping agent so as to block residual unreactive free isocyanate groups and produce a substrate having substantially low signal background due to non-specific nanoparticle binding relative to a surface not contacted with a capping agent.

In any of the above methods for making a substrate, the isocyanate compound may be is selected from the group consisting of 2-Trimethoxysilane-6-triisocyanatosilanceureabenzene, 3-(triethoxysilyl) propylisocyanate), and tetraisocyanatosilane.

The spacer molecule can be any substance having a molecular structure that provides a plurality of functional groups. In one embodiment, the spacer molecule includes at least one first functional group that can react with free isocyanate groups previously attached to the surface; at least one second functional group for attachment to a cross-linker molecule which can then be subsequently attached to another spacer molecule or to a capture probe; and at least one optional third functional groups for providing a negative charge. Both the first and second functional groups are any suitable nucleophilic group that can react with a reactive functional group such as isocyanate. Examples of nucleophilic groups include -OH, -SH, -NH-, and NH₂. The optional third function group includes a carboxylate group. Preferably, the first and second functional groups are free amino groups.

Spacer molecules may include many different types of polymers; preferably those incorporating multiple functional groups. Representative examples of these types of polymers include, without limitation, poly (dimmer acid-co-allrylpolyamine)-95, poly(dimmer acid-co-alkylpolyamine)-140, poly(allylamine), and poly(m-xylendiamine-epichlorohydrin diamine terminated, and PAMAM dendrimer generation 0. Types of polymers also include, without limitation, carbohydrates and polysaccharides. A representative example includes neomycin. Other spacer molecules include low molecular weight compounds that provide the designated functionality; preferred examples include 3,3'-diaminobenzidene, and tris(2-aminoethylamine).

The capping reagents include amino acid, protein, carbohydrate, carboxylate, thiol, alcohol, and amine. A representative, but non-limiting, example includes glycine.

Representative, but non-limiting examples of isocyanate compound include phenylene 1,4-diisocyanate, tolylene-2,6-diisocyanate, tolylene-α,4-diisocyanate, and isophorone diisocyanate.

Non-limiting examples of linker molecules include ethylene glycolbis (succinimidylsuccinate), disuccinimidyl suberate, 1,6-diisocyanatohexane, methylene bis-(4-cyclohexylisocyanate, glutaric dialdehyde, methylene-p-phenyl diisocyanate, and triethyl citrate.

Any suitable substrate may be used in the above methods. Preferably, the substrate surface (e.g, glass, plastic, metal) includes at least one group that reacts with the disilyl compounds, such as hydroxyl, amino, or carboxylate groups, or any combination thereof.

Substrate materials amenable for nanoparticle-based detection methods encompass a variety of relevant surface properties. Thus, the substrate material has a refractive index in the range of 1.400 to 1.900. In another aspect, the substrate material provides a light transmittance, that is, the amount of light which passes through the substrate without being either absorbed or being reflected by the surface, either by way of light passing through substrate material parallel to the horizontal axis, or light passing through the substrate material perpendicular to the horizontal axis, of greater than 80%.

In yet another aspect, the substrate surface modified produces a background signal upon imaging using visual or fluorescent light having substantially reduced background signal relative to a substrate not having said polymeric layer. The substrate surface prior to attaching capture probes has a preferred water contact angle in the range of 25 - 75 degrees.

The substrate comprises a surface having a polymeric layer comprising negatively charged ionic groups and free isocyanate groups capable of binding said capture probes.

A substrate for use in target analyte detection is provided. The substrate comprises a surface modified by any of the above methods.

A kit is provided for detecting target analytes. The kit comprises any of the above substrates and substrates prepared by the above methods.

A method is provided for detecting one or more target analytes in a sample, the target analyte having at least two binding sites. The method comprises: (a) providing a substrate pprepared by any of the methods of the invention, said substrate having at least one type of capture probes immobilized on a surface of the substrate, each type of capture probes specific for a target analyte; (b) providing at least one type of detection probe comprising a nanoparticle and a detector probe, the detector probe specific for a target analyte; (c) contacting the capture probes, the detection probes and the sample under conditions that are effective for the binding of the capture probes and detector probes to the specific target analyte to form an immobilized complex onto the surface of the substrate; (d) washing the surface of the substrate to remove unbound nanoparticles; and (e) observing for the presence or absence of the complex as an indicator of the presence or absence of the target molecule.

A method for immobilizing nanoparticles on a substrate surface is disclosed. 3' amine linked oligonucleotides may be synthesized. The 5' end of the 3' amine linked oligonucleotides are attached to nanoparticles, for instance via sulfide linkers. Techniques for functionalizing oligonucleotides with sulfide groups and attachment to nanoparticles are described for instance in published U.S. patent application numbers 2003/0143598A1 and 2002/0155442A1. A preferred sulfide linker for linking the oligonucleotide to the nanoparticle is an epiandrosterone linker. The oligonucleotide is additionally modified on the 3' end to form a 3' end modified oligonucleotide, which is then contacted to the nanoparticle surface. The nanoparticles with the modified oligonucleotides attached thereto are then contacted with an aldehyde modified substrate surface, resulting in immobilization of the nanoparticles on the substrate surfaces

The method may comprise synthesizing 3' silyl functionalized oligonucleotides to form a 3' end modified oligonucleotide. The modified oligonucleotides are attached to the surface of the nanoparticles through sulfide linkers on the oligonucleotide. The nanoparticles with the 3' silyl linked oligonucleotides attached thereto are then contacted with an aldehyde modified substrate surface, resulting in immobilization of the nanoparticles on the substrate surface.

The immobilization of nanoparticles on substrate surfaces as described herein is useful in several detection techniques. For example the immobilization method of the invention is useful for analyzing silver amplification reagents on a glass surface. For instance, gold nanoparticles immobilized on glass surfaces by the methods of the invention can be used as a positive control in silver amplification based DNA detection assay techniques. Alternatively, a DNA probe can be directly hybridized to the DNA capture strand which is coupled to the glass surface as a positive control. This hybridization method varies from batch to batch of modified glass slides and signal is dependent on hybridization efficiency.

In addition, using the method of the invention one can avoid the capture-probe hybridization procedure in assays that utilize a capture probe for immobilizing a nanoparticle on the substrate surface. Generally, in such assays, a positive control is provided by hybridizing an oligonucleotide on a nanoparticle to a complementary oligonucleotide on the substrate surface. The invention eliminates this hybridization step, because the nanoparticle is directly immobilized on the substrate surface.

Further, the direct immobilization of nanoparticles is also highly useful in detecting DNA targets using surface plasmon resonance (SPR) angle shift technique with different sizes of DNA modified nanoparticle probes. When DNA target is hybridized to the immobilized nanoparticle linked capture strand and DNA modified nanoparticle detection probe in a sandwich assay format, SPR angle shift can be measured using spectroscopy techniques. This provides a method for detecting target DNA using SPR spectroscopy in the presence of large size DNA linked nanoparticle probes. Even single nuclear polymorphs (SNPs) can be detected using this nanoparticle linked capture -nanoparticle linked probe method.

Any substrate whose surface can be modified to provide a surface comprised of aldehyde groups can be used in the invention. Suitable substrates include transparent solid surfaces (e.g., glass, quartz, plastics and other polymers), opaque solid surface, and conducting solid surfaces. The substrate can be any shape or thickness, but generally will be flat and thin. Preferred are glass substrates, such as glass slides.

A comparative method for immobilizing a nanoparticle onto a surface, is a method comprising the steps of (a) providing a substrate having a surface and a nanoparticle having oligonucleotides bound thereto, at least a portion of the oligonucleotides have a free amine group at an end not bound to the nanoparticle; (b) contacting the nanoparticle with an agent so as to form a reactive intermediate, said agent having a formula i:

(R₁)(R₂)(R₃)Si-X-NCY i

wherein R₁, R₂ and R₃ independently represents C₁-C₆ alkoxy, C₁-C₆ alkyl, phenyl, or aryl substituted with one or more groups selected from the group consisting of C₁-C₆ alkyl and C₁-C₆ alkoxy; X represents linear or branched C₁-C₂₀ alkyl or aryl substituted with one or more groups selected from the group consisting of C₁-C₆ alkyl and C₁-C₆ alkoxy, optionally substituted with one or more heteroatoms comprising oxygen, nitrogen, or sulfur; and Y represents oxygen or sulfur, with the proviso that at least one of R₁, R₂ or R₃ represents C₁-C₆ alkoxy; and (b)contacting the reactive intermediate with said surface so as to immobilized the molecule onto said surface.

Nanoparticles include metal (e.g., gold, silver, copper and platinum), semiconductor (e.g., CdSe, CdS, and CdS or CdSe coated with ZnS) and magnetic (e.g., ferromagnetite) colloidal materials. Other nanoparticles useful in the practice of the invention include ZnS, ZnO, TiO₂, AgI, AgBr, HgI₂, PbS, PbSe, ZnTe, CdTe, In₂ S₃, In₂ Se₃, Cd₃ P₂, Cd₃ As₂, InAs, and GaAs. The size of the nanoparticles is preferably from about 5 nm to about 150 nm (mean diameter), more preferably from about 5 to about 50 nm, most preferably from about 10 to about 30 nm. The nanoparticles may also be rods. Other nanoparticles include silica and polymer (e.g. latex) nanoparticles.

Methods of making metal, semiconductor and magnetic nanoparticles are well-known in the art. See, e.g., Schmid, G. (ed.) Clusters and Colloids (VCH, Weinheim, 1994); Hayat, M. A. (ed.) Colloidal Gold: Principles, Methods, and Applications (Academic Press, San Diego, 1991); Massart, R., IEEE Taransactions On Magnetics, 17, 1247 (1981); Ahmadi, T. S. et al., Science, 272, 1924 (1996); Henglein, A. et al., J. Phys. Chem., 99, 14129 (1995); Curtis, A. C., et al., Angew. Chem. Int. Ed. Engl., 27, 1530 (1988). Methods of making silica nanoparticles impregnated with fluorophores or phosphors are also well known in the art (see Tan and coworkers, PNAS, 2004, 101, 15027-15032).

Methods of making ZnS, ZnO, TiO₂, AgI, AgBr, HgI₂, PbS, PbSe, ZnTe, CdTe, In₂ S₃, In₂ Se₃, Cd₃ P₂, Cd₃ As₂, InAs, and GaAs nanoparticles are also known in the art. See, e.g., Weller, Angew. Chem. Int. Ed. Engl., 32, 41 (1993); Henglein, Top. Curt. Chem., 143, 113 (1988); Henglein, Chem. Rev., 89, 1861 (1989); Brus, Appl. Phys. A., 53, 465 (1991); Bahncmann, in Photochemical Conversion and Storage of Solar Energy (eds. Pelizetti and Schiavello 1991), page 251; Wang and Herron, J. Phys. Chem., 95, 525 (1991); Olshavsky et al., J. Am. Chem. Soc., 112, 9438 (1990); Ushida et al., J. Phys. Chem., 95, 5382 (1992).

Suitable nanoparticles are also commercially available from, e.g., Ted Pella, Inc. (gold), Amersham Corporation (gold) , Nanoprobes, Inc. (gold), and Quantom Dot Inc. (core-shell semiconductor particles such as CdSe/ZnS).

The nanoparticles, the oligonucleotides or both are functionalized in order to attach the oligonucleotides to the nanoparticles. Such methods are known in the art. For instance, oligonucleotides functionalized with alkanethiols at their 3'-termini or 5'-termini readily attach to gold nanoparticles. See Whitesides, Proceedings of the Robert A. Welch Foundation 39th Conference On Chemical Research Nanophase Chemistry, Houston, TX, pages 109-121 (1995). See also, Mucic et al. Chem. Commun. 555-557 (1996) (describes a method of attaching 3' thiol DNA to flat gold surfaces; this method can be used to attach oligonucleotides to nanoparticles). The alkanethiol method can also be used to attach oligonucleotides to other metal, semiconductor and magnetic colloids and to the other nanoparticles listed above. Other functional groups for attaching oligonucleotides to solid surfaces include phosphorothioate groups (see, *e.g*., U.S. Patent No. 5,472,881 for the binding of oligonucleotide-phosphorothioates to gold surfaces), substituted alkylsiloxanes (see, *e.g.* Burwell, Chemical Technology, 4, 370-377 (1974) and Matteucci and Caruthers, J. Am. Chem. Soc., 103, 3185-3191 (1981) for binding of oligonucleotides to silica and glass surfaces, and Grabar et al., Anal. Chem., 67, 735-743 for binding of aminoalkylsiloxanes and for similar binding of mercaptoaklylsiloxanes). Oligonucleotides terminated with a 5' thionucleoside or a 3' thionucleoside may also be used for attaching oligonucleotides to solid surfaces. The following references describe other methods which may be employed to attached oligonucleotides to nanoparticles: Nuzzo et al., J. Am. Chem. Soc., 109, 2358 (1987) (disulfides on gold); Allara and Nuzzo, Langmuir,1, 45 (1985) (carboxylic acids on aluminum); Allara and Tompkins, J. Colloid Interface Sci., 49, 410-421 (1974) (carboxylic acids on copper); Iler, The Chemistry Of Silica. Chapter 6, (Wiley 1979) (carboxylic acids on silica); Timmons and Zisman, J. Phys. Chem., 69, 984-990 (1965) (carboxylic acids on platinum); Soriaga and Hubbard, J. Am. Chem. Soc., 104, 3937 (1982) (aromatic ring compounds on platinum); Hubbard, Acc. Chem. Res., 13, 177 (1980) (sulfolanes, sulfoxides and other functionalized solvents on platinum); Hickman et al., J. Am. Chem. Soc., 111, 7271 (1989) (isonitriles on platinum); Maoz and Sagiv, Langmuir, 3, 1045 (1987) (silanes on silica); Maoz and Sagiv, Langmuir, 3, 1034 (1987) (silanes on silica); Wasserman et al., Langmuir, 5, 1074 (1989) (silanes on silica); Eltekova and Eltekov, Langmuir, 3, 951 (1987) (aromatic carboxylic acids, aldehydes, alcohols and methoxy groups on titanium dioxide and silica); Lec et al., J. Phys. Chem., 92, 2597 (1988) (rigid phosphates on metals). U.S. Patent no. 6,767,702 also describes methods for attaching oligonucleotides to nanoparticles using cyclic disulfides and polythiols.

In one aspect, the surface is a glass surface.

In another aspect, the surface has at least one group that reacts with the reactive intermediate. Representative examples of groups include hydroxyl, amino, or carboxylate group. A non-limiting example of agent includes 3-(isocyanatopropyl) triethoxysilane or 3-(isocyanatopropyl)dimethylmonoethoxysilane.

In another aspect, the oligonucleotides may be bound to the nanoparticle through a functional moiety such as a thiotic acid, alkyl thiol or disulfide group (e.g., epiandrosterone disulfide)

A method is provided for immobilizing a nanoparticle onto a surface. The method comprises the steps of: (a) providing a substrate having a surface comprising reactive moieties that reacts with amine groups and a nanoparticle having oligonucleotides bound thereto, at least a portion of the oligonucleotides have a amine group at an end not bound to the nanoparticle; and (b) contacting the reactive moieties with the nanoparticle so as to immobilized the nanoparticles onto said surface.

In one aspect, the surface is a glass surface.

In another aspect, the surface has at least one group that reacts with the reactive intermediate. Representative examples of groups include hydroxyl, amino, or carboxylate group. A non-limiting example of agent includes 3-(isocyanatopropyl) triethoxysilane or 3-(isocyanatopropyl)dimethylmonoethoxysilane.

In another aspect, the oligonucleotides may be bound to the nanoparticle through a functional moiety such as a thiotic acid, alkyl thiol or disulfide group (e.g., epiandrosterone disulfide)

In another aspect, the reactive moieties comprise isocyanates, anhydrides, acyl halides, or aldehydes.

Kits are provided for preparing modified substrates. The kits may include optional reagents for silyating molecules and optional substrtes, buffers for carrying out assays including washing and binding steps.

### EXAMPLES

The invention is demonstrated further by the following illustrative examples. The examples are offered by way of illustration and are not intended to limit the invention in any manner. In these examples all percentages are by weight if for solids and by volume if for liquids, and all temperatures are in degrees Celsius unless otherwise noted.

### Example 1: Preparation of DNA array chips

This Example provides a general procedure for the covalent attachment of a molecule, e.g., 3' or 5'-silylated DNA, directly to surfaces such as pre-cleaned glass surface via single silylated molecule or dendritic silylated molecule procedure.

### (a) Method No. 1

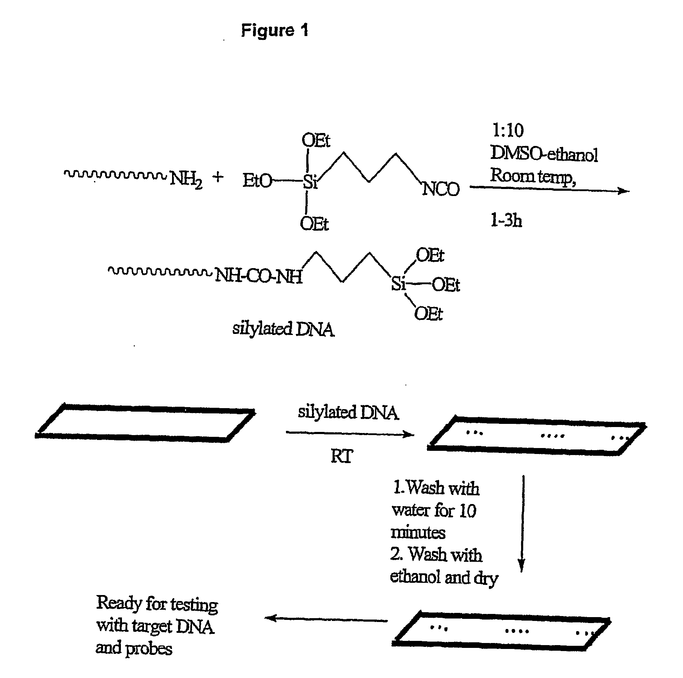

As shown in Figure 1, a method is shown for attaching a 3'-amino or 5'-amino DNA molecule to a pre-cleaned glass surface. 3'-Amine linked DNA is synthesized by following standard protocol for DNA synthesis on DNA synthesizer. The 3' amine modified DNA synthesized on the solid support was attached through succinyl linker to the solid support. After synthesis, DNA attached to the solid support was released by using aqueous ammonia, resulting in the generation of a DNA strand containing a free amine at the 3'-end. The crude material was purified on HPLC, using triethyl ammonium acetate (TEAA) buffer and acetonitrile. The dimethoxytrityl (DMT) group was removed on the column itself using triflouroacetic acid.

After purification, 1 equivalents of 3'-amine linked DNA was subsequently treated with 1.2 equivalents of triethoxysilyl isocyanate (GELEST, Morrisville, PA, USA) for 1-3h in 10% DMSO in ethanol at room temperature. Traces of water that remained in the DNA following evaporation did not effect the reaction. After 3h, the reaction mixture was evaporated to dryness and spotted directly on pre-cleaned glass surface using an arrayer (Affymetrix, GMS 417 arrayer with 500 micron pins for spotting). Typically, 1mM silylated DNA was used to array a glass surface and the arrayed substrate is then kept in the chamber for 4h-5h. Thereafter, the slides were incubated in nanopure water for 10 minutes to remove the unbound DNA, washed with ethanol, and dried in the dessicator. After drying, these plates were tested with target DNA samples.

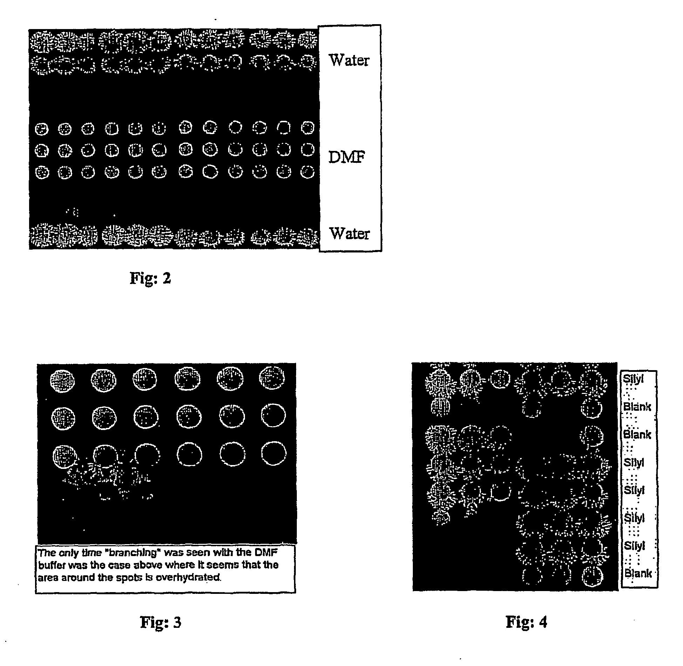

In a preliminary study using linear silyl oligonucleotides prepared by the above procedure to spot a glass surface, it was observed that spotting in DMSO or DMF medisurprisingly controlled spot branching or diffusion. See Figure 2. The spot morphology was clean and discrete. If the substrate was overhydrated in the dessicator chamber prepared by filing a portion of a chamber with water and storing the glass slides on a rack above the water level overnight, the slides become overhydrated.. Undesirable branching of the spot was observed on overhydrated slides, even when DMSO or DMF solvent is used. See Figure 3. When water was used as the sole solvent for spotting, the resultant spots were branched out and spread to other spots. See Figure 4. Without being bound to any theory of operation, an aqueous spotting solution and/or the presence of water in a overhydrated substrate results in the polymerization of silyl oligonucleotides and thus interfered with the modification of the surface with the desired molecule. Thus, dried polar aprotic solvents such as DMF, DMSO and dried polar solvents like ethanol, isopropanol and mixture of solvents like DMF/Pyridine were found to be suitable solvents for arraying the silyl modified oligonucleotides. The presence of water (>1%) in the spotting solution or over hydration of slidesresults in spot branching after arraying. Spot branching is undesirable because it may lead to false positive results in binding studies.

### (b) Method No. 2

As shown in Figure 5, a method is shown for attaching multiple 5' or 3' amino DNA molecules to a glass surface. To 1 equivalent of silyl amine in dry acetonitrile, 1.2 equivalents of tetraisocyante is added dropwise and the reaction mixture is stirred at room temperature for 10 minutes to form compound 3. 5' or 3'-amine linked oligonucleotide is synthesized and deprotected using aqueous ammonia conditions by conventional procedures. After HPLC purification, 5' or 3'-amine free oligonucleotide is treated with compound 3 in a 1:10 DMSO/ethanol (v/v) mixture. After 10 minutes, the modified oligonucleotides are evaporated under vacuum and spotted on unmodified glass surface in DMSO or DMF media.

### Example 2: Detection of Factor V target sequence using a DNA array chip

This Example illustrates that DNA plates prepared as described in Example 1 are useful for sandwich hybridization assays for detection of nucleic acid targets.

### (a) Gold Colloid preparation:

Gold colloids (13 nm diameter) were prepared by reduction of HAuCl₄ with citrate as described in Frens, Nature Phys. Sci., 241, 20 (1973) and Grabar, Anal. Chem., 67, 735 (1995). Briefly, all glassware was cleaned in aqua regia (3 parts HCl, 1 part HNO₃), rinsed with Nanopure H₂O, then oven dried prior to use. HAuCl₄ and sodium citrate were purchased from Aldrich Chemical Company. Aqueous HAuCl₄ (1 mM, 500 mL) was brought to reflux while stirring. Then, 38.8 mM sodium citrate (50 mL) was added quickly. The solution color changed from pale yellow to burgundy, and refluxing was continued for 15 min. After cooling to room temperature, the red solution was filtered through a Micron Separations Inc. 1 micron filter. Au colloids were characterized by UV-Vis spectroscopy using a Hewlett Packard 8452A diode array spectrophotometer and by Transmission Electron Microscopy (TEM) using a Hitachi 8100 transmission electron microscope. Gold particles with diameters of 13 nm will produce a visible color change when aggregated with target and probe oligonucleotide sequences in the 10-35 nucleotide range.

### (b) Synthesis Of Oligonucleotides:

Oligonucleotides were synthesized on a 1 micromole scale using a Milligene Expedite DNA synthesizer in single column mode using phosphoramidite chemistry. Eckstein, F. (ed.) *Oligonucleotides and Analogues: A Practical Approach* (IRL Press, Oxford, 1991). All solutions were purchased from Milligene (DNA synthesis grade). Average coupling efficiency varied from 98 to 99.8%, and the final dimethoxytrityl (DMT) protecting group was cleaved from the oligonucleotides to do final epiendrosterone coupling on the synthesizer itself. Capture strands were synthesized with DMT on procedure and purified on HPLC system.

### (c) Purification of oligonucleotides

Reverse phase HPLC was performed with using Agilent 1100 series system equipped with Tosch Biosep Amberchrom MD-G CG-300S column(10x118mm, 35µm particle size) using 0.03 M Et₃NH⁺ OAc⁻ buffer (TEAA), pH 7, with a 1%/min. gradient of 95% CH₃CN/5% TEAA. The flow rate was 1 mL/ min. with UV detection at 260 nm. The final DMT attached was deprotected on HPLC column itself using 1-3 % trifluoro acetic acid and TEAA buffer. After collection and evaporation of the buffer contained the DMT cleaved oligonucleotides, was then evaporated to near dryness. The amount of oligonucleotide was determined by absorbance at 260 nm, and final purity assessed by reverse phase HPLC.

The same protocol was used for epiendrosterone linked-oligonucleotides for probe preparation and no DMT removal needed ¹⁰.

### (d) Attachment Of Oligonucleotides To Gold Nanoparticles

Probes used in the Example: (3'-act tta aca ata g-a₂₀-Epi-5'and 3'-t taa cac tcg c - a20-Epi-5') (SEQ ID NO:1) was attached in the following fashion. These probes were designed for M13 target sequence detection.

A 1 mL solution of the gold colloids (15nM) in water was mixed with excess (3.68 :M) 5'epi-endrosterone linked-oligonucleotide (33 and 31 bases in length) in water, and the mixture was allowed to stand for 12-24 hours at room temperature. Then, 100 µL of a 0.1 M sodium hydrogen phosphate buffer, pH 7.0, and 100 µL of 1.0 M NaCl were premixed and added. After 10 minutes, 10 µL of 1% aqueous NaN₃ were added, and the mixture was allowed to stand for an additional 20 hours then increased the salt concentration to 0.3. After standing 4h at 0.3 M NaCl again increased to 1M Nacl and kept further 16h. This "aging" step was designed to increase the surface coverage by the epi disulfide linked-oligonucleotides and to displace oligonucleotide bases from the gold surface. Somewhat cleaner, better defined red spots in subsequent assays were obtained if the solution was frozen in a dry-ice bath after the 40-hour incubation and then thawed at room temperature. Either way, the solution was next centrifuged at 14,000 rpm in an Eppendorf Centrifuge 5414 for about 15 minutes to give a very pale pink supernatant containing most of the oligonucleotide (as indicated by the absorbance at 260 nm) along with 7-10% of the colloidal gold (as indicated by the absorbance at 520 nm), and a compact, dark, gelatinous residue at the bottom of the tube. The supernatant was removed, and the residue was resuspended in about 200 µL of buffer (10 mM phosphate, 0.1 M NaCl) and recentrifuged. After removal of the supernatant solution, the residue was taken up in 1.0 mL of buffer (10 mM phosphate, 0.1 M NaCl) and 10 µL of a 1% aqueous solution of NaN₃. Dissolution was assisted by drawing the solution into, and expelling it from, a pipette several times. The resulting red master solution was stable (*i.e.*, remained red and did not aggregate) on standing for months at room temperature, on spotting on silica thin-layer chromatography (TLC) plates, and on addition to 2 M NaCl, 10 mM MgCl₂, or solutions containing high concentrations of salmon sperm DNA.

For examples 2-5 we prepared different set of Factor V probes using an aqueous solution of 17nM (150 µL) Au colloids, as described above, was mixed with 3.75 µM (46 µL) 5'-epiendrosterone- a₂₀-tattcctcgcc (SEQ ID NO:2), and allowed to stand for 24 hours at room temperature in 1 ml Eppendorf capped vials. A second solution of colloids was reacted with 3.75 µM (46 µL) 5'-epiendrosterone-a₂₀- attccttgcct-3'.(SEQ ID NO:3). Note that these oligonucleotides are non-complementary. The residue was dissolved using the same procedure described above and the resulting solution was stored in a glass bottle until further use.

### (e) Hybridization conditions

Stock buffer solution: For the hybridization buffer, the following stock solution was used: 3.0 NaCl, 0.3 M Na-Citrate, 10mM MgCl₂, 4.0 mM NaH₂PO₄ and 0.005% SDS.

Hybridization assay was performed using diluted buffer (0.78M NaCI, 70 mM sodium citate, 2.64 mM MgCl₂, 1.1mM sodium phosphate, 0.01%) from the stock buffer solution by adding 0.5% of Tween. In a typical experiment procedure, target and probe were mixed with the hybridization buffer and heated the mixture at 95°C for 5minutes. After cooling to room temperature aliquots were transferred on to the glass substrate and placed in humidity chamber for hybridization (Different assays were done at different temperature conditions since each probe has a different melting temperature). After hybridization, plates were washed with two different wash buffers and spin dried. Plates dried were treated with silver amplification solutions (silverA+silverB) (silver amplification kit available from SIGMA, St.Louis, MO 63178, catalog no: S 5020 and S 5145) and the data was collected from the amplified plates using an imaging system for data collection described in (Nanosphere, Inc. assignee) U.S. Patent application no. 10/210,959 and PCT/US02/24604, both filed August 2, 2002, which are incorporated by reference in their entirety.

### (f) Target Sequence used

This Factor V target sequence was used in examples 2-6 for detection. M13 probes were used in example 1 for direct probe targeting to capture strand test the plates and no target detection was performed here. But from example 2-5 Factor V target detection was done in presence of Factor V probes and M13 probes. Here M13 probes served as controls. In plate no: 5 different combination of assay were performed on one plate including Factor V wild type and mismatch detection. Each well in plate no:6 was clearly defined with target and probes used.

### Factor V wild type sequence:

5’gacatcgcctctgggctaataggactacttctaatctgtaagagcagatccctggacaggcaaggaatacaggtattttgtcctt
gaagtaacctttcag 3’ (SEQ ID NO:4)

### Probe sequence:

Probe FV (13D): 5'-Epi-a₂₀- tattcctcgcc 3' (SEQ ID NO: 5)
Probe FV (26D): 5'-Epi-a₂₀-attccttgcct3' (SEQ ID NO: 6)

### Capture Strand Sequence For factor V target detection:

5'-tcc tga tga aga tta gac att ctc gtc- NH-CO-NH- Si-(OEt)₃-3' (SEQ ID NO:7)

Stock buffer solution: For the hybridization buffer, the following stock solution was used: 3.0 NaCI, 0.3 M Na-Citrate, 10mM MgCl₂, 4.0 mM NaH₂P04 and 0.005% SDS.

### Example 3: Detection of M13 target sequence using DNA array chip

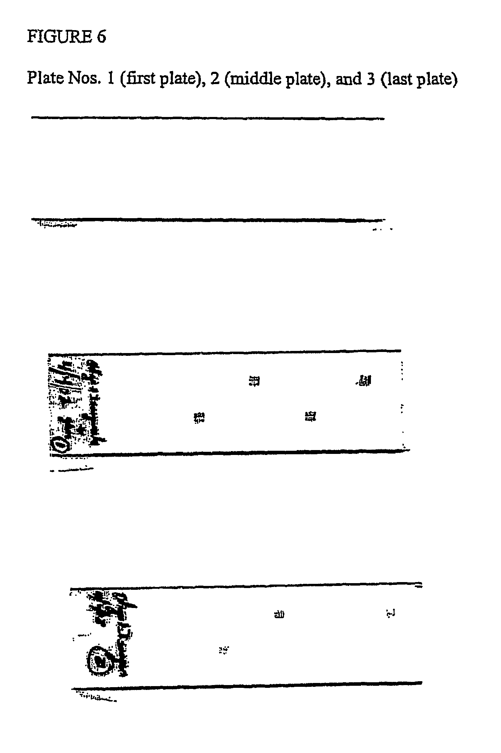

In this Example, probe was targeted directly to the capture strand and a detection assay was performed. Plates Nos. 1-3 were prepared as described in Example 1 (method no. 1). In Plates 2 & 3, probes (Figure 6) were clearly hybridized to the capture strand within 45 minutes. The gold colloid nanoparticles hybridized to the capture were clearly visible before silver amplification. In plate no 1 (Figure 6), a different probe was used and the assay was developed to show the specificity. After silver stain development, signals were not shown on the glass surface even after silver amplification. This experiment established the specificity of the DNA chip prepared in accordance with the invention.

### M13 Capture sequence:

5'-tga aat tgt tat c- NH-CO-NH-- Si-(OEt)₃-3' (SEQ ID NO: 8)

### Probe used on plates Nos. 2- 3 plates:

3'-act tta aca ata g-a₂₀-Epi-5 ' (SEQ ID NO: 9)

On plate no.1, a detection probe 3'-t taa cac tcg c-a₂₀-Epi-5 ' (SEQ ID NO: 10) was used which was non-complementary to the capture strand for sequence specificity testing (no signals). This clearly showed the specificity of the both capture strand sequence and the probe. In both cases, 6nM probe was used in diluted buffer conditions. In a typical experimental procedure, 3µl of the diluted buffer (1.3M NaCl, 130mM sodium citrate, 4.38mM MgCl₂, 1.82mM sodium phosphate, 0.003% SDS) and 20µl of probe (10nM) was flooded on the arrayed glass chip and allowed to hybridize for 1.5h at room temperature. The final concentration of probe was 4nM and buffer concentration was 0.78M NaCl, 70 mM sodium citrate, 2.64mM MgCl₂, 1.1mM sodium phosphate, 0.002% SDS. Thereafter, the chip was washed with 0.75 M sodium chloride, 75mM citrate and 0.05% Tween buffer and then washed again with 0.5M sodium nitrate buffer. Then plates were treated with silver amplification solutions silver A+ SilverB (1mL+1mL = total 2mL) for 4 minutes and washed with nanopure water. Finally, the plates were exposed to the imaging system for data collection as discussed above.

### Example 4: Detection of Factor V target sequence using a DNA array chip

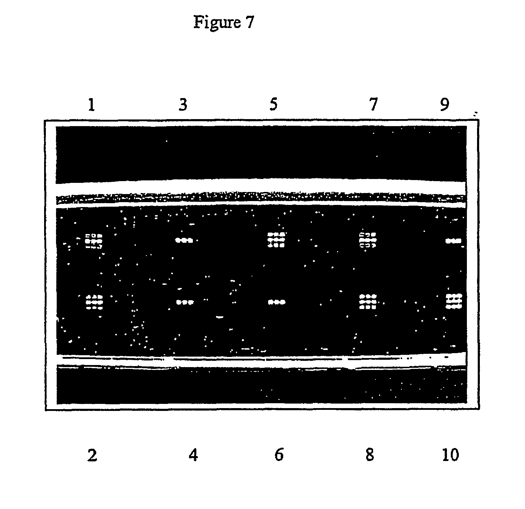

In this Example, two different silanized capture strands were spotted directly on the plate and detected. The plate was prepared as described in Example 1 (method no. 1). The middle row always carried the positive control capture with other capture on top and bottom rows. Here, wild type, mutant and heterozygous samples were used for the detection. All samples were showed signals in the proper place using the above mentioned assay conditions. See Figure 7.
a) Positive controls capture sequence:
   5'-tga aat tgt tat c- NH-CO-NH- Si-(OEt)₃-3' (SEQ ID NO:10)
   Probe used was for positive control:
   3'-act tta aca ata g-a₂₀-Epi-5' (SEQ ID NO:11)
b) Probes used for target detection are:
   Probe FV 13D (probe for wild type target): 5'-Epi-a₂₀-tattcctcgcc 3' (SEQ ID NO:12)
   Probe FV 26D (probe for mutant target): 5'-Epi-a₂₀-attccttgcct3' (SEQ ID NO: 13)

### Capture Strand Sequence For factor V target detection:

5'-tcc tga tga aga tta gac att ctc gtc- NH-CO-NH-- Si-(OEt)₃-3'

### Factor V wild type target sequence:

5'gacatcgcctctgggctaataggactacttctaatctgtaagagcagatccctggacaggcaaggaatacaggtattttgtcctt
gaagtaacctttcag 3' (SEQ ID NO: 13)

### Mutant Factor V target sequence:

gtaggactacttctaatctgtaagagcagatccctggacaggtaaggaatacaggtattttgtccttgaagtaacctttcag-3' (SEQ ID NO: 14)

Heterozygous: 50% of wild type and 50% of mutant target.
Well 1: Heterozygous- Probe 26D was used
Well 2: Heterozygous- Probe 13D was used
Well 3: Control- with probe 26D, only positive control should show up
Well 4: Control- with probe13D, only positive control should show up
Well 5: Mutant - target with mutant probe 26D + positive control probe
Well 6: Mutant target -with wild type probe 13D + positive control
Well 7: Heterozygous- with probe 26D
Well 8: Heterozygous- with probe 13D
Well 9: Wild type target - with mutant probe 26D
Well 10: Wild type target- with wild type probe13D

### Example 5: Detection of MTHFR target sequence on a DNA array plate

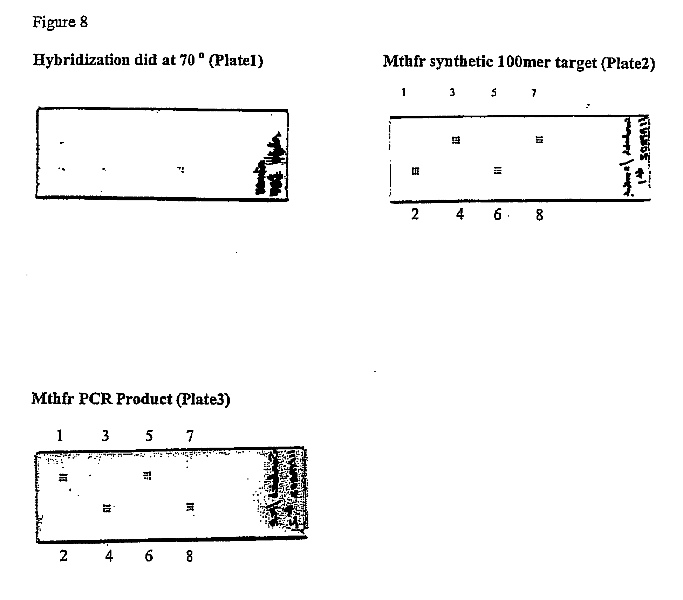

In this Example, an MTHFR 100mer synthetic target and 208 base pair PCR product (10nM ∼50nM) was used in the detection assay. The plates were prepared as described in Example 1 (method no. 1). Alternative wells were used as controls using M13 target and MTHFR 18mer probe and did not show even traces of silver, following silver signal amplification. As shown in plate no. 1 (Figure 8), an experiment was performed at 70°C to show that probe does not hybridize above melting temperature (MTHFR target and 18mer probe). The results show probe specificity and that at high temperature, the probes are not binding nonspecifically to the silyl oligo-attached substrate.

### 100mer synthetic target:

5’-aag cac ttg aag gag aag gtg tct gcg gga gcc gat ttc atc atc acg cag ctt ttc ttt gag gct gac
aca ttc ttc cgc ttt gtg aag gca tgc acc ga-3’ (SEQ ID NO: 14)

### 18mer probe sequence used on all three plates:

3'-ctg tgt aag aag gcg ttt-A₂₀-Epi-5' (SEQ ID NO: 15)

### PCR product: 208 base pair

5'ccttgaacaggtggaggccagcctctcctgactgtcatccctattggcaggttaccccaaaggccaccccgaagcagggag
ctttgaggctgacctgaagcacttgaaggagaaggtgtctgcgggagccgatttcatcatcacgcagcttttctttgaggctgaca
cattcttccgctttgtgaaggcatgcaccgacatgggcatcacttgccccatcgtccccgggatctttcccatccaggtgaggggc
ccaggagagcccataagctccctccaccccactctcaccgc (SEQ ID NO:16)

### Experimental conditions:

In a typical experimental procedure (on plate no:2), to 30µl of the diluted buffer (1.3M NaCl, 130mM sodium citrate, 4.38mM MgCl₂, 1.82mM sodium phosphate, 0.003% SDS),10µl of 18mer probe ( 10nM) and 2 µl of 100mer synthetic target (10 µM ) 8µl of water were mixed and flooded on the arrayed glass chip and allowed to hybridize for 1.5h at room temperature. The final concentration of probe was 2nM and target concentration was 400pM and buffer concentration was 0.78M NaCl, 70 mM sodium citate, 2.64 mM MgCl₂, 1.1mM sodium phosphate, 0.01%). After that washed with 0.75 M sodium chloride, 75mM citrate and 0.05% Tween buffer and then washed again with 0.5M sodium nitrate buffer. After that plates were treated with silver A+ SilverB (1mL+1mL = total 2mL) (silver amplification kit available from SIGMA, St.Louis, MO 63178, catalog no: S 5020 and S 5145) for 4 minutes and washed with nanopure water. Finally plates were exposed to imaging system for data collection as discussed above. In Example 3 on plate no:2, wells no: 2 1,4, 5, 8 are controls and controls made up with M13 synthetic target and MTHFR 18mer probe (5'-tat gct tcc ggc tcg tat gtt gtg tgg aat tgt gag cgg ata aca att tca-3'). (SEQ ID NO: 17)

As mentioned earlier, the experiment on plate no.1 (Figure 8) was performed at 70 °C to show that above melting temperature probe 18mer probe did not bind to the capture probe.

Plate no.3 (Figure 8) was generated following the same experimental procedure and using the same probes. 10 µl (2nm∼10nM) of MTHFR PCR product was used as target. Plate no.3 wells 2, 3, 6 and 7 are the controls with Factor V 99mer mutant target and MTHFR 18mer probe.

### Factor V 99mer Mutant Factor V target had the following sequence:

5'gtaggactacttctaatctgtaagagcagatccctggacaggtaaggaatacaggtattttgtccttgaagtaacctttcag-3') (SEQ ID NO: 18)

### Example 6: Detection of Factor V target sequence on DNA array plate

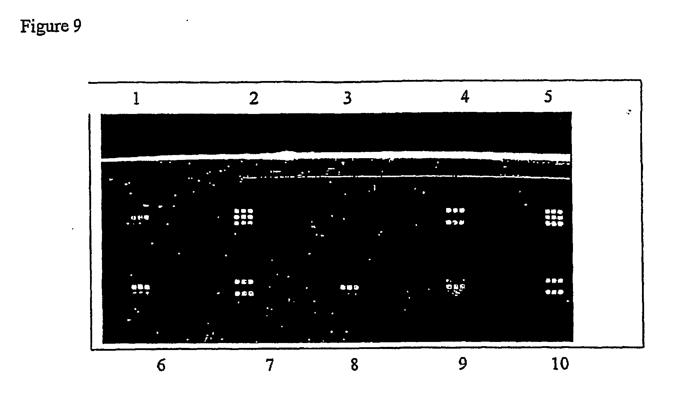

In this Example and in the following Example 7, the same capture strands were arrayed on the plate. The purpose of this experiment was to find out the difference in intensity of the spots after silver development when same oligomer was spotted on the slide at different places. Positive control was spotted in the middle of two Factor V 4G oligomer captures on the slide. The results are shown in Figure 9.

### Capture strand sequence for Factor V target detection was:

5' tcc tga tga aga tta gac att ctc gtc-NH-CO-NH- Si-(OEt)₃-3' (SEQ ID N0:19)

### Positive capture control capture spotted was (M13):

5' tga aat tgt tat c-NH-CO-NH-- Si-(OEt)₃-3' (SEQ ID NO:20)

The target sequence used was wild type Factor V 99base pair single strand DNA having the following sequence:
gtaggactacttctaatctgtaagagcagatccctggacaggcaaggaatacaggtattttgtccttgaagtaacctttcag-3') (SEQ ID NO:21)

### Mutant Factor V target had the following sequence:

gtaggactacttctaatctgtaagagcagatccctggacaggtaaggaatacaggtattttgtccttgaagtaacctttcag-3') (SEQ ID NO:22)
and probes used had the following sequence:
probe FV 13D: 5'-Epi-a₂₀-tattcctcgcc 3' (SEQ ID NO:23),
probe FV 26D: 5 ' -Epi-a₂₀-attccttgcct3 ' (SEQ ID NO:24).

### Capture Strand Sequence for factor V target detection:

5'-tcc tga tga aga tta gac att ctc gtc- NH-CO-NH-- Si-(OEt)₃-3' (SEQ ID NO:25)

Positive control sequence: 5'-tga aat tgt tat c-NH₂-3' (SEQ ID NO: 26)
and probe used for positive control was: 3'-act tta aca ata g-a₂₀-Epi-5' (SEQ ID NO: 27)

In a typical experimental procedure, to 25µl of the diluted buffer (1.3M NaCl, 130mM sodium citrate, 4.38mM MgCl₂, 1.82mM sodium phosphate, 0.003% SDS),10µl of probe (10nM) and 10 µl of PCR target (15-50nM) and 5 µl of positive control probe (10nM) were mixed and flooded on the arrayed glass chip and allowed to hybridize for 1.5h at room temperature. The final concentration of probe was 2nM, and buffer concentration was 0.78M NaCl, 70 mM sodium citate, 2.64 mM MgCl₂, 1.1mM sodium phosphate, 0.01%), that the plates was then washed with 0.75 Sodium chloride, 75mM citrate and 0.05% tween buffer and then washed again with 0.5M Sodium Nitrate buffer. The plates were treated with silver A+ SilverB (1mL+1mL = total 2mL) for 4 minutes and washed with nanopure water. Finally, the plates were exposed to the imaging system described above for data collection. Both positive control probe and target reacted probe were mixed and the assay was run to show the selectivity of the probe. The wells were identified as follows:
Wells 1, 6, 8 and 9 have only positive control probe with target and buffer.
Wells 2, 5 had both positive control probe and target probe with targets and buffer.
Wells 4, 7 and 10 have only target probe with target and buffer and here positive control probe and target were absent.
Well 3 did not have any target and positive control probe but it had target probe and buffer.

These results (Figure 9) show that probes were specific to target detection and no non-specific background noise was observed when target was absent.

### Example 7: Detection of Factor V target sequence

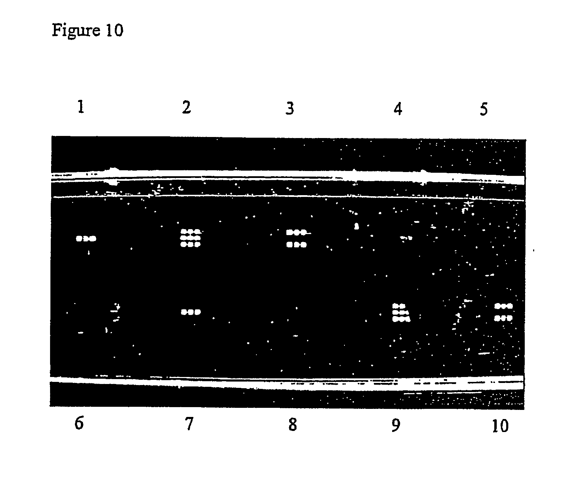

In this Example, all capture strands pattern is the same as described in Example no.6. Moreover, the same experimental conditions and concentrations described in Example 6 were used to perform the assay at 52°C. Wild type and mutant targets were given in the example 6. The results are shown in Figure 10. The wells are identified as follows:
Well 1: Positive control probe directly probing to the capture strand in the same buffer conditions mentioned in example 4.
Well 2: Factor V Probe 5'-Epi-a₂₀-attccdgcct-3 ' (26D) (SEQ ID NO: 27) and Factor V 99base pair mutant target, positive control probe and buffer.
Well 3: Factor V Probe 5'-Epi-a₂₀-attccUgcct-3 ' (26D) (SEQ ID NO: 28) and Factor V 99base pair mutant target and hybridization buffer.
Well 4: Probe 13D and Factor V mutant PCR target, positive control and hybridization buffer.
Well 5: Probe 13D and Factor V mutant PCR target, and hybridization buffer.
Well 6: Control (MTHFR target and Probe 13D and hybridization buffer).
Well 7: Wild type Factor V target, probe (26D), positive control probe and hybridization buffer,
Well 8: Wild type Factor V target and probe (26D), and hybridization buffer.
Well 9: Wild type Factor V target, probe 13(D), positive control probe and hybridization buffer.
Well 10: Wild type Factor V target, probe 13(D), and hybridization buffer.
Probe FV 13D: 5'-Epi-a₂₀-tattcctcgcc-3' (SEQ ID NO: 29)
Probe FV 26D: 5'-Epi-a₂₀-attccttgcct-3' (SEQ ID NO: 30)

These results (Figure 10) show that probes were reacted specifically to the target and there is no cross hybridization between probes and targets were observed when probes were mixed with different targets.

### Example 8: Protocol for preparing polymer-coated substrates

A general protocol for preparing polymer-coated substrates and for printing amine modified DNA on the polymer coated surface is as follows.

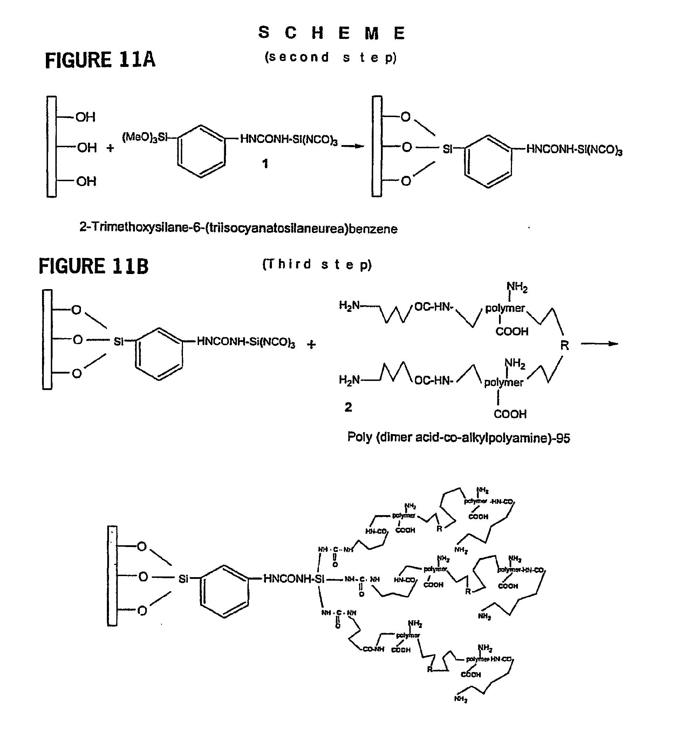

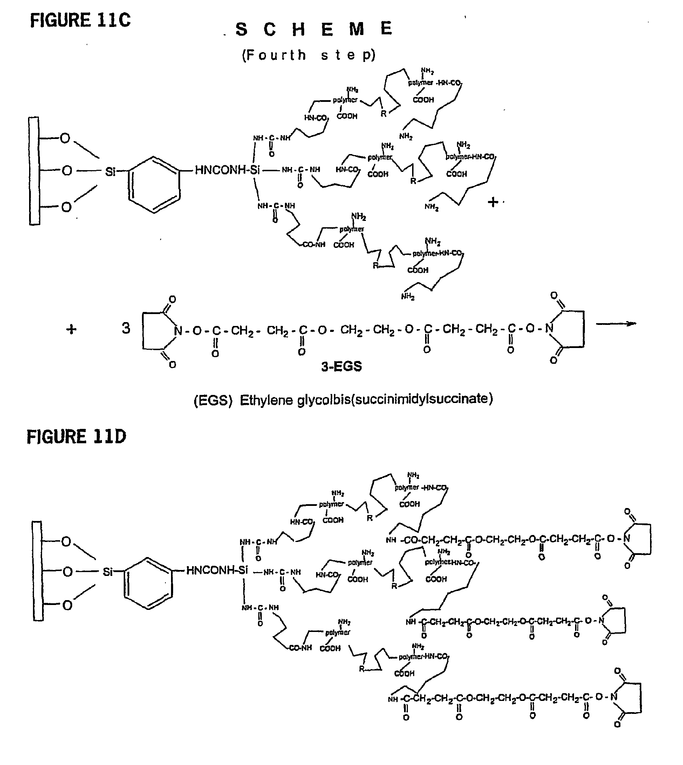

Amine modified DNA is made from gene synthesizer using standard protocols and purified on HPLC. Oligonucleotides are printed using pH 8.5 300mM phosphate buffer using GMS Affymatrix® arrayer. Figure 11 is a schematic showing a representative method for preparing a substrate for detecting target analytes involving modifying a substrate with a isocyanate compound (2-trimethoxysilane-6-(triisocyanatosilaneurea)benzene) to form a surface having isocyanate groups, contacting the surface having the isocyanate groups with a spacer molecule (poly(dimmer acid-co-alkylpolyamine)-95) having a plurality of amino groups to form a surface having free amino groups, contacting the surface having free amino groups with a linker molecule (EGS) to form a surface having reactive moieties. The resulting surface can be used to attach capture probes such as nucleic acids molecules.

### Materials used:

| | |
|---|---|
| *Glass slides:* | Gold seal products, catalogue no: 3011 Fisher Scientific, Catalogue no: 12-544-1 |
| *Silanes:* | 3-(Triethoxysilyl)propylisocyanate, Sigma-Aldrich, Ctalogue no: 41336-4. m-aminophenyltrimethoxysilane, Gelest, catalogue no:SIA0599.0 Tetraisocyanatosilane, Gelest, catalogue no: SIT125.0 |
| *Polymers:* | Poly(dimmer acid-co-alkylpolyamine)-140, Sigma-Aldrich, Catalogue no: 191043 Poly(dimmer acid-co-alkylpolyamine)-90, Sigma-Aldrich, Catalogue no: 191019 Neomycin, Sigma-Aldrich, Catalogue no: N1142 Poly(allylamine), Sigma-Aldrich, Catalogue no: 479144 Poly(m-xylylendiamine-epichlorohydrine), diamine terminated, Sigma-Aldrich, Catalogue no: 456888 Tris(2-aminoethylamine), Sigma-Aldrich, Catalogue no: 22563-0 Panam Dendrimer Generation7, Sigma-Aldrich, Cat no: 53672-5 |
| *Linkers:* | Ethyleneglycol-bis-(succinimidyl-succinate) EGS, Pierce, cat no: 21565 Disuccinimidyl Suberate,(DSS), Pierce, cat no:21555 1,6 Diisocyanatohexane, Sigma-Aldrich, Catalogue no: D 12470-2 Glutaric Dialdehyde, Sigma-Aldrich, Catalogue no: 34085-5 |

***Cleaning Slides:*** All the slides were first soaked in NaOH (5% in water) for 30 minutes at room temperature and washed with water to obtain the pH of 7. Then they were soaked in 5%-HCl for 30 minutes at room temperature and again washed one time with water. Finally, the slides were treated with 3% H2O2 in 5%-HCl for 3 hours at room temperature, washed with water three times (till pH=7) and with Ethyl Alcohol three times, and dried by spinning. Afterward, the air-dried slides were cured in the oven overnight at 120 OC

### Step1: Synthesis of 2-Trimethoxysilane-6-(triisocyanatosilaneurea) benzene:

1.96 (0.01M) of Tetraisocyanatosilane and 2.13g (0.01M) of m-Aminophenyltrimethoxysilane were mixed and stirred for 20 minutes at room temperature. 200 mL of Ethyl alcohol was added to the mixture and stirred additional 60 minutes at room temperature **(see** **Figure 11****).**

### Step2: Silanation:

Twenty five slides were treated with solution 4.09g of 2-Trimethoxysilane-6-(triisocyanatosilaneurea) benzene (0.01M) (1) in 200mL solution of Ethanol for 2 hours at room temperature under slight agitation. After 2h of reaction, slides were removed from the bath and washed three times with Ethanol and used for the next step without further drying **(seeFig 11).**

### Step 3: polymer coating:

1.5 g of poly(dimer acid-co-polyamine) - 95 (2) (purchased from Aldrich Chemicals, St. Louis, MO, USA) was added to the pyridine-dichloromethane mixture (150mL; 5: 1) and stirred for 1.5 hours at room temperature. Silylated slides were placed in this solution and kept at room temperature for 4.5 hours. Then slides were washed in the following order **(seeFig 11)**
● two times with pyridine (for 5 min)
● two times with dichloromethane (for 5 min each time)
● three times with ethanol (for 5 min each time).

### Step 4: Cross linker addition on the surface:

### EGS-Cross linker

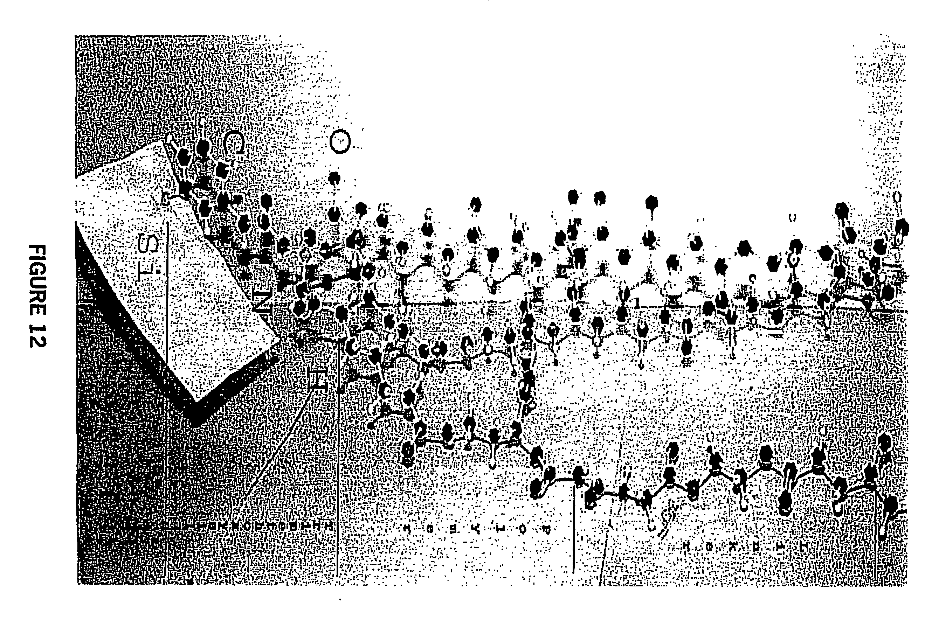

a). Slides prepared in Step 3 were treated with 20mM solution of (0.456g in 50ml of DMSO) ethylene glycol-bis-succinimidylsuccinate **(3-EGS)** for 4 hours at room temperature. The treated slides were then washed with ethanol: DMSO mixture (9:1) once, three times with ethanol, then dried. Figure 11 illustrates the chemistry of the branched silane coating: Branched silane was used for the surface coating to improve the sensitivity of target detection. In fact, this branched silane helped in reproducibility of target detection and to some extent in improving sensitivity. Figure 12 is a model picture of polymer coating on glass surface showing the polymer positioned on the glass surface angularly which is optimal for DNA printing.

### Diisocyanate cross linker

b). Slides prepared in Step 3 were treated with 26mM solution of (0.436g in 100ml DMSO) of 1,6-Diisocyanatohexane (3-C6) for 4 hours at room temperature. The treated slides were then washed once with ethanol: DMSO (9:1) mixture, three times with ethanol and dried. After linker addition, 3' amine modified DNA was arrayed and kept in the humid chamber for 12h. the arrayed slide was washed with water prior to assay development, then dried.

### Example 9: Cy3 Oligonucleotide spotting on polymer coated slides and dendrimer modified slides and average intensity comparison:

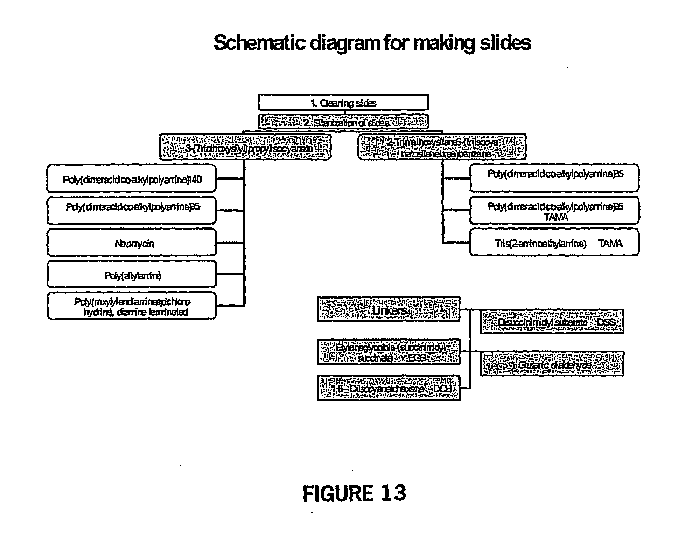

Various polymers and linkers were evaluated as surface coatings to produce branched surfaces. Figure 13 provides a schematic diagram for representative polymers and linkers and order of use. Among all these polymers, poly(dimer acid co-alkyl poly amine)-95 gave good results in detecting target DNA using gold nanoparticles probes and gave reproducible results with minimum background noise. Amine rich compounds like dendrimers, Tris (2-amino ethylamine), poly (allylamine) coated surfaces gave little higher backgrounds compared to Poly (dimer acid co-alkyl poly amine)-95 coated surface using gold nanoparticle probes. Without being bound by any theory of operation, it is believed that the gold nanoparticles are binding to unreacted amines on the surface. High backgrounds were observed using CY3 labeled oligonucleotides printing on amine-rich dendrimer surface.

For all polymer surface modifications, the procedures described in Example 8, including slide cleaning and surface modification, were followed. All the slides were cleaned prior to the dendrimer coating using cleaning procedure from page 2. The slides were treated with 5% 3-isocyanatopropylltriethoxysilane in anhydrous ethanol and kept at room temperature for 1h, washed with ethanol three times, and dried in dessicator under vacuum. The dried slides were then treated with Panam Starbust® G7 dendrimer (0.15% final concentration) in DMSO and kept for 5h at room temperature. The treated slides washed with DMSO and ethanol (2 times) and dried in dessicator under vacuum. Finally, dendrimer-linked slides were cross-linked with 100mM EGS linker in DMSO for 6h and washed with DMSO, ethanol successively and dried in dessicator under vacuum. After overnight drying in a dessicator, the slides were used for DNA printing..

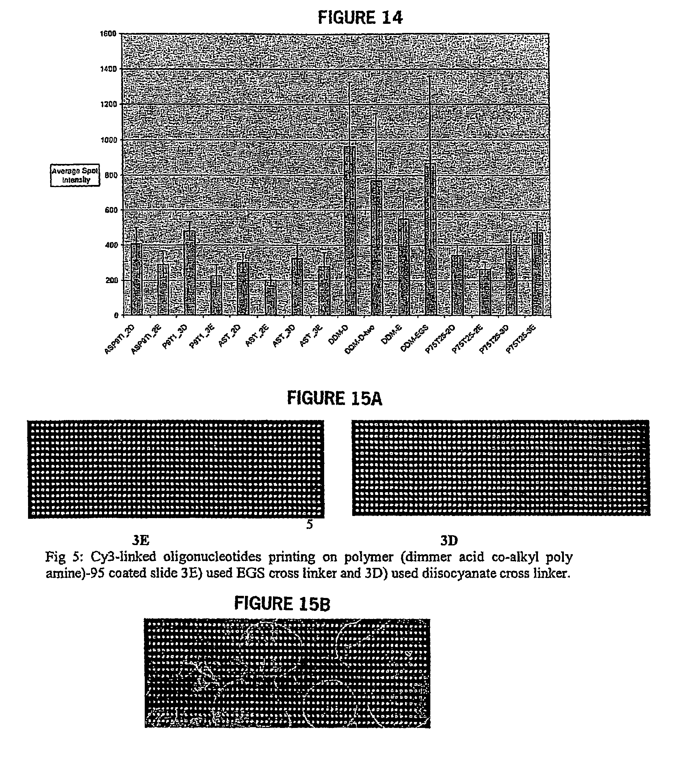

In this example, CY3 -linked oligonucleotide were printed on the polymer-coated surface and washed after 6h with water. CY3 intensity from oligonucleotide coupled to the surface was measured using fluorescent scanner. The results are shown in Figure 14. This Figure shows the average spot intensity of amine linked CY3 linked oligonnucleotide coupled to the modified surface. Different types of slides after CY3 oligonucleotide spotting are shown in Figures 15 (a) for 3E and 3D and Figure 16. Figure 15(a) and (b) illustrate polymer-coated slides after CY3-linked oligonucleotide spotting. Figure 16 illustrates dendrimer modified slides after CY3-linked oligonucleotide spotting

Comparing polymer-coated slides (Figure 15(a) and (b)) with the dendrimer slides (Figure 16), the dendrimer slides produced higher background noise when employed in gold nanoparticle-based assay and CY3 attachment studies. Without being bound by any theory of operation, it is believed that this may be due to the excess amine groups on the surface creating a positively-charged environment that absorbs negatively-charged molecules like DNA gold probes.

The protocol used in this Experiment is as follows:
1. Oligo Solution prepared FV43H+Cy3 (75nmole+60pmole)
2. Total 960 spots per slide were spotted using Cartesian arrayer
3. The slides where hydrated for 18 hours in a chamber
4. The slides were then dried and scanned, also washed and scanned, then analyzed
5. An oligo mix was prepared using the below amounts of DNA:
   a. FV43H = GGCGAGGAATA-(peg)3-NH2 (75nmoles) +
   b. CY3 oligo = Cy3-TCATCATCA-(Spacer18)-NH2 (60 pmoles)
6. Solution is dispensed across the substrate, the substrate was hydrated which allows the oligo mix to bind to the substrate
7. Substrates were then dried and washed with 0.2% SDS followed by MilliQ water
8. Substrates were then scanned using GenePix 4100A Scanner at 400 PMT, 40um resolution
9. The scanned images are saved and gridded using GenePix Pro 4 software.
10. Data was gathered and organized in MS Excel
11. Higher signal intensity is consistent with more Cy3 tags being present and therefore more binding capacity
The assay results do not indicate about how much DNA was hybridized and/or how proficient the substrate was in limiting background after silver stain. All the slides made following the above protocol were shown in Figures 15(a) and (b) and Figure 16 and a comparison of their intensity is shown in Figure 14. This test shows that amine-linked oligonucleotides can be covalently coupled to the modified surface all over the slide.

### Example 10: Evaluation of Polymer coated substrates

This Example illustrates detection of PCR products using the polymer-coated substrates of the invention. A one-step hybridization assay procedure based on nanoparticle-based probes was used.

**PCR Product Detection:** Slides 1, 2 & 3 PCR amplified duplex (80nM) were used for detection. The results are shown in Figures 16(a)-(c) respectively.

### FV99 PCR product

CTGAAAGGTTACTTCAAGGACAAAATACCTGTATTCCTCGCCTGTCCAGGGATCTGC
TCTTACAGATTAGAAGTAGTCCTATTAGCCCAGAGGCGATGTC

### Captures:

Wild type Factor V capture: FV43H - 5'- GGC GAG GAA TA- (spacer 18)₃-NH₂-3'
Mutant Factor V capture: FV44H - 5'- AGG CAA GGA AT- (spacer 18)₃-NH₂-3'
Positive control capture: PHA2H - 5'- TGA AAT TGT TAT C- (spacer 18)₃-NH₂-3'
Factor V probe: FV45Q - 5' Epi -AAA AAA AAA AAA AAA - (Spacer 18)₁ - CT TCT AAT CTG TAA GAG CAG 3'
Positive control probe: PHA1D - 5' Epi -AAA AAA AAA AAA AAA AAA AAG ATA ACA ATT TCA-3'

Nanoparticle loading was performed by standard protocol. Typically, 5'-epi disulfide (epi = epiandrosterone) oligonucleotide was loaded on citrate modified gold nanoparticles and kept at room temperature for 24h in the dark (4µM of modified oligonucleotide loaded per 1mL). Then salt addition was started and increased to 0.5 M in 6h of time and kept in the salt conditions total 40h at room temperature in the dark. After 40h oligonucleotide-linked gold nanoparticles were filtered and centrifuged using plastic tubes. The nanoparticle conjugates were then washed with water and resuspended in 0.1 M NaCl, 10nM phosphate, 0.01% azide pH: 7 buffer.

**Experimental procedure for slides 1, 2 & 3:** All the slides were washed with 0.2% SDS and water just prior to the experiment and dried by spinning at the room temperature. Aliquots for the assay were prepared using Factor V probe and positive control probe and place in the wells. Typically, 35µl of hybridization buffer (2X SSC, 0.2 tween), 5µl of water, 5 ul of target, 10 µl of colloid were mixed for each well and heated at 97°C and cooled at room temperature for 3 minutes. Then aliquots were transferred to the respective wells on the slide using pipette. For positive controls we used water in place of target since it is probe-capture hybridization. After 120 minutes hybridization at 40 °C, slides were washed with 5M NaNO₃ and amplified with a commercial silver amplification solution for 4 minutes at room temperature. Then washed with water twice and dried by spinning the slide. Slide was imaged on VERIGENE (Nanosphere Imaging System) and transferred the images to the word file.

**Slide no 1:** Xylene polymer coated slide used for DNA detection. The slide was prepared in accordance with Example 8. Wells 1 & 3 are used for positive controls and 2 & 4 used for wild type target. See Figure 16(a).

**Slide no 2:** PCR duplex detection on Tris-(2, amino ethylene) coated slide. The slide was prepared in accordance with Example 1. As shown in the picture this amine rich slide gave backgrounds with gold nanoparticle probes. See Figure 16(b)

**Slide no: 3:** PCR wild type duplex detection on polymer-95 coated surface. The slide was prepared as described in Example 8. Wells 1, 4, 5, 9 &10 used for probe controls. Wells 2, 3, 7 & 8 used for wild type factor V target and well 6 used for +ve control probe. See Figure 16(c).

From the above three slides and as shown in Figures 16(a)-(c), copolymer (dimer acid-co-alkyl polyamine)-95 slides worked better in terms of background and sensitivity. However, slide no: 2 which is coated with tris (2, amino ethylene) slide gave higher background using gold nanoparticle probe in the assay. This result tells us that amine rich surfaces are not suitable for gold nanoparticles assay for direct use and may be blocking is necessary.

**Slide no 4:** Dendrimer-linked slide: Dendrimer slide and polymer -95 coated were compared using gold nanoparticles. See Figures 16(d) and (e). The dendrimer slide was prepared as described in Benters et al., Nucleic Acids Research, Vol. 30, No. 2, e10, pp. 1-7 (2002). Dendrimer slide showed higher backgrounds compared to polymer coated slide in our experiment.

**Experimental procedure for slide 4:** Both the slides were washed with 0.2% SDS and water just prior to the experiment. After washing, all the slides were dried by spinning at room temperature. 700ul of hybridization buffer (2X SSC, 0.2 tween), 100ul of water, 200ul of colloid were mixed and layered on the slide using pipette. After 60 minutes at 40°C, slides were washed with 5M NaNO3 and amplified with a commercial silver amplification solution for 4 minutes at room temperature. The silver-treated slides were washed with water twice and dried by spinning and imaged on VERIGENE.

**Slide no 5:** In this experiment PCR target was diluted in different concentrations and used for detection on polymer -95 coated slides. See Figure 16(f).

**Experimental procedure:** The modified slide was washed with 0.2% SDS and water just prior to the experiment. After washing, all the slides were dried by spinning at room temperature. In each well different concentration of target was used to check the sensitivity (180nM, 1.8nM, 180pM & 18pM respectively). Typically, 35ul of hybridization buffer (2X SSC, 0.2 tween), 5ul of water, 5 ul of target, 10 ul of colloid were mixed for each well and heated at 97°C and cooled at room temperature for 3 minutes. Then aliquots were transferred to the respective wells on the slide using pipette. After 120 minutes hybridization at 40°C, slides were washed with 5M NaNO₃ and amplified with silver for 4 minutes at room temperature. Then washed with water twice and dried by spinning the slide. Slide was imaged on VERIGENE (Nanosphere Imaging System) and transferred the images to word file. From the images, it was observed that 18pm target could be detected using polymer (dimer acid co alkyl amine)-95-coated slides.

**Slide no 6:** This slide shows the detection of wild type, mutant and heterozygous targets detection simultaneously on polymer -95 coated slides. See Figure 16(g).

**Experimental Procedure for slide no. 6:** The modified slide was washed with 0.2% SDS and water just prior to the experiment. After washing, all the slides were dried by spinning at room temperature. Different types of samples such as wild type, mutant and heterozygous targets were used in different wells to check the specificity. Typically, 35ul of hybridization buffer (2X SSC, 0.2 Tween), 5ul of water, 5 ul of target, 10 ul of colloid were mixed for each well, heated at 97°C, then cooled at room temperature for 3 minutes. Aliquots were then transferred to the respective wells on the slide using pipette. For positive controls, water was used in place of target since it is probe-capture hybridization. After 120 minutes, hybridization at 40°C, the slides were washed with 5M NaNO₃ and amplified with a commercial silver amplification solution for 4 minutes at room temperature. Then washed with water twice and dried by spinning the slide. Slide was imaged on VERIGENE (Nanosphere Imaging System) and transferred the images to word file.

**Slide no 7:** A polymer -95 slide was compared with a commercial Codelink® slide (Amersham Corp.) using direct capture-probe hybridization assay. The instant polymer-95 coated slide of the invention performed nearly the same like the Codelink® slide, based on the results of this experiment. See Figure 16(h).

**Experimental Procedure:** In this experiment positive control probe directly hybridized to the capture strand to compare polymer coated slide with codelink slide. To 35ul of hybridization buffer (2X SSC, 0.2 tween), 15ul of water, 5 ul of colloid were mixed and added to each well. The slide kept under humidity chamber for 1h and washed with 5M NaNO₃ solution at room temperature and amplified with silver solution for four minutes. Both polymer coated slide (a) and codelink (b) slide gave similar result in terms hybridization.

The entire slides showed from 1 to 7 were developed using positive controls and Factor V PCR targets. From the results shown in Figures 16(a)-(i), it was concluded that the polymer -95 coated substrate was comparable with the commercially available Codelink® slides. However, amine-rich polymer coated slides had higher backgrounds **(slide 2 (****Figure 16(b)****) and** **4a**) (Figure 16(d)) in gold nanoparticle-based assay. In conclusion, amine-coated surfaces were not as good for gold nanoparticle probe-based assays and blocking steps were need to obtain better results.

After completing PCR product detection, detection of genomic targets Factor V and Factor II were attempted in a one-step assay format on polymer -95 coated slides. All the results using genomic target are shown below.

### Captures used:

**FV genomic WT -** 5'- TGG ACA GGC GAG GAA TAC AGG TAT -NH₂ - 3'
**FV genomic Mut -** 5'- CTG GAC AGG CAA GGA ATA CAG GTA TT -NH₂ - 3'

### Detection Probe used

**FV Epi Pro 46** - 5' epi- CCA CAG AAA ATG ATG CCC AGT GCT TAA CAA GAC CAT ACT ACA GTG A 3'

**Experimental Procedure for slides G1 to G3:** A modified slide was washed with 0.2% SDS and water just prior to the experiment. After washing, all the slides were dried by spinning at room temperature. Typically, 35ul of hybridization buffer (2X SSC, 0.2 tween), 5ul of formamide, 5 ml of genomic target (4 mg per ml), 2 ml of magnesium chloride (24.5mM) 10 ml of colloid were mixed for each well and heated at 97°C and cooled at room temperature for 5 minutes. All the controls prepared using water instead of target DNA. Then, aliquots were transferred to the respective wells on the slide using pipette. After 120 minutes hybridization at 40 °C, the slides were washed with 5M NaNO₃ and amplified with a commercial silver amplification solution for 4 minutes at room temperature. The treated slides were then washed with water twice and dried by spinning the slide. Slides were imaged on VERIGENE (Nanosphere Imaging System) and the images were transferred into MS Word files. Where "T" is marked in that well target was used and where "C" is marked there control used.

**Slide G1:** Genomic DNA Factor V (5ug /ul sample) detection and total 20ug of the sample was used in the assay. Assay was performed at 40 °C and both mutant and wild type capture showed the signals because both wild type and mutant hybrids have above 40 °C melting point. See Figure 16(j).

**Slide G 2:** Genomic DNA Factor V (5ug /ul sample) detection and total 20ug of the sample was used in the assay. Assay was performed at 40 °C and both mutant and wild type capture were shoed up at 40 °C. See Figure 16(k).

**Slide G 3:** Two times diluted genomic DNA sample detection (2.5 ug/ul sample) and total 10 ug of the genomic DNA was used for each well. See Figure 16(L).

**Slide G4:** Genomic DNA Factor V (5ug /ul sample) detection and total 20ug of the sample was used in the assay. Assay was performed at 47 °C and at higher. See Figure 16(m) temperature target bound very weakly to mutant capture as shown in the picture.

**Experimental Procedure for slides G4 to G5:** Modified slide was washed with 0.2% SDS and water just prior to the experiment. After washing, all the slides were dried by spinning at room temperature. Typically, 35ul of hybridization buffer (2X SSC, 0.2 tween), 5ul of formamide, 5 ul of factor V genomic target (4 ug per ml), 2ul of magnesium chloride (24.5 mM) 10 ul of colloid were mixed for each well and heated at 97°C and cooled at room temperature for 5 minutes. All the controls prepared using water instead of target DNA. Then aliquots were transferred to the respective wells on the slide using pipette. After 120 minutes hybridization at 47 °C, slides were washed with 5M NaNO₃ and amplified with a commercial silver amplification solution for 4 minutes at room temperature. Then washed with water twice and dried by spinning the slide. Slide was imaged on VERIGENE (Nanosphere Imaging System) and transferred the images to word file. Where "T" is marked in that well target was used and where "C" is marked there control used. Here we could differentiate wild type capture to mutant capture at 47 °C.

**Slide G5:** Genomic DNA Factor V (5ug /ml sample) detection and total 20 ug of the sample was used in the assay. Assay was performed at 47 °C and here mutant did not show up and wild type capture showed the signal. See Figure 16(n).

**Slide G6:** Genomic DNA Factor II (5ug /ml sample) detection and total 20 ug of the sample was used in the assay. Genomic DNA Factor II (5ug /ml sample). See Figure 16(o).

**Experimental Procedure for slide G6:** Modified slide was washed with 0.2% SDS and water just prior to the experiment. After washing, all the slides were dried by spinning at room temperature. Typically, 35ul of hybridization buffer (2X SSC, 0.2 tween), 5ul of formamide, 5 ml of genomic target (4 ug per ul), 2 ul of magnesium chloride (24.5mM) 10 ul of factor II colloid were mixed for each well and heated at 97°C and cooled at room temperature for 5 minutes. Then aliquots were transferred to the respective wells on the slide using pipette. After 120 minutes hybridization at 40 °C, slides were washed with 5M NaNO₃ and amplified with silver for 4 minutes at room temperature. Then washed with water twice and dried by spinning the slide. Slide was imaged on VERIGENE (Nanosphere Imaging System) and transferred the images to word file. Where "T" is marked in that well target was used and where "C" is marked there control used. All the controls prepared using water instead of target DNA. The results are shown in Figures 16(j) to (o).

In conclusion, we demonstrated both Factor V PCR and genomic sample target detection on newly developed copolymer-95 coated surface using oligonucleotides modified gold nanoparticle probes. This surface process is simple and economic to prepare in a commercially required environment.

### Example 11: Synthesis of 3' amine modified DNA linked gold nanoparticles (Fig. 17)

3'- amine linked oligonucleotide was synthesized on expedite gene synthesizer using 3'-amine support from Glen Research. At the end of the synthesis, 5' end epiandrosterone ("epi") disulfide phosphoramidite was attached to the 3'-amine oligonucleotide without using the detritylation step. After completing the synthesis the solid oligonucleotide linked support was dried and placed in ammonia solution at 55 °C overnight. After 18 hours of deprotection time, the ammonia was removed from the solution using a nitrogen flow into the tube. Then it was filtered and purified on HPLC using reverse phase column running under pH 7 phosphate buffer conditions. After purification, the 3'-amine and 5'- epi disulfide linked oligonucleotide was quantified by UV-Vis spectroscopy. The 3'-amine and 5'-epi disulfide oligonucleotide was loaded on citrate modified gold nanoparticles and kept at room temperature for 24 hours in the dark (4µM of modified oligonucleotide loaded per 1mL). See J. Am. Chem. Soc. 120, 1959-1964*;* Bioconjugate Chemistry, Vol 11, Number 2, P 289-291 which are incorporated by reference in its entirety. Then salt addition was started and increased to 0.5 M in 6 hours time and kept under salt conditions for a total of 40 hours at room temperature in the dark. After 40 hours the 3' amine linked gold nanoparticles were filtered and centrifuged using plastic tubes. The sample was then washed with water and resuspended in 0.1 M NaCl, 10nM phosphate, 0.01% azide pH 7 buffer. The resulting DNA modified gold nanoparticle probes were stored in 0.1M NaCl, 10mM Phosphate and 0.01% sodium azide pH 7 buffer.

These 3'-amine modified DNA linked gold nanoparticles were then spotted on an aldehyde modified glass surface, at different concentrations for immobilization, using a Cartesian arrayer (see Example 13). After spotting slides were allowed to sit in the chamber for 8-12 hours and washed with water and amplified with silver to see the amplified signals of immobilized gold nanoparticles (Fig. 17).

### Example 12: Synthesis of 3'-silyl linked gold nanoparticles preparation: (Fig 18)

3'- amine linked oligonucleotide was synthesized on an **expedite gene synthesizer** using 3'-amine support material obtained from **Glen Research.** At the end of the synthesis, 5' end epi disulfide phosphoramidite was attached to the 3'-amine oligonucleotide without using the detritylation step. After completing the synthesis, the solid oligonucleotide linked support was dried and put in ammonia solution at 55 °C overnight. After 18 hours of deprotection time all the ammonia was removed from the solution using nitrogen flow into the tube. It was then filtered and purified on HPLC using reverse phase column running under pH 7 phosphate buffer conditions. After purification, the 3'-amine and 5'- epi androsterone disulfide linked oligonucleotide was quantified using UV spectroscopy. The purified "3'amine-5'epianadrosterone" oligonucleotide was treated with 3-isocyanato propyl triethoxysilane in EtOH/DMSO mixture at room temperature to provide the 3'-silyl linked and 5'-epi disulfide oligonucleotide. After 1 hour, the reaction mixture was evaporated to dryness.

3'-silyl linked and 5'-epi disulfide oligonucleotide was loaded on citrate modified gold nanoparticles and kept at room temperature for 24 hours in the dark (4 µM of modified oligonucleotide loaded per 1mL). Salt addition was initiated and increased to 0.5 M in 6 hours of time and kept in the salt conditions total 40 hours at room temperature in the dark. The 3' amine linked gold nanoparticles were then filtered and centrifuged using plastic tubes. The sample was then washed with water and resuspended in 0.1 M NaCl, 10 nM phosphate, 0.01% azide pH 7 buffer.

### Example 13: Procedure for printing 3' amine linked gold nanoparticles and 3'-silyl linked gold nanoparticles on glass slides:

3'-silyl linked DNA modified gold nanoparticles and 3'-amine modified gold nanoparticles (DSPs) were arrayed on slides as part of a two component system. Component A contained 2x the final concentration of DSPs in probe storage buffer (100 mM Sodium Chloride in 10mM Sodium Phosphate pH 7). Component B contained 2x Telechem Micro spotting Plus Buffer (Cat. I.D.: MSP, TeleChem International). Glass substrate slides used for arraying were NoAb Hydrogel Aldehyde Activated Slides obtained from Noab BioDiscoveries (Cat. number UAS0005HA).

Slides were arrayed using a ***Cartesian ProSys 4510-8SQ*** arrayer. The room temperature during the arraying process was between 20 and 25 °C, and the humidity inside the arrayer while the slides were being spotted was between 25% and 40% relative humidity. One hour after completion of the arraying run, the slides were incubated in a sealed chamber at greater than 85% relative humidity for 8-18 hours. The slides were vacuum desiccated for at least one hour (but less than 24 hours), washed twice for two minutes with 0.2% sodium dodecyl sulfate (DNase, RNase, Protease free and 0.2um filtered) and washed twice for two minutes with MilliQ water. The slides were centrifuged to dryness (approx. 1 minute) and stored in a dessicator (relative humidity < 30%) until use.

The silver development process followed was as follows: Prior to enhancement Sigma silver solutions A and B were transferred from 4 °C to a 25 °C water bath and equilibrated for at least 20 minutes. Equal volumes of Sigma A and B were mixed, and used to develop signal for between 2-7 minutes. The reaction was stopped by adding 5% acetic acid, rinsed in acetic acid wash (x3), and slides finally rinsed with MilliQ water (x4). Slides were spun-dried for 45 seconds and scanned on an ArrayWorx E Biochip Reader.

The scan settings for the Arrayworx were: Channel: 4, Exposure time: 0.2 seconds, Sensitivity: Hi Dynamic Range and Resolution: 13.0080 um (Figures 19 and 20)). In Fig. 19, four dilutions for each prep were spotted starting at 3.5 nM. The TriEthoxy seems to give a much larger signal and a dose response. No carry-over was observed. The spots to the right were analyzed and will be presented as data. The spots to the left were not analyzed.

In Fig. 20, 0.8 M means probe was loaded in 0.8M salt conditions and 0.5 M means probe was loaded in 0.5 M salt conditions. Triethoxy means triethoxy silyl linked gold nanoparticles and monoethoxy means dimethylmonoethoxy silyl liked gold nanoparticles. Above shows the average of all data from 8 slides. No error bars are shown because the variability from slide to slide (in signal) is very large. However, the same trend was observed. A dose response was observed in all conditions however, the triethoxy had significantly higher signal.

### Example 14: Preparation of additional substrates and evaluation

In this Example, several additional substrates were prepared and evaluated. The reagents used and conditions are described below.

### Cleaning of Glass Slides (1a):

Glass slides were washed and prepared by immersion in four separate solutions at ambient temperature followed by rinsing with water three times after each wash. First, the slides were in a 5% w/v solution of sodium hydroxide in water for 30 minutes. The slides were then washed in a 5 % v/v hydrochloric acid solution for 30 minutes, followed by a 3 % hydrogen peroxide:5% v/v hydrochloric acid solution (H₂O₂:HCl) for 30 minutes. After rinsing with water the slides were rinsed with ethanol, three times, dried by spinning using a slide centrifuge. After air drying, the slides were placed in an oven overnight at 120 °C.

### Cleaning of Plastic Slides (1b):

Plastic slides were washed and prepared by immersion in four separate solutions at ambient temperature followed by rinsing with water three times after each wash. First, the slides were washed in an ethanol solution for 30 minutes, followed by a wash in a 5% w/v solution of sodium bicarbonate in water for 30 minutes. The slides were then washed in a 5 % v/v hydrochloric acid solution for 30 minutes, followed by a 3 % hydrogen peroxide:5% v/v hydrochloric acid solution (H₂O₂:HCl) for 30 minutes. After rinsing with water the slides were rinsed with ethanol, three times, dried by spinning using a slide centrifuge. After air drying, the slides were placed in a dessicator. Plastic is polycarbonate (Lexan®, Plexiglass®), polyphenol (Backlite®) and poly-cyclicnorbornene.

### Preparation of 2-Trimethoxysilane-6-(triisocyanatosilaneurea) benzene (4):

Tetraisocyanatosilane **(2)** (6.6692 g, 0.034 mol) and *m*-aminophenyltrimethoxysilane **(3)** (7.2954 g, 0.0342 mol) were mixed and stirred for 20 minutes at room temperature. To the solution was added dry ethanol (850 mL) and was stirred an additional 45 minutes at room temperature to produce **(4). 4** was left as a solution and added to the slides.

### Silanation of Glass Slides (5a) with 4:

Glass slides (25) were treated with a 250 mL of 0.04 M **(4)** {(2-Trimethoxysilane-6-triisocyanatosilaneurea) benzene} for 2 hours at ambient temperature with slight agitation. The slides were then removed, washed with ethanol three times and dried by spinning using a slide centrifuge to produce **(5a).** The substrate **(5a)** was further dried overnight in a desiccator under vacuum.

### Silanation of Plastic Slides (5b) with 4:

Plastic slides (65) in two containers were treated with a 300 mL of 0.04 M **(4)** {(2-Trimethoxysilane-6-triisocyanatosilaneurea) benzene} each, for 2 hours at ambient temperature with slight agitation. The slides were then removed, washed with ethanol three times and dried by spinning using a slide centrifuge to produce **(5b).** The substrate **(5b)** was further dried overnight in a desiccator under vacuum.

### Reaction of 5a (glass) and 5b (plastic) with Diaminobenzidine (DAB) to give 6a (glass) and 6b (plastic):

3,3'-Diaminobenzidine tetrahydrochloride dihydrate (8.9 g, 0.0225 mol) and triethyl amine (11.84 g, 0.117 mol) (TEA) were added to 834 mL of ethanol and stirred for one hour at room temperature and filtered. Twenty five silylated glass slides were placed in 250 mL of solution DAB at ambient temperature for 3 hours and then washed three times with ethanol for 5 minutes followed drying via spinning using the slide centrifuge to produce 6a. Sixty five plastic slides in two containers were treated with 300 mL and 250 mL each of a solution diaminobenzedine ( DAB) for 3 hours at ambient conditions. The plastic slides were washed with ethanol (3X) for five minutes each followed drying via spinning using the slide centrifuge to produce **6b.**

### Reaction of 5b (plastic) with Water:Ethanol to produce 6c (plastic):

Slides **(5b)** were treated with 130 mL of water-ethanol mixture (1:1) under slight agitation for 2 hours at ambient temperature. The slides were removed and washed with ethanol (2 X 100 mL) and dried in a dessicator at ambient temperature under vacuum to produce 6c.

### Capping of residual isocyanate groups of 6a (glass) and 6b (plastic) with glyine to produce 7a (glass) and 7b (plastic):

Glycine (6.38 g, 0.085 mol) was added to 850 mL of water and stirred for 15 minutes at ambient temperature to make 0.1 M glycine solution. Twenty five glass slides **(6a)** and plastic slides **(6b)** were placed in the 0.1 M Glycine solution at ambient temperature with slight agitation for two hours. The slides were washed with water (2X) for five minutes, followed with ethanol (3X) for five minutes each time and dried by spinning using a slide centrifuge to produce **7a** (Glass) and **7b** (plastic).

### Reaction of 7a (glass) and 7b (plastic) with tolylene-2,6-diisocyanate to produce 8a (glass) and 8b(plastic):

To 0.2624 g (0.00146 mol) of tolylene-2,6-diisocyanate, 850 mL of dry hexane was added and stirred for 15 minutes under ambient conditions. Twenty four glass slides **(7a)** were placed in 250 mL of solution of tolylene diisocyanate and 64 plastic **(7b)** in two separate containers of 300 mL and 250 mL respectively, and were kept at ambient temperature for 4 hours. The slides were washed with hexane (3X) and dried by spinning using a slide centrifuge to produce **8a** (glass) and **8b** (plastic). Note: tolylene-2,6-diisocyanate was replaced with 1,4-phenylene diisocyanate with a similar process.

### Reaction of 7a (glass) with 1,4-Phenylene Diisocyanate to produce 8c (glass).

1, 4-Phenylene diisocyanate, (0.095 g, 0.000594M) was added to 250 mL of dimethylformamide (DMF) (0.0024 mol/L solution) and stirred for 15 minutes at room temperature. Twenty-five glass slides **(7a)** were placed in the diisocyanate solution and kept at room temperature for 3 hours. After completion, slides were washed one time with DMF and three times with ethanol (for 5 min each time) and dried by spinning using a slide centrifuge to produce **8c.**

### Reaction of 8a (glass) with Poly (dimer acid-co-polyamine)-95 (P95) to produce 9a (glass):

Poly (dimer acid-co-polyamine)-95 (3.0 grams) was added to the mixture of 225 mL of pyridine and 35 mL of dichloromethane and stirred for 1.5 hours at ambient temperature. Twenty-four glass slides **(8a)** were treated with polymer solution for 4 hours under slight agitation at ambient temperature. The slides were then washed with pyridine for 5 minutes (2 x 250 mL), dichloromethane for 5 minutes (1 x 250mL), ethanol for 5 minutes (1 x 250 mL), di-isopropylethylamine:ethanol:water solution (5:127:127 mL ratio) for 2h followed with ethanol for 5 minutes (3 x 250 mL). Slides were dried by spinning using a slide centrifuge to produce **9a.**

### Reaction of 8b (plastic) with 3,3'-Diaminobenzidine Tetrahydrochloride Dihydrate (DAB) to produce 9b (plastic):

3,3'-Diaminobenzidine tetrahydrochloride dihydrate, (1.07 g, 0.0027mol), and triethyl amine (TEA) (1.4207 g, 0.014 mol) were added to 270 mL of ethanol and stirred for 1 hour at ambient temperature and filtered. Fifteen plastic slides **(8b)** were placed in solution and kept at temperature for 4 hours under slight agitation. The slides were then washed three times with ethanol (for 5 min each time) and dried by spinning using the slide centrifudge to produce **9b.**

### Reaction of 8b (plastic) with PAMAM Dendrimer Generation 0 to produce 9c (plastic):

A 20% methanol solution of PAMAM (0.58 mL) was added to 150 mL of ethanol, stirred for 30 minutes at ambient temperature. Five slides were placed in this solution and were treated for 4 hours under slight agitation. Then slides were washed three times with ethanol (for 5 min each time) and dried by spinning using the slide centrifudge to produce **9c.**

### Reaction of 8b (plastic) with Poly(m-xylylendiamine-epichlorohydrine), diamine terminated (PoXyl) to produce 9d (plastic):

PoXyl, (4.19 g, 0.00624 mol) was added to 520 mL of ethanol, stirred for 20 minutes at room temperature. Forty four plastic slides were placed in this solution. Slides were treated for 4 hours under slight agitation. Then slides were washed three times with ethanol (for 5 min each time) and dried by spinning using the slide centrifudge to produce **9d.** Dried *in vacuum* overnight.

### Reaction of 5b (plastic) with DAB-Am-8, Polypropylenimine Octaamine Dendrimer, Generation 2.0 to produce 9e (plastic):

Polypropylenimine Octaamine (0.60 g, 0.000775 M, MW = 773.31 g/mole) was added to 125 mL of ethanol and stirred for 30 minutes at ambient temperature. Eight silylated slides **(5b)** were reacted with the solution polyamine solution at ambient temperature for 2 hours. The slides were washed three times with ethanol (3 x 100 mL, for 5 min each time) and dried by spinning using the slide centrifuge.

### Reaction of 8c (glass) with Poly (dimmer acid-co-polyamine)- 95 (P95) to produce 9f (glass):

Poly (dimmer acid-co-polyamine)- 95 (3.0 g) was added to the mixture of 225 mL of pyridine and 35 mL of Dichloromethane and stirred for 1.5 hours at ambient temperature. Twenty-five slides **(8c)** were placed in the P95 solution for 3 hours. Then slides were washed with pyridine (2X, 5 minutes each), dichloromethane (5 minutes), ethanol (5 minutes), a solution of triethylamine:ethanol ( 5 mL:225 mL) for 2 hours, and a final rinse with ethanol (3X, 5 minutes each). The slides were dried by spinning using a slide centrifuge to produce **9f.**

### Reaction of 5a (glass) with Poly (dimmer acid-co-polyamine)- 95 (P95) to produce 9g (glass):

Poly (dimer acid-co-polyamine) - 95 (1.5 g) was added to 150 mL pyridine-dichloromethane mixture (5: 1) and stirred for 1.5 hours at ambient temperature. Slides **(5a)** were placed in the P95 solution and kept at room temperature for 4.5 hours. Then slides were washed with pyridine (5 minutes each), dichloromethane (2X 5 minutes), ethanol (3X, 5 minutes). The slides were dried by spinning using a slide centrifuge to produce **9g.**

### Reaction of 9a (glass) with Hexamethylene diisocyanate (DCH) to produce 10a (glass):

Hexamethylene diisocyanate (DCH), 22.891 grams (0.1361 mol) was added to 208 mL of hexane and stirred for 15 minutes at ambient conditions. Fifteen slides **(9a)** were treated with DCH solution for 5 hours under slight agitation. The slides were washed with hexane (2 x 250 mL) and ethanol (2 x 250 mL). Slides were dried by spinning using a slide centrifuge and stored in a dessicator to produce **10a.**

### Reaction of 9a (glass) with Triethyl Citrate to produce 10b (glass):

Triethyl citrate (4.421 grams, 0.016 mol) and 3.48 mL of diisopropylethylamine (DIPEA) were added to 228 mL of ethanol and stirred for 15 minutes at room temperature. Fourteen glass slides **9a** were treated with citrate solution for 5 hours under slight agitation. The slides were washed with ethanol (3 x 250 mL) and dried by spinning using a slide centrifuge.

### Reaction of 9a (glass), 9b (plastic), 9c (plastic), 9d (plastic) with 4,4'-Dicyclohexylmethane diisocyanate (HMDI) to produce 10e (glass), 10f (plastic), 10g (plastic), 10h (plastic) respectively.

A solution of 4,4'-Dicyclohexylmethane diisocyanate (HMDI) was prepared for 62 plastic and 23 glass slides from a combination of plastic and glass from **9a - 9d.** HMDI (77.64 g, 0.296 mol) and 1050 mL of Hexane (0.26mol/L) were combined and stirred for 15 minutes at room temperature. Sixty two plastic slides in two containers were treated with 300 mL and 250 mL of HMDI solution and twenty three glass slides in a separate container were treated with 250 mL of HMDI solution at ambient temperature for 6 hours. The slides were washed three times with hexane, ethanol once and dried by spinning on the slide centrifuge to produce **10e** (glass), 10f, **10h, 10g** (plastic).

### Reaction of 6c (plastic) with 1,6-Hexamethylene diisocyanate (DCH) to produce 10i (plastic):

1,6-Hexamethylene diisocyanate (13.08 g) and 75ml of dry ethanol were mixed together at ambient temperature. Five slides after **(6c)** were placed into vertical slide staining dish and treated with DCH solution for 4 hours under slight agitation. The slides were washed with ethanol (3 x 50 mL) and dried by spinning.

### Reaction of 6c (plastic) with Triethyl Citrate to produce 10j (plastic):

Triethyl citrate (27.6 g) and triethyl amine(3.03 g) were added to 75 mL of ethanol and stirred for 15 minutes. Five slides from **(6c)** were treated with citrate solution for 24 hrs at ambient temperature under slight agitation. The slides were washed three times with ethanol (3 x 50 mL) and dried by spinning using the slide centrifuge.

### Reaction of 9e (plastic) with Hexamethylene diisocyanate (DCH) to produce 10k (plastic):

1,6-Hexamethylene diisocyanate (DCH) (10.01 g, 0.060 M) was added to 120 mL of dry ethanol and mixed thoroughly at ambient temperature. Eight slides **(9e)** were treated with DCH solution for 5 hours at ambient temperature under agitation. The slides were washed three times with ethanol (3x100mL) and dried by spinning using the slide centrifuge.

### Reaction of 9f (glass) with 1, 6-Hexamethylene Diisocyanate to produce 10L (glass):

Four slides **(9f)** were placed into Vertical Slide Staining Dish containing 10 mL of a 0.5 M solution of (10.1 g in 9.9 mL of ethanol) 1, 6-hexamethylene diisocyanate in 110mL of EtOH. Slides were treated diisocyante for 5 hours at ambient temperature. Slides were removed, washed three times with ethanol and dried by spinning using a slide centrifuge to give **10L.**

### Reaction of 9f (glass) with 4,4'-Dicyclohexylmethane diisocyanate (HMDI) to produce 10m (glass):

Four slides **(9f)** were placed in Vertical Slide Staining Dish containing 70 mL of 0.50 M solution of (9.18 g/ 8.6 mL (0.035 Mol) 4,4'-Dicyclohexylmethane diisocyanate **(HMDI)** in 61 mL of dimethylformamide (DMF) for 5 hours at ambient temperature. Slides were removed, washed three times with DMF and dried by spinning using a slide centrifuge to give **10m.**

### Reaction of 9f (glass) with 4,4'-Methylenediphenyl diisocyanate (MDI) to produce 10n (glass):

Four slides **(9f)** were placed in Vertical Slide Staining Dish containing 70 mL of 0.50 M solution of (8.76 g, 0.035 Mol) 4,4'-Methylenediphenyl diisocyanate **(MDI)** in 70 mL of dimethylformamide (DMF) for 5 hours at ambient temperature. Slides were removed, washed three times with DMF and dried by spinning using a slide centrifuge to give **10n.**

### Reaction of 9f (glass) with to produce 10o (glass)

Four slides **(9f)** were placed in Vertical Slide Staining Dish containing 120 mL of 0.50 M solution of (16.58 g, 0.006 Mol) triethyl citrate **(T)** in 101 mL of ethanol and (3.33 g, 0.034 M) of triethyl amine (TEA). Slides were kept at room temperature for 24 hours and washed three times with ethanol and dried by spinning using a slide centrifuge to give **10o.**

### Reaction of 9f (glass) with triethyl citrate and 1,6-Hexamethylene diisocyanate to produce 10p (glass):

Three slides (9f) were placed in Vertical Slide Staining Dish containing a solution of triethyl citrate (7.425 g, 0.027 mol), 1,6-Hexamethylene diisocyanate (1.35 g, 0.008 mol) and triethylamine (0.97 g, 0.010 mol) in in 61 mL of ethanol for 5 hours at ambient temperature. Slides were removed, washed three times with ethanol and dried by spinning using a slide centrifuge to give **10p.**

### Reaction of 9f (glass) with triethyl citrate and 4,4'- Dicyclohexylmethane diisocyanate (HMDI) to produce 10q (glass):

Three slides **(9f)** were placed in Vertical Slide Staining Dish containing a solution of triethyl citrate (7.425 g, 0.027 mol), 4,4'- Dicyclohexylmethane diisocyanate (HMDI) (2.10 g, 0.008 mol) and triethylamine (0.97 g, 0.010 mol) in in 60 mL of dimethylformamide (DMF) for 5 hours at ambient temperature. Slides were removed, washed three times with DMF and dried by spinning using a slide centrifuge to give 10q.

### Reaction of 9f (glass) with Triethyl Citrate and 4,4'-Methylenediphenyl diisocyanate (MDI) to produce 10r (glass):

Three slides **(9f)** were placed in Vertical Slide Staining Dish containing a solution of triethyl citrate (7.425 g, 0.027 mol), 4,4'-Methylenediphenyl diisocyanate (MDI) (2.0 g, 0.008 Mol) and triethylamine (0.97 g, 0.010 mol) in in 62 mL of dimethylformamide (DMF) for 5 hours at ambient temperature. Slides were removed, washed three times with DMF and dried by spinning using a slide centrifuge to give **10r.**

### Reaction of 9f (glass) with ethylene glycol-bis-succinimidylsuccinate (3-EGS) to produce 10s (glass):

Slides **9f** were treated with a 20 mM solution of (0.456g in 50 mL of DMSO) ethylene glycol-bis-succinimidylsuccinate **(3-EGS)** in DMSO for 4 hours at room temperature. The treated slides were then washed with ethanol: DMSO mixture (9:1) once, three times with ethanol, then dried.

### Reaction of 9f (glass) with 1,6 Hexamethylene diisocyanate to produce 10t (glass):

Slides **9f** were treated with a 26 mM solution of (0.436g in 100 mL DMSO) of 1,6-Hexamethylene diisocyanate for 4 hours at ambient temperature. The treated slides were then washed once with ethanol: DMSO (9: 1) mixture, three times with ethanol and dried.

### Arraying of Slides:

Amine modified oligonucleotide captures were arrayed using a pin style or non-contact arraying system. The capture oligonucleotides were dissolved in aqueous solutions to prepare a range of concentrations (40 mM to 400 mM) with (0.001% to a 0.1% concentration) sodium dodecylsulfate (SDS), 300 mM phosphate buffer at pH = 7.2, and/or formamide. Several different types of capture oligonucleotide sequences were prepared with either 5' or 3' modified amino sequences ranging from 10 to 100 bases in length. The oligonucleotides may also have 3'-amino modified linkers attached to the end.

### Blocking of Arrayed Slides:

Ten slides were placed in a solution of 0.525 g of glycine in 70 mL of water and kept at ambient conditions under slight agitation for 2 hours. Slides were washed with SDS (0.2%) solution and three times with water. Slides were dried for 2 minutes on the slide centrifuge.

### Typical hybridization Conditions for Modified Slides - Single Step Method:

Arrayed slides were washed with 0.2% SDS prior to the assay development. A reaction volume of 50 µL was prepared per well using hybridization buffer (10 x SSC; 0.1% tween, formamide concentration of 18-70%). Nanoparticle probes (1 nM) and genomic target concentrations of 1µg/1µl were used in the hybridization mixture. The hybridization mixture was heated at 97 °C for 5 minutes and cooled at ambient temperture for 5 minutes, then transferred to the wells. Slides were kept at 40 °C - 41 °C for hybridization under humid conditions and then washed with buffer (0.5 M NaNO₃, 0.01 % tween). The nanopartilces on the surface were then exposed to silver enhancement reagents (Sigma-Aldrich) for 5 minutes and imaged with Verigene® detector (Nanosphere) for data analysis. The development of nanoparticle probes with oligonucleotides is described elsewhere. (Techniques for functionalizing oligonucleotides with sulfide groups and attachment to nanoparticles are described for instance in published U.S. patent application numbers 2003/0143598A1 and 2002/0155442A1, each of which is incorporated herein by reference in its entirety. A preferred sulfide linker for linking the oligonucleotide to the nanoparticle is an epiandrosterone linker. See PCT/US01/01190, filed January 12, 2001, which is incorporated by reference in its entirety.)

Each well ismarked with appropriate letter and **T, C, +ve C, M** and **Het.**
**T** = Genomic wild type DNA target was used with specific probe and hybridization buffer. No positive control probe and no other target were used.
**C** = Genomic probes mixed with hybridization buffer were used without any target or positive control probe.
**+ve C** = Positive control probe in hybridization buffer was used without any target or any other probes.
**M** = Genomic mutant type target was used in these wells with hybridization buffer and specific probe. No positive control probe or other target were used.
**Het** = Genomic heterozygous type target was used in these wells with hybridization buffer and specific probe. No positive control probe or other target were used.

Each well is marked with appropriate letter and T = genomic target used in these wells, C = Control, no target, no +Ve control probe, +VeC = Positive control probe, no target, M= mutant target used and Het = heterozygous target was used.

### Typical hybridization Conditions for Modified Slides - Dual Step Method:

**Step 1:** Arrayed slides were washed with 0.2% SDS just prior to the assay development. A total of 50 µL of reaction volume was prepared per well using hybridization buffer (10 x SSC; 0.1% tween, formamide concentration of 18-70%), genomic DNA target concentrations varied from 10² to 10⁷ copies per µL. Hybridization mixture was denatured at 95 °C for 4 min and cooled at ambient temperature for 3 minutes then transferred to the respective wells. Slides were incubated at 40 °C for 2h for hybridization under humid conditions.
**Step 2:** After target hybridization, the slides were washed with NaNO₃, and tween and spin dried. The probe solution was mixed with the hybridization mixture and was added to each well and incubated at 40°C under humid conditions for 30-120 minutes. Slides were washed with wash buffer and treated with silver solutions A and B for five minutes and imaged with Verigene® instrument for data analysis. The development of nanoparticle probes with oligonucleotides is described elsewhere (Techniques for functionalizing oligonucleotides with sulfide groups and attachment to nanoparticles are described for instance in published U.S. patent application numbers 2003/0143598A1 and 2002/0155442A1, each of which is incorporated herein by reference in its entirety. A preferred sulfide linker for linking the oligonucleotide to the nanoparticle is an epiandrosterone linker. See PCT/US01/01190, filed January 12, 2001, which is incorporated by reference in its entirety).

Each well is marked with appropriate letter and **T, C, +ve C, M** and **Het.**
**T** = Genomic wild type DNA target was used with specific probe and hybridization buffer. No positive control probe and no other target were used.
**C** = Genomic probes mixed with hybridization buffer were used without any target or positive control probe.
**+ve C** = Positive control probe in hybridization buffer was used without any target or any other probes.
**M** = Genomic mutant type target was used in these wells with hybridization buffer and specific probe. No positive control probe or other target were used.
**Het** = Genomic heterozygous type target was used in these wells with hybridization buffer and specific probe. No positive control probe or other target were used.

### Reagent List:

NaOH - Sodium Hydroxide; Pellets; Fisher Scientific
HCl - Hydrochloric Acid, 35-38%; Fisher Scientific
H₂O₂ - Hydrogen Peroxide, 30%; Fisher Scientific
EtOH - Ethyl Alcohol, 200 proof, ACS/USP Grade; Pharmco, Inc.
NaHCO₃ - Sodium Bicarbonate; Fisher Scientific
Si(NCO)₄ - Tetraisocyanatosilane; Gelest, Inc.
m-Aminophenyltrimetoxysilane, Gelest, Inc.
DAB - 3,3'-Diaminobenzidine tetrahydrochloride dihydrate
TEA - Triethylamine; Fisher Scientific
GLY - Glycine, Sigma-Aldrich
TDIC - Tolylene-2,6-diisocyanate, Aldrich
Hxn - Hexane, Fisher Scientific
PDIC - 1,4-Phenylene diisocyanate, Aldrich
P95 - Poly(dimmer acid-co-polyamine)-95, Sigma-Aldrich
Pyridine - Sigma-Aldrich
DCM - Dichloromethane, Fisher Scientific
DiPEA - Diisopropylethylamine, Aldich
PAMAM Dendrimer Generation 0,Sigma-Aldrich
PoXyl - Poly(m-xylylendiamine-epichlorohydrine), diamine terminated, Aldrich
DAB-Am-8 - Polypropyleneimine Octaamine Dendrimer, Generation 2.0, Aldrich
DCH -Hexamethylenediisocyanate, Sigma-Aldrich
T - Triethyl Citrate, Sigma-Aldrich
HMDI - 4,4'-Dicyclohexylmethane diisocyanate, Aldrich
MDI - 4,4'-Methylenediphenyl diisocyanate, Aldrich
3-EGS - Ethylene glycol-bis-succinimidylsuccinate, Pierce
DMSO - Dimethyl sulfoxide, Aldrich
SDS - Sodium Dodecyl Sulfate, 20% solution, Fisher Scientific
Formamide, Sigma-Aldrich, Fisher Scientific
20xSSC Buffer, Invitrogen, Fisher Scientific
Tween 20 - Polyoxyethylenesorbitan monolaurate, Sigma-Aldrich
NaNO₃ - Sodium Nitrate, Sigma-Aldrich
Silver enhancer solution A, Sigma-Aldrich
Silver enhancer solution B, Sigma-Aldrich

### EQUIPMENT AND MATERIALS

**Timer** refers to Fisher Scientific, Traceable Timer, Cat. # 06-662-55, Calibr. Due 06/05, #320808935
**Centrifuge** refers to Telechem Int'l, Microarray High-Speed Centrifuge, Cat. # MHC110V
**Termix Stirrer** refers to Fisher Scientific, Cat. # 14-493-120S, Model 120S
**Hg-Thermometer** refers to Fisher Sciencific, Cat. # 103606, -10°C - +350 °C, 1 °C, Ser. #2121
Filter paper, Whatman Int, Ltd., Cat. # 1202-320

### References:

1. Nucleic Acids research, vol 22, 5456-5465 (1994).
2. Nucleic Acids research, vol 24, 3040-3047 (1996).
3. Nucleic Acids research, vol 24, 3031-3039 (1996).
4. Nucleic Acids research, vol 27, 1970-1977 (1999).
5. Angew Chem. Int. Ed, 38, No.9, 1297 (1999)
6. Analytical biochemistry 280, 143-150 (2000).
7.
   (a) Nucleic Acids research, vol. 28, No. 13 E71 (2000);
   (b) Huber et al. WO 01/46214, published June 28, 2001
   (c) Huber et al. WO 01/46213, published June 28, 2001
   (d) Huber et al. WO 01/46464, published June 28, 2001
*8.* Nucleic Acids research, vol 29, 955-959 (2001).
*9.* Nucleic Acids research, vol 29, No.13 e69 (2001).
10. Bioconjugate Chemistry, 2000, 11, 289-291

## Claims

1. A method for making a substrate for use in target analyte detection, said method comprising:
(a) providing a substrate having a surface;
(b) contacting said surface with a isocyanate compound so as to provide a surface comprising free isocyanate groups, the isocyanate compound is a member selected from the group consisting of:
Si(NCY)₄;
[(R₁)(R₂)(R₃)Si-X-Z-CYNH]₂-Si (NCY)₂ vi;
and
(R₁)(R₂)(R₃)Si-X-Z-CYNH-Si (NCY)₃ iv;
wherein R₁, R₂ and R₃ independently represents C₁-C₆ alkoxy, C₁-C₆ alkyl, phenyl, or aryl substituted with one or more groups selected from the group consisting of C₁-C₆ alkyl and C₁-C₆ alkoxy; X represents linear or branched C₁-C₂₀ alkyl or aryl substituted with one or more groups selected from the group consisting of C₁-C₆ alkyl and C₁-C₆ alkoxy, optionally substituted with one or more heteroatoms comprising oxygen, nitrogen, or sulfur, Y represents oxygen or sulfur; and Z represents oxygen or NH, with the proviso that at least one of R₁, R₂, or R₃ represents C₁-C₆ alkoxy.

2. The method of claim 1, further comprising, after step (b):
(c) contacting said surface comprising free isocyanate groups with a spacer molecule so as to provide a surface comprising free amino groups; and
(d) contacting said surface comprising free amino groups with a linker molecule so as to provide a reactive surface having free reactive groups.

3. The method of claim 2, further comprising repeating steps (c) and (d) one or more times.

4. The method of claims 2 or 3, further comprising, after step (d):
(e) contacting said reactive surface with at least one type of capture probe specific for the target analyte so as to provide a surface comprising immobilized capture probes; and
(f) contacting said surface comprising immobilized capture probes with a capping agent being a member selected from the group consisting of amino acid, protein, carbohydrate, carboxylate, thiol, alcohol, and amine, so as to block residual unreacted free isocyanate groups on areas of the surface not having immobilized capture probes and produce a substrate having substantially low signal background due to non-specific nanoparticle binding relative to a surface not contacted with a capping agent.

5. The method of claim 1, further comprising:
(c) contacting the surface comprising free isocyanate groups with water so as to provide a surface comprising free amino groups; and
(d) contacting said surface comprising free amino groups with a linker molecule so as to provide a reactive surface having free reactive groups.

6. The method of claim 5, further comprising, after step (d):
(e) contacting said surface comprising free isocyanate groups with a spacer molecule so as to provide a surface comprising free amino groups; and
(f) contacting said surface comprising free amino groups with a linker molecule so as to provide a reactive surface having free reactive groups.

7. The method of claim 6, further comprising repeating steps (e) and (f) one or more times.

8. The method of claims 5, 6 or 7, further comprising:
(i) contacting said reactive surface with at least one type of capture probe specific for the target analyte so as to provide a surface comprising immobilized capture probes; and
(ii) contacting said surface comprising immobilized capture probes with a capping agent being a member selected from the group consisting of amino acid, protein, carbohydrate, carboxylate, thiol, alcohol, and amine, so as to block residual unreacted free isocyanate groups on areas of the surface not having immobilized capture probes and produce a substrate having substantially low signal background due to non-specific nanoparticle binding relative to a surface not contacted with a capping agent.

9. The method according to any one of claims 1 to 8 wherein the disilylisocyanate compound is selected from the group consisting of 2-Trimethoxysilane-6-triisocyanatosilanceureabenzene and tetraisocyanatosilane.

10. The method of any one of claims 2 or 6 wherein the spacer molecule has at least two functional groups that can react with a isocyanate group.

11. The method of claim 10 wherein the spacer molecule is polymer, a carbohydrate, or antibiotic.

12. The method of claims 10 or 11, wherein the spacer molecule is a member selected from the group consisting of poly (dimmer acid-co-alkylpolyamine)-95, poly(dimmer acid-co-alkylpolyamine)-140, poly(allylamine), poly(m-xylendiamine-epichlorohydrin diamine terminated, tris(2-aminoethylamine), and PAMAM dendrimer generation 0, neomycin, and 3,3'-diaminobenzidene.

13. The method of claim 4 or 8 wherein the capping reagent is glycine.

14. The method of any one of claims 2, 5 or 6 wherein the linker molecule is a member selected from the group consisting of phenylene 1,4-diisocyanate, tolylene-2,6-diisocyanate, tolylene-α,4-diisocyanate, and isophorone diisocyanate.

15. The method of any one of claims 2, 5 or 6 wherein the linker molecule is selected from the group consisting of ethylene glycolbis (succinimidylsuccinate), disuccinimidyl suberate, 1,6-diisocyanatohexane, methylene bis-(4-cyclohexylisocyanate, glutaric dialdehyde, methylene-p-phenyl diisocyanate, and triethyl citrate.

16. The method of any one of claims 4 or 8, wherein the capture probe is a nucleic acid.

17. The method of any one of claims 4 or 8 wherein more than one type of capture probes are contacted with the surface having second reactive moieties, each type of capture probes is specific for a particular target analyte.

18. The method of claim 4 or 8 wherein the capture probes are arrayed in discrete predetermined areas on the surface of the substrate.

19. The method of claim 1, said method further comprising:
(c) contacting said surface comprising free isocyanate groups with a spacer molecule so as to provide a surface comprising free amino groups;
(d) contacting said surface comprising free amino groups with a linker molecule so as to provide a reactive surface having free reactive groups;
(e) contacting said reactive surface with at least one type of capture probe specific for the target analyte so as to provide a surface comprising immobilized capture probes; and
(f) contacting said surface comprising immobilized capture probes with a capping agent being a member selected from the group consisting of amino acid, protein, carbohydrate, carboxylate, thiol, alcohol, and amine, so as to block residual unreactive free isocyanate groups and produce a substrate having substantially low signal background due to non-specific nanoparticle binding relative to a surface not contacted with a capping agent.

20. The method according to any one of claims 1 to 19 wherein the substrate has at least one group that reacts with the isocyanate compound.

21. The method of claim 20 wherein the group comprises hydroxyl, amino, or carboxylate.

22. A substrate for use in detection of one or more target analytes, said substrate comprising a surface with an attached capture probe is prepared by the method of any of claims 4, 8, or 19.

23. The substrat of claim 22 comprising a surface having a polymeric layer comprising free amino groups capable of binding said capture probes, and negatively charged ionic groups.

24. The substrate of claim 23, wherein said surface produces a background signal upon imaging using visual or fluorescent light having substantially reduced background signal relative to a substrate not having said polymeric layer.

25. The substrate of claim 22, wherein said substrate has a water contact angle ranging from about 25 to 75 degrees.

26. The substrate of claim 25, wherein the substrate has a refractive index ranging from about 1.400 to 1.900.

27. A method for detecting one or more target analytes in a sample, the target analyte having at least two binding sites, comprising:
(a) providing a substrate of any one of claims 1 or 19, said substrate having at least one type of capture probes immobilized on a surface of the substrate, each type of capture probes specific for a target analyte;
(b) providing at least one type of detection probe comprising a nanoparticle and a detector probe, the detector probe specific for a target analyte;
(c) contacting the capture probes, the detection probes and the sample under conditions that are effective for the binding of the capture probes and detector probes to the specific target analyte to form an immobilized complex onto the surface of the substrate;
(d) washing the surface of the substrate to remove unbound nanoparticles; and
(e) observing for the presence or absence of the complex as an indicator of the presence or absence of the target molecule.

28. A substrate prepared by the methods of any one of claims 1 or 19.

29. A kit comprising a substrate of claim 28.

## Patentansprüche

1. Verfahren zum Herzustellen eines Substrats zur Verwendung in der Target-Analyten-Detektion, das Verfahren umfassend:
(a) Bereitstellen eines Substrats mit einer Oberfläche;
(b) Kontaktieren der Oberfläche mit einer Isozyanat-Verbindung, um so eine Oberfläche, umfassend freie Isozyanat-Gruppen, bereitzustellen, wobei die Isozyanat-Verbindung ein Mitglied der Gruppe ist, die besteht aus:
Si(NCY)₄;
[(R₁)(R₂)(R₃)Si-X-Z-CYNH]₂-Si (NCY)₂ vi ;
und
(R₁)(R₂)(R₃)Si-X-Z-CYNH-Si (NCY)₃ iv ;
wobei R₁, R₂ und R₃ unabhängig C₁-C₆-Alkoxy, C₁-C₆-Alkyl, Phenyl oder Aryl darstellen, substituiert mit einer oder mehrerer Gruppen, ausgewählt aus der Gruppe bestehend aus C₁-C₆-Alkyl und C₁-C₆-Alkoxy; X stellt lineares oder verzweigtes C₁-C₂₀-Alkyl oder Aryl dar, substituiert mit einer oder mehrer Gruppen ausgewählt aus der Gruppe bestehend aus C₁-C₆-Alkyl und C₁-C₆-Alkoxy, optional substituiert mit einem oder mehreren Heteroatomen umfassend Sauerstoff, Stickstoff oder Schwefel; Y stellt Sauerstoff oder Schwefel dar; und Z stellt Sauerstoff oder NH dar, mit der Maßgabe, das mindestens einer von R₁, R₂ oder R₃ C₁-C₆-Alkoxy darstellt.

2. Verfahren von Anspruch 1, ferner umfassend nach Schritt (b):
(c) Kontaktieren der Oberfläche, die die freien Isozyanat-Gruppen umfasst, mit einem Spacer-Molekül, um so eine Oberfläche bereitzustellen, die freie Amino-Gruppen umfasst; und
(d) Kontaktieren der Oberfläche, die freie Amino-Gruppen umfasst, mit einem Linkermolekül, um so eine reaktive Oberfläche mit freien reaktiven Gruppen bereitzustellen.

3. Verfahren von Anspruch 2, ferner umfassend das Wiederholen der Schritte (c) und (d) für ein oder mehrere Male.

4. Verfahren von Ansprüchen 2 oder 3, ferner umfassend nach Schritt (d):
(e) Kontaktieren der reaktiven Oberfläche mit mindestens einem Typ von Fänger-Sonde, die für den Target-Analyten spezifisch ist, um so eine Oberfläche bereitzustellen, die immobilisierte Fänger-Sonden umfasst; und
(f) Kontaktieren der Oberfläche, die immobilisierte Fänger-Sonden umfasst, mit einem Capping-Agens, das ein Mitglied ist, ausgewählt aus der Gruppe bestehend aus Aminosäure, Protein, Karbohydrat, Carboxylat, Thiol, Alkohol und Amin, um so verbliebene nicht reagierte freie Isozyanat-Gruppen in Gebieten der Oberfläche zu blocken, die keine immobilisierten Fänger-Sonden besitzen und ein Substrat herzustellen, das im Wesentlichen geringen Signalhintergrund wegen der nicht spezifischen Nanopartikelbindung relativ zu einer Oberfläche besitzt, die nicht mit Capping-Agens in Kontakt gebracht wurde.

5. Verfahren von Anspruch 1, ferner umfassend:
(c) Kontaktieren der Oberfläche, die freie Isozyanat-Gruppen umfasst, mit Wasser, um so eine Oberfläche bereitzustellen, die freie Amino-Gruppen besitzt; und
(d) Kontaktieren der Oberfläche, die freie Amino-Gruppen umfasst, mit einem Linkermolekül, um so eine reaktive Oberfläche mit freien reaktiven Gruppen bereitzustellen.

6. Verfahren von Anspruch 5, ferner umfassend nach Schritt (d):
(e) Kontaktieren der Oberfläche, die die freien Isozyanat-Gruppen umfasst, mit einem Spacer-Molekül, um so eine Oberfläche bereitzustellen, die freie Amino-Gruppen umfasst; und
(f) Kontaktieren der Oberfläche, die freie Amino-Gruppen umfasst, mit einem Linkermolekül, um so eine reaktive Oberfläche mit freien reaktiven Gruppen bereitzustellen.

7. Verfahren von Anspruch 6, ferner umfassend das Wiederholen der Schritte (e) und (f) für ein oder mehrere Male.

8. Verfahren von Ansprüchen 5, 6 oder 7, ferner umfassend:
(i) Kontaktieren der reaktiven Oberfläche mit mindestens einem Typ von Fänger-Sonde, die für den Target-Analyten spezifisch ist, um so eine Oberfläche bereitzustellen, die immobilisierte Fänger-Sonden umfasst; und
(ii) Kontaktieren der Oberfläche, die immobilisierte Fänger-Sonden umfasst, mit einem Capping-Agens, das ein Mitglied ist, ausgewählt aus der Gruppe bestehend aus Aminosäure, Protein, Karbohydrat, Carboxylat, Thiol, Alkohol und Amin, um so verbliebene nicht reagierte freie Isozyanat-Gruppen in Gebieten der Oberfläche zu blocken, die keine immobilisierten Fänger-Sonden besitzen und ein Substrat herzustellen, das im Wesentlichen geringen Signalhintergrund wegen der nicht spezifischen Nanopartikelbindung relativ zu einer Oberfläche besitzt, die nicht mit Capping-Agens in Kontakt gebracht wurde.

9. Verfahren gemäß irgendeinem der Ansprüche 1 bis 8, wobei die Disilylisozyanat-Verbindung ausgewählt ist aus der Gruppe bestehend aus 2-Trimethoxysilan-6-triisozyanatosilanceureabenzen und Tetraisozyanatosilan.

10. Verfahren von irgendeinem der Ansprüche 2 oder 6, wobei das Spacer-Molekül mindestens zwei funktionale Gruppen besitzt, die mit einer Isozyanat-Gruppe reagieren können.

11. Verfahren von Anspruch 10, wobei das Spacer-Molekül ein Polymer, Karbohydrat oder Antibiotikum ist.

12. Verfahren von Anspruch 10 oder 11, wobei das Spacermolekül ein Mitglied ist, ausgewählt aus der Gruppe, bestehend aus Poly(Dimmersäure-Co-Alkylpolyamin)-95, Poly(Dimmersäure-Co-Alkylpolyamin)-140, Poly(allylamin), Poly(m-Xylendiamin-Epichlorohydrin-Diamin terminiert, Tris(2-aminoethylamin) und PAMAM Dendrimer Generation 0, Neomycin und 3,3'-Diaminobenziden.

13. Verfahren von Anspruch 4 oder 8, wobei das Capping-Agens Glycin ist.

14. Verfahren von irgendeinem der Ansprüche 2, 5 oder 6, wobei das Linker-Molekül ausgewählt ist aus der Gruppe bestehend aus Phenylen-1,4-Diisozyanat, Tolylen-2,6-Diisozyanat, Tolylen-[alpha],4-Diisozyanat und Isophoron-Diisozyanat.

15. Verfahren von irgendeinem der Ansprüche 2, 5 oder 6, wobei das Linker-Molekül ausgewählt ist aus der Gruppe bestehend aus Ethylen-Glycolbis (Succinimidylsuccinat), Disuccinimidyl-suberat, 1,6-Diisozyanatohexan, Methylen-bis-(4-Cyclohexylisozyanat, Glutardialdehyd, Methylen-p-Phenyl-diisozyanat und Triethylzitrat.

16. Verfahren von irgendeinem der Ansprüche 4 oder 8, wobei die Fänger-Sonde eine Nukleinsäure ist.

17. Verfahren von irgendeinem der Ansprüche 4 oder 8, wobei mehr als ein Typ von Fänger-Sonden mit der Oberfläche, die zweite reaktive Einheiten besitzt, in Kontakt gebracht wird, wobei jeder Typ von Fänger-Sonden spezifisch für einen bestimmten Target-Analyten ist.

18. Verfahren von Anspruch 4 oder 8, wobei die Fänger-Sonden in bestimmten zuvor festgelegten Gebieten der Oberfläche des Substrates angeordnet sind.

19. Verfahren von Anspruch 1, das Verfahren ferner umfassend:
(c) Kontaktieren der Oberfläche, die die freien Isozyanat-Gruppen umfasst, mit einem Spacer-Molekül, um so eine Oberfläche bereitzustellen, die freie Amino-Gruppen umfasst;
(d) Kontaktieren der Oberfläche, die freie Amino-Gruppen umfasst, mit einem Linkermolekül, um so eine reaktive Oberfläche mit freien reaktiven Gruppen bereitzustellen.
(e) Kontaktieren der reaktiven Oberfläche mit mindestens einem Typ von Fänger-Sonde, die für den Target-Analyten spezifisch ist, um so eine Oberfläche bereitzustellen, die immobilisierte Fänger-Sonden umfasst; und
(f) Kontaktieren der Oberfläche, die immobilisierte Fänger-Sonden umfasst, mit einem Capping-Agens, das ein Mitglied ist, ausgewählt aus der Gruppe bestehend aus Aminosäure, Protein, Karbohydrat, Carboxylat, Thiol, Alkohol und Amin, um so verbliebene nicht reagierte freie Isozyanat-Gruppen zu blocken, und ein Substrat herzustellen, das im Wesentlichen geringen Signalhintergrund wegen der nicht spezifischen Nanopartikelbindung relativ zu einer Oberfläche besitzt, die nicht mit Capping-Agens in Kontakt gebracht wurde.

20. Verfahren gemäß irgendeinem der Ansprüche 1 bis 19, wobei das Substrat mindestens eine Gruppe besitzt, die mit der Isozyanat-Verbindung reagiert.

21. Verfahren von Anspruch 20, wobei die Gruppe Hydroxyl, Amino oder Carboxylat umfasst.

22. Substrat für die Verwendung in der Detektion von einem oder mehreren Target-Analyten, wobei das Substrat, das eine Oberfläche mit einer daran angebrachten Fänger-Sonde umfasst, durch das Verfahren irgendeines der Ansprüche 4, 8 oder 19 hergestellt wird.

23. Substrat von Anspruch 22, das eine Oberfläche mit einer Polymerschicht besitzt, die freie Aminogruppen umfasst, die in der Lage sind die Fänger-Sonden und negativ geladene Ionengruppen zu binden.

24. Substrat von Anspruch 23, wobei die Oberfläche ein Hintergrundsignal nach Visualisierung mit sichtbarem oder fluoreszentem Licht erzeugt, die ein wesentlich vermindertes Hintergrundsignal relativ zu einem Substrat besitzt, das die Polymerschicht nicht besitzt.

25. Substrat von Anspruch 22, wobei das Substrat einen Wasserkontaktwinkel im Bereich von ungefähr 25 bis 75 Grad besitzt.

26. Substrat von Anspruch 25, wobei das Substrat einen Brechungsindex im Bereich von ungefähr 1400 bis 1900 besitzt.

27. Verfahren zum Detektieren eines oder mehrerer Target-Analyten in einer Probe, wobei der Target-Analyt mindestens zwei Bindungsstellen besitzt, umfassend:
(a) Bereitstellen eines Substrates irgendeines der Ansprüche 1 oder 19, wobei das Substrat mindestens einen Typ von Fänger-Sonden besitzt, die auf einer Oberfläche des Substrats immobilisiert sind, wobei jeder Typ von Fänger-Sonden spezifisch für ein Target-Analyt ist,
(b) Bereitstellen mindestens eines Typs von Detektionssonde, die einen Nanopartikel und eine Detektorsonde umfasst, wobei die Detektorsonde für einen Target-Analyten spezifisch ist;
(c) In Kontakt Bringen der Fänger-Sonden, der Detektionssonden und der Probe unter Bedingungen, die das Binden der Fänger-Sonden und Detektorsonden an den spezifischen Target-Analyten bewirken, um einen immobilisierten Komplex auf der Oberfläche des Substrats zu bilden;
(d) Waschen der Oberfläche der Substrats, um ungebundene Nanopartikel zu entfernen; und
(e) Beobachten des Vorliegens oder der Abwesenheit des Komplexes als einen Indikator für das Vorliegen oder die Abwesenheit des Target-Moleküls.

28. Substrat hergestellt durch die Verfahren irgendeines der Ansprüche 1 oder 19.

29. Kit umfassend ein Substrat von Anspruch 28.

## Revendications

1. Un procédé pour fabriquer un substrat pour l'utilisation dans une détection d'une substance cible à analyser, ledit procédé comprenant
(a) mise à disposition d'un substrat ayant une surface;
(b) mise en contact de ladite surface avec un composé d'isocyanate pour fournir une surface comprenant des groupes d'isocyanate libre, le composé d'isocyanate est un élément sélectionné à partir du groupe composé de :
Si (NCY)₄;
[(R₁) (R₂) (R₃) Si-Y-Z-CYNH]₂-Si (NCY)₂ vi ;
et
(R₁) (R₂) (R₃) Si - X - Z - CYNH - Si (NCY)₃ iv ;
où R₁, R₂, et R₃ représentent de façon indépendant ( C₁ - C₆ ) alkoxy, ( C₁ - C₆ ) alkyl, phényle, ou aryle avec une ou plusieurs groupes sélectionné (s) à partir du groupe composé de ( C₁ - C₆ ) alkyl et ( C₁ - C₆ ) alkoxy, X représente (C₁ - C₂₀ ) alkyl ou ( C₁ - C₂₀ ) aryle linéaires ou ramifiés substitués par une ou plusieurs groupes sélectionné (s) à partir du groupe composé de ( C₁ - C₆ ) alkyl, ( C₁ - C₆ ) alkoxy, de façon optionnelle substitué (s) par un ou plusieurs hétéroatomes comprenant de l'oxygène, du nitrogène, ou du soufre ; Y représente de l'oxygène ou du soufre ; et Z représente de l'oxygène ou NH avec la condition qu'au moins un de R₁, R₂, et R₃ représente (C₁ - C₆) alkoxy.

2. Le procédé selon la revendication 1, comprenant en outre après l'étape (b) :
( c ) mise en contact de ladite surface comprenant des groupe d'isocyanate libre avec un espaceur moléculaire pour la mise à disposition d'une surface comprenant des groupes aminés libres ; et
( d ) mise en contact de ladite surface comprenant des groupes aminés libres avec un molécule de liaison pour la mise à disposition d'une surface réactive ayant des groupes réactifs libres.

3. Le procédé selon la revendication 2, comprenant en outre une ou plusieurs fois la répétition des étapes ( c ) et ( d ) .

4. Le procédé selon les revendications 2 ou 3, comprenant en outre après l'étape (d) :
( e ) mise en contact de ladite surface réactive avec au moins un type de sonde de capture spécifique pour la substance cible à analyser pour la mise à disposition d'une surface comprenant des sondes de capture immobilisées, et
( f ) mise en contact de ladite surface comprenant des sondes de capture immobilisées avec un agent d'enrobage , étant un élément sélectionné dans le groupe composé d'acide aminé, de protéine, de glucide, de carboxylate, de thiol, d'alcool et d'amine, pour bloquer des groupes d'isocyanate libre inaltéré résiduel sur des zones de la surface n'ayant pas de sondes de capture immobilisées et pour produire un substrat ayant un signal de fond essentiellement bas en raison de nanoparticules non spécifiques établissant une liaison par rapport à une surface non mise en contact avec un agent d'enrobage.

5. Le procédé selon la revendication 1, comprenant en outre :
( c ) mise en contact de la surface comprenant des groupe d'isocyanate libre avec de l'eau pour la mise à disposition d'une surface comprenant des groupes aminés libres; et
( d ) mise en contact de ladite surface comprenant des groupes aminés libres avec un molécule de liaison pour la mise à disposition d'une surface réactive ayant des groupes réactifs libres.

6. Le procédé selon la revendication 5, comprenant en outre, après l'étape ( d) :
( e ) mise en contact de ladite surface comprenant des groupe d'isocyanate libre avec un espaceur moléculaire pour la mise à disposition d'une surface comprenant des groupes aminés libres ; et
( d ) mise en contact de ladite surface comprenant des groupes aminés libres avec un molécule de liaison pour la mise à disposition d'une surface réactive ayant des groupes réactifs libres.

7. Le procédé selon la revendication 6, comprenant en outre une ou plusieurs fois la répétition des étapes ( e ) et ( f ).

8. Le procédé selon les revendications 5, 6 ou 7, comprenant en outre:
( i ) mise en contact de ladite surface réactive avec au moins un type de sonde de capture spécifique pour la substance cible à analyser pour la mise à disposition d'une surface comprenant des sondes de capture immobilisées, et
( ii ) mise en contact de ladite surface comprenant des sondes de capture immobilisées avec un agent d'enrobage, étant un élément sélectionné dans le groupe composé d'acide aminé, de protéine, de glucide, de carboxylate, de thiol, d'alcool et d'amine, pour bloquer des groupes d'isocyanate libres inaltérés résiduels sur des zones de la surface n'ayant pas de sondes de capture immobilisées et pour produire un substrat ayant un signal de fond essentiellement bas en raison de nanoparticules non spécifiques établissant une liaison par rapport à une surface non mise en contact avec un agent d'enrobage.

9. Le procédé selon n'importe laquelle des revendications 1 à 8, où le composé de disilylisocyanate est sélectionné dans le groupe composé de 2 - triméthoxysilane - 6 - triisocyanatosilaneeureabenzène et tétracyanatosilane.

10. Le procédé selon n'importe laquelle des revendications 2 ou 6, où l'espaceur moléculaire a au moins deux groupes fonctionnels qui peuvent réagir avec le groupe d'isocyanate.

11. Le procédé selon la revendication 10, où l'espaceur moléculaire est un polymère, un glucide ou un antibiotique.

12. Le procédé selon la revendication 10 ou 11, ou l'espaceur moléculaire est un élément sélectionné dans le groupe composé de poly ( dimmer acide - co - alkypolyamine ) - 95, poly ( dimmer acide - co - alkypolyamine ) - 140, poly ( allylamine ) , poly ( m-xylènediamine - épichlorohydrine diamine terminé) tris ( 2 - aminoéthylamine ) et PAMAM dendrimère génération 0, néomycine, et 3, 3' - diaminobenzidène.

13. Le procédé selon les revendications 4 ou 8, où le réactif d'enrobage est de la glycine.

14. Le procédé selon les revendications 2, 5 ou 6, où la molécule de liaison est un élément sélectionné dans le groupe composé de phénylène 1, 4 - diisocyanate, tolylène - 2, 6 - diisocyanate, tolylène - α, 4- diisocyanate , et isophorone diisocyanate.

15. Le procédé selon les revendications 2, 5 ou 6, où la molécule de liaison est sélectionné dans le groupe composé de gycolbis ( succinimidylsuccinate ), disuccinimidyle subérate, 1, 6 - diisocyanatohexane, méthyle bis - ( 4 - cyclohexylisocyanate, dialdéhyde glutarique, méthylène - p - phényle diisocyanate, et citrate de triéthyle.

16. Le procédé selon n'importe laquelle des revendications 4 ou 8, où la sonde de capture est acide nucléique.

17. Le procédé selon les revendications 4 ou 8, où plus qu'un type de sondes de capture est mis en contact avec la surface ayant deuxièmes fragments réactifs, chaque type de sonde de capture est spécifique pour une substance cible particulière à analyser.

18. Le procédé selon les revendications 4 ou 8, où les sondes de capture sont disposées dans des zones prédéterminées discrètes sur la surface du substrat.

19. Le procédé selon la revendication 1 ledit procédé comprenant en outre :
( c ) mise en contacte de ladite surface comprenant des groupes d'isocyanate libre avec un espaceur moléculaire pour la mise à disposition d'une surface comprenant des groupes amino libres ;
( d) mise en contacte de ladite surface comprenant des groupes amino libre avec un molécule libre pour la mise à disposition d'une surface ayant des groupes réactifs libres,
( e) mise en contact de ladite surface réactive avec au moins un type de sonde de capture pour la substance cible à analyser pour la mise à disposition d'une surface comprenant des sondes de capture immobilisées, et
( f ) mise en contact de ladite surface comprenant des sondes de capture immobilisées avec un agent d'enrobage, étant un élément sélectionné dans le groupe composé d'acide aminé, de protéine, de glucide, de carboxylate, de thiol, d'alcool et d'amine, pour bloquer des groupes d'isocyanate libre inaltéré résiduel et pour produire un substrat ayant un signal de fond essentiellement bas en raison de nanoparticules non spécifiques établissant une liaison par rapport à une surface non mise en contact avec un agent d'enrobage.

20. Le procédé selon n'importe laquelle des revendications 1 à 19, où le substrat a au moins un groupe qui réagit avec le composé d'isocyanate.

21. Le procédé selon la revendication 20, où le groupe comprend hydroxyle, amino, ou carboxylate.

22. Un substrat pour l'utilisation dans la détection d'une ou plusieurs substances cibles à analyser, où ledit substrat comprenant une surface avec des sondes de capture attachées est préparé par le procédé de n'importe laquelle des revendications 4, 8 ou 19.

23. Le substrat selon la revendication 22, comprenant une couche polymérique comprenant des groupes amino capables de lier lesdites sondes de capture, et des groupes ioniques ayant une charge négative.

24. Le substrat selon la revendication 23, où ladite surface produit un signal de fond après le traitement d'image en utilisant une lumière visible ou fluorescente ayant essentiellement réduit le signal de fond par rapport à un substrat ayant aucune couche polymérique.

25. Le substrat selon la revendication 22, où ledit substrat a un angle de contact avec l'eau entre environ 25 et 75 °.

26. Le substrat selon la revendication 25, où le substrat a un indice de refraction entre environ 1.400 et 1.900.

27. Un procédé pour détecter un ou plusieurs substances cibles à analyser dans un échantillon, la substance cible à analyser ayant au moins deux sites de liaison, comprenant :
(a) mise à disposition d'un substrat selon n'importe laquelle des revendications 1 ou 19, ledit substrat ayant au moins un type de sondes de capture immobilisées sur une surface du substrat, où chaque type de sondes de capture spécifique est spécifique ;
(b) mise à disposition d'au moins d'un type de sonde de détection comprenant un nanoparticule et une sonde de détection, où la sonde de détection est spécifique pour une substance cible à analyser,
(c) mise en contact des sondes de capture, où les sondes de détection et l'échantillon répondent à des conditions qui sont efficaces pour la liaison des sondes de capture et les sondes de détection à la substance cible à analyser pour former un complexe immobilisé sur la surface du substrat ;
(d) lavage de la surface pour enlever des nanoparticules non liés, et
(e) observation de la présence ou absence du complexe comme indicateur de la présence ou absence du molécule cible.

28. Un substrat préparé selon les procédés selon n'importe laquelle des revendications 1 ou 19.

29. Un kit comprenant un substrat selon la revendication 28.
